# EUROPEAN PATENT SPECIFICATION

(11) **EP 0 475 367 B1**
(45) Date of publication and mention of the grant of the patent: **20.03.1996**
(21) Application number: 91115308.8
(22) Date of filing: 10.09.1991
(51) Int. Cl.: C07D 251/52, C08K 5/3492

(54) **Diamino-s-triazinon derivatives and self-extinguishing polymeric compositions containing them**
Diamino-s-Triazinonderivate und diese enthaltende selbstlöschende polymere Zusammensetzungen
Dérivés de la diamino s-triazinone et compositions polymères auto-extinctrices les contenant

(30) Priority: 11.09.1990 IT 2142090
(43) Date of publication of application: 18.03.1992
(73) Proprietor: MINISTERO DELL' UNIVERSITA' E DELLA RICERCA SCIENTIFICA E TECNOLOGICA, I-00196 Roma (IT)
(72) Inventor: Cipolli, Roberto, Dr., I-28100 Novara (IT); Nucida, Gilberto, I-20098 San Giuliano Milanese, Milan (IT); Masarati, Enrico, Dr., I-29010 Castelnuovo Valtidone, Piacenza (IT); Oriani, Roberto, I-20137 Milan (IT); Pirozzi, Mario, Dr., I-20097 San Donato Milanese, Milan (IT)
(74) Representative: Barz, Peter, Dr.

(56) References cited:
- EP-A- 0 014 463
- DD-A- 39 717
- DE-A- 3 322 318
- DE-A- 3 537 724
- US-A- 2 945 762
- US-A- 2 950 196
- US-A- 4 201 705
- US-A- 4 504 610
- CHEMICAL ABSTRACTS, vol. 100, No. 9, February 27, 1984, Columbus, Ohio, USA; GOJMERAC, TIHOMIRA "Degradation of N,N'-bis(4-chloro-6-ethylamino-S-triazinyl-2)-cystine in buffered extract of cucumber seedlings (Cucumis sativus)" page 197, column 2, abstract -No. 63 390f

## Description

The present invention relates to derivatives of 2,4-diamino-6-hydroxy-1,3,5-triazine (ameline).

More particularly the present invention relates to ameline derivatives and to the use thereof for the preparation of self-extinguishing polymeric compositions based on thermoplastic polymers and/or polymers endowed with elastomeric properties, especially olefinic polymers and copolymers, in combination with ammonium and/or amine phosphates and/or ammonium and/or amine phosphonates.

Several methods of reducing or eliminating the combustibility of polymers are known in the art. Some of said methods are based on the use of metal compounds, in particular on antimony, bismuth or arsenic, in combination with partially halogenated, thermally unstable organic compounds, such as chlorinated paraffin waxes.

Other methods are based on the use of substances capable of yielding intumescence. The formulations of the intumescent type generally are composed of the polymer and at least three main additives, i.e., an essentially phosphorus-containing additive, whose purpose is to form, during the combustion, an impermeable, semi-solid and vitreous layer essentially composed of polyphosphoric acid and to activate the process of formation of intumescence; a second additive, containing nitrogen, which is to serve as foaming agent; and a third, carbon-containing additive, which acts as a carbon donor in order to allow an insulting cellular carbonaceous layer ("char") to be formed between the polymer and the flame.

Examples of intumescent formulations of said type may be found in US-A-3,810,862 (Phillips Petroleum Co.; based on melamine, pentaerythritol and ammonium polyphosphate); US-A-4,727,102 (Vamp S.r.l.; based on melamine cyanurate, a hydroxyalkyl derivate of isocyanuric acid and ammonium polyphosphate); and WO-85/05626 (Plascoat U.K. Limited; on the basis of various phosphorus and nitrogen compounds among which, in particular, a combination of melamine phosphate, pentaerythritol and ammonium polyphosphate may be mentioned).

In more recent formulations, together with an organic or inorganic phosphorus compound, a nitrogen-containing organic compound was used, generally consisting of an aminoplastic resin obtained by means of condensation of urea, melamine or dicyandiamide with formaldehyde.

Examples of formulations containing two additives are those described in US-A-4,504,610 (Montedison S.p.A.; based on oligomeric derivatives of 1,3,5-triazine and ammonium polyphosphate) and EP-A-14,463 (Montedison S.p.A.; based on organic compounds selected from benzylguanamine and reaction products of aldehydes and several cyclic nitrogen compounds, in particular benzylguanamine-formaldehyde copolymers, and ammonium polyphosphate).

Self-extinguishing compositions can also be obtained by using single-component additives, which contain in their organic molecule both nitrogen and phosphorus atoms, as disclosed in US-A-4,201,705 (Borg-Warner Corp.).

These intumescent flame retardant systems endow the polymers to which they are added with the property of forming a carbonaceous residue when they burn or are exposed to a flame. This kind of flame-retardant system offers numerous advantages, i.e., absence of corrosion phenomena in the machinery in which the polymers are processed, a lower emission of smokes as compared to systems containing metal compounds and halogenated hydrocarbons, and, above all, the possibility of endowing the polymers with satisfactory flame-proof properties with a smaller amount of total additive and therefore without excessively impairing the mechanical properties thereof.

It has now surprisingly been found that it is possible to endow polymers with very good antiflame properties by using a class of compounds derived from 2,4-diamino-6-hydroxy-1,3,5-triazine, said derivatives being more effective than products known in the prior art.

Furthermore, said derivatives also show a good heat stability so that they retain their high activity as flame-retardants even when the polymeric compositions containing them are heat-processed.

Moreover, polymeric compositions containing the compounds of the present invention have the advantage of emitting, in the event of fire, only small amounts of (non-darkening) smokes.

Accordingly, the present invention provides ameline deriva

tives of general formula (I): 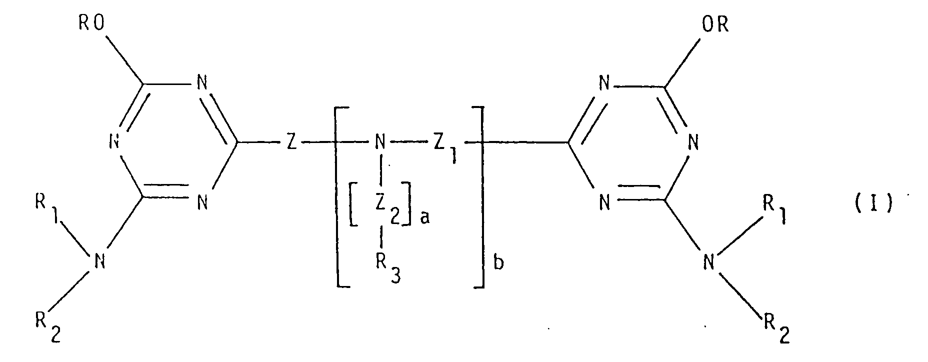
wherein:
R = H; (C₂-C₆)-alkenyl; (C₆-C₁₂)-cycloalkyl; (C₆-C₁₂)-alkylcycloalkyl; (C₆-C₁₂)-aryl; (C₇-C₁₂)-aralkyl; or ⁅CₙH₂ₙ⁆Y, n being an integer of from 1 to 8, preferably from 1 to 4, and Y representing H; CN; -O-(C₁-C₄)-alkyl; -O-(C₂-C₄)-alkenyl; (C₆-C₁₂)-cycloalkyl; (C₆-C₁₂)-alkylcycloalkyl; -O-(C₆-C₁₂)-aryl or -N(R₄)₂, wherein the groups R₄, the same or different from each other, are (C₁-C₄)-alkyl or (C₃-C₄)-alkenyl or the moiety -N(R₄)₂ is replaced by an N-heterocyclic radical which is linked to the alkyl chain through the nitrogen atom and is selected from pyrrolidyl; piperidyl; morpholyl; thiomorpholyl; piperazyl; 4-methylpiperazyl and 4-ethylpiperazyl;
the groups R₁ and R₂, the same or different from each other and having the same or different meanings in each triazine ring, are selected from H; (C₁-C₁₈)-alkyl; (C₂-C₈)-alkenyl; (C₆-C₁₆)-cycloalkyl; (C₆-C₁₆)-alkylcycloalkyl;
⁅CₘH₂ₘ⁆O-R₅; and 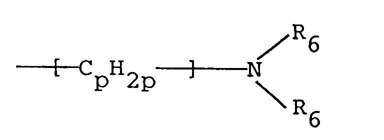
wherein:

- m: = integer of from 2 to 8, preferably from 2 to 4;
- p: = integer of from 2 to 6;
- R₅: = H; (C₁-C₈)- and preferably (C₁-C₄)-alkyl; (C₂-C₆)-alkenyl;
⁅C_{q}H_{2q}⁆O-R₇, q being an integer of from 1 to 4 and R₇ being H or (C₁-C₄)-alkyl; (C₆-C₁₂)-cycloalkyl; and (C₆-C₁₂)-alkylcycloalkyl;
the groups R₆, the same or different from each other, are selected from H; (C₁-C₈)-alkyl; (C₂-C₆)-alkenyl; (C₆-C₁₂)-cycloalkyl; (C₆-C₁₂)-alkylcycloalkyl; and (C₁-C₄)-hydroxyalkyl; or the moiety -N(R₆)₂ is replaced by an N-heterocyclic radical which is linked to the alkyl chain through the nitrogen atom and is
selected from aziridyl; pyrrolidyl; piperidyl; morpholyl; thiomorpholyl; piperazyl; 4-methylpiperazyl and 4-ethylpiperazyl;
or in general formula (I) one or both of the moieties -NR₁R₂ are replaced by an N-heterocyclic radical which is linked to the triazine ring through the nitrogen atom and is selected from aziridyl; pyrrolidyl; piperidyl; morpholyl; thiomorpholyl; piperazyl; 4-methylpiperazyl; 4-ethylpiperazyl; 2-methylpiperazyl; 2,5-dimethylpiperazyl; 2,3,5,6-tetramethylpiperazyl; 2,2,5,5-tetramethylpiperazyl; 2-ethylpiperazyl; and 2,5-diethylpiperazyl;
a is 0 or 1;
b is 0 or an integer of from 1 to 5;
R₃ is hydrogen or a group of formula 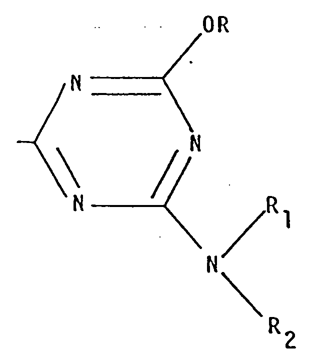
and its meaning may vary within each repeating unit;
when b is O:
Z is a divalent radical selected from those of the following formulae: 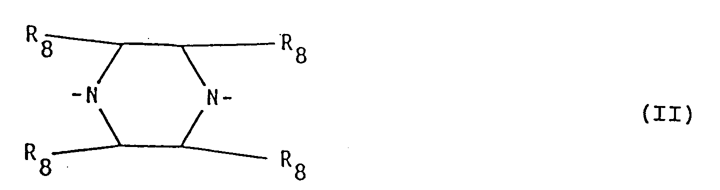
wherein the groups R₈, the same or different from each other, represent hydrogen or (C₁-C₄)-alkyl; 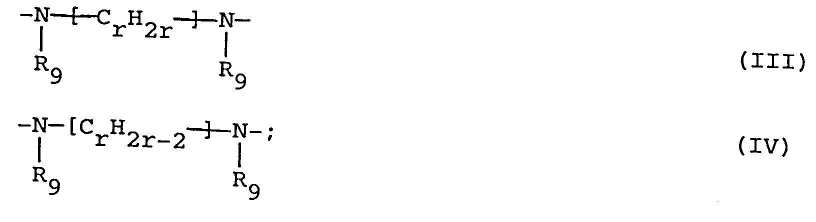
wherein r is an integer of from 2 to 14 and R₉ is selected from hydrogen; (C₁-C₄)-alkyl; (C₂-C₆)-alkenyl; (C₁-C₄)-hydroxyalkyl; 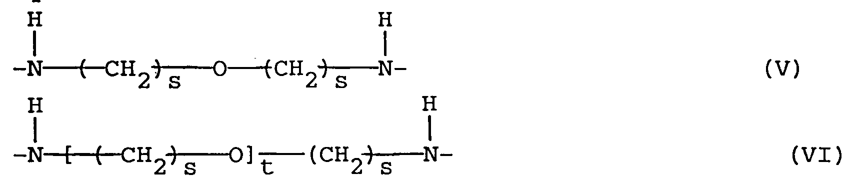
wherein s is an integer of from 2 to 5 and t is an integer of from 1 to 3; 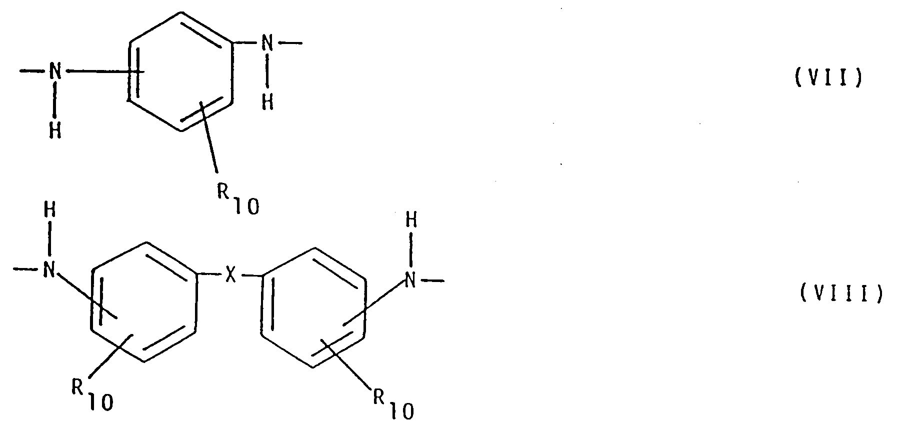
wherein X is a direct carbon-carbon bond (C-C); O; S; S-S;
SO; SO₂; NH; NHSO₂; NHCO; N=N; or CH₂; and
R₁₀ is hydrogen; hydroxy; (C₁-C₄)-alkyl or (C₁-C₄)-alkoxy; 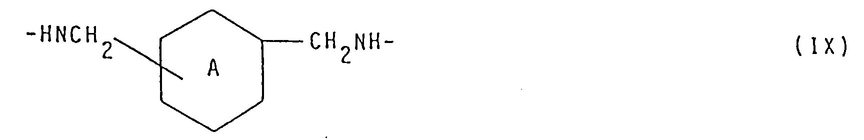
wherein A is an either saturated or unsaturated ring; 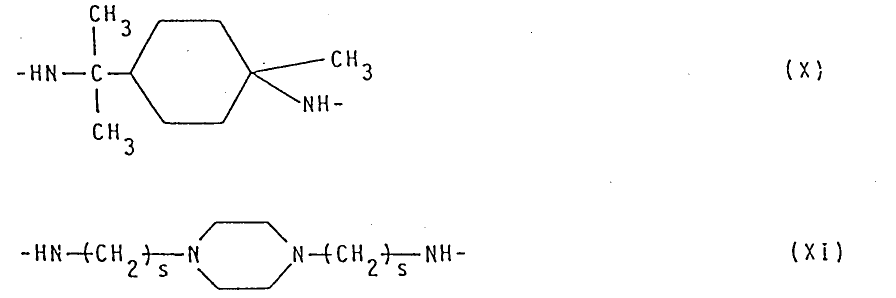
wherein s is an integer of from 2 to 5;
for all designated states except Denmark with the proviso that when R represents (C₁-C₈)-alkyl, (C₆-C₁₂)-cycloalkyl, (C₇-C₁₂)-aralkyl or a phenyl or naphthyl group optionally substituted with inert radicals, and R¹ and R² are (C₁-C₃)-alkyl or (C₆-C₁₂)-cycloalkyl groups, or -NR¹R² represents a piperidinyl or morpholinyl group, Z cannot be of formula (II) wherein all four of the groups R₈ are hydrogen (in view of EP-A-413 999, a document relevant under Art. 54(2) EPC);

### when b is an integer of from 1 to 5:

the moiety of formula 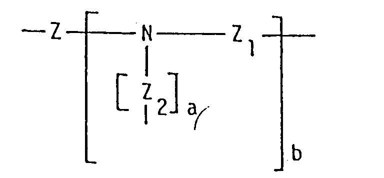
is a polyvalent radical selected from those of the following formulae; 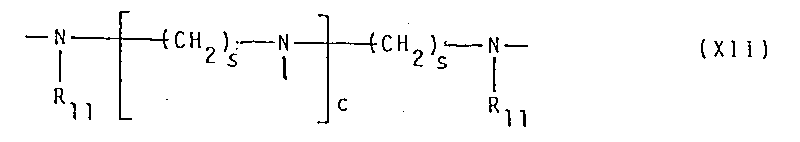
wherein R₁₁ is hydrogen or (C₁-C₄)-alkyl;
c is an integer of from 1 to 5;
the subscripts s, the same or different from each other, are integers of from 2 to 5; 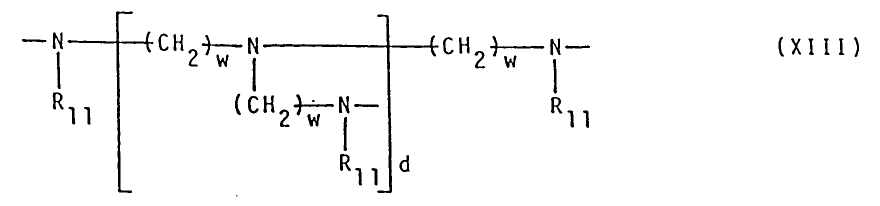
wherein R₁₁ is hydrogen or (C₁-C₄) alkyl;
w is an integer of from 2 to 4; and
d is 1 or 2.

General formula (I) also covers derivatives with an asymmetric structure in that the groups R, R₁ and R₂ may have different meanings on each triazine ring.

Specific examples of groups R in general formula (I) are methyl; ethyl; propyl; isopropyl; n-butyl; isobutyl; tert-butyl; n-pentyl; isopentyl; n-hexyl; tert-hexyl; octyl; tert-octyl; ethenyl; propenyl; butenyl; isobutenyl; hexenyl; cyclohexyl; propylcyclohexyl; butylcyclohexyl; phenyl; benzyl; 2-phenylethyl; cyanomethyl; 2-cyanoethyl; 2-methoxyethyl; 2-methoxypropyl; 4-methoxybutyl; 5-methoxypentyl; 6-methoxyhexyl; 7-methoxyheptyl; 7-methoxyoctyl; 2-ethoxyethyl; 3-ethoxypropyl; 4-ethoxybutyl; 5-ethoxypentyl; 3-propoxypropyl; 3-butoxypropyl; 4-butoxybutyl; 4-isobutoxybutyl; 5-propoxypentyl; 2-cyclohexyloxyethyl; 2-ethenyloxyethyl; 2-phenoxyethyl; 2-(N,N-dimethylamino)ethyl; 3-(N,N-dimethylamino)propyl; 4-(N,N-dimethylamino)butyl; 5-(N,N-dimethylamino)pentyl; 6-(N,N-dimethylamino)hexyl; 4-(N,N-diethylamino)butyl; 5-(N,N-diethylamino)pentyl; 2-(N,N-dipropylamino)ethyl; 4-(N,N-dipropylamino)butyl; 2-(N,N-diisopropylamino)ethyl; 2-(N-methyl-N-1-propenylamino)ethyl; 2-(N,N-di-1-propenylamino)ethyl; 4-(N,N-di-1-propenylamino)butyl.

Specific examples of groups R₁ and R₂ in general formula (I) are methyl; ethyl; propyl, isopropyl; n-butyl; isobutyl; tert-butyl; n-pentyl; isopentyl; n-hexyl; tert-hexyl; octyl; tert-octyl; decyl; dodecyl; octadecyl; ethenyl; propenyl; butenyl; isobutenyl; hexenyl; octenyl; cyclohexyl; propylcyclohexyl; butylcyclohexyl; decylcyclohexyl; hydroxycyclohexyl; hydroxyethylcyclohexyl; 2-hydroxyethyl; 2-hydroxypropyl; 3-hydroxypropyl; 3-hydroxybutyl; 4-hydroxybutyl; 3-hydroxypentyl; 5-hydroxypentyl; 6-hydroxyhexyl; 3-hydroxy-2,5-dimethylhexyl; 7-hydroxyheptyl; 7-hydroxyoctyl; 2-methoxyethyl; 2-methoxypropyl; 3-methoxypropyl; 4-methoxybutyl; 6-methoxyhexyl; 7-methoxyheptyl; 7-methoxyoctyl; 2-ethoxyethyl; 3-ethoxypropyl; 4-ethoxybutyl; 4-propoxypropyl; 3-butoxypropyl; 4-butoxybutyl; 4-isobutoxybutyl; 5-propoxypentyl; 2-cyclohexyloxyethyl; 2-ethenyloxyethyl; 2-(N,N-dimethylamino)ethyl; 3-(N,N-dimethylamino)propyl; 4-(N,N-dimethylamino)butyl; 5-(N,N- dimethylamino)pentyl; 4-(N,N-diethylamino)butyl; 5-(N,N-diethylamino)pentyl; 5-(N,N-diisopropylamino)pentyl; 3-(N-ethylamino)-propyl; 4-(N-methylamino)butyl; 4-(N,N-dipropylamino)butyl; 2-(N,N-diisopropylamino)ethyl; 6-(N-hexenylamino)hexyl; 2-(N-ethenylamino)ethyl; 2-(N-cyclohexylamino)ethyl; 2-(N-2-hydroxyethylamino)ethyl; 2-(2-hydroxyethoxy)ethyl; 2-(2-methoxyethoxy)ethyl; 6-(N-propylamino)hexyl.

Examples of divalent radicals -Z- are those which are obtained by removing a hydrogen from each amino group of the following diamino compounds: piperazine; 2-methylpiperazine; 2,5-dimethylpiperazine; 2,3,5,6-tetramethylpiperazine; 2-ethylpiperazine; 2,5-diethylpiperazine; 1,2-diaminoethane; 1,3-diaminopropane; 1,4-diaminobutane; 1,5-diaminopentane; 1,6-diaminohexane; 1,8-diaminooctane; 1,10-diaminodecane; 1,12-diaminododecane; N,N'-dimethyl-1,2-diaminoethane; N-methyl-1,3-diaminopropane; N-ethyl-1,2-diaminoethane; N-isopropyl-1,2-diaminoethane; N-(2-hydroxyethyl)-1,2-diaminoethane; N,N'-bis(2-hydroxyethyl)-1,2-diaminoethane; N-(2-hydroxyethyl)-1,3-diaminopropane; N-hexenyl-1,6-diaminohexane; N,N'-diethyl-1,4-diamino-2-butene; 2,5-diamino-3-hexene; 2-aminoethylether; (2-aminoethoxy)methylether; 1,2-bis(2-aminoethoxy)ethane; 1,3-diaminobenzene; 1,4-diaminobenzene; 2,4-diaminotoluene; 2,4-diaminoanisole; 2,4-diaminophenol; 4-aminophenylether; 4,4'-methylenedianiline; 4,4'-diaminobenzanilide; 3-aminophenylsulfone; 4-aminophenylsulphone; 4-aminophenylsulfoxide; 4-aminophenyldisulfide; 1,3-bis(aminomethyl)benzene; 1,4-bis-(aminomethyl)benzene; 1,3-bis(aminomethyl)cyclohexane; 1,8-diamino-p-menthane; 1,4-bis(2-aminoethyl)piperazine; 1,4-bis(3-aminopropyl)piperazine; 1,4-bis(4-aminobutyl)piperazine; 1,4-bis(5-aminopentyl)piperazine.

Examples of polyvalent radicals of formula 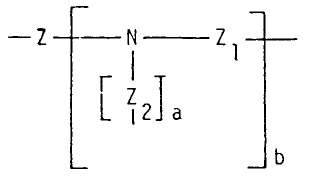
are those obtained by removing a hydrogen atom from each reacted amino group of the following polyamino compounds: bis(2-aminoethyl)amine; bis(3-aminopropyl)amine; bis(4-aminobutyl)amine; bis(5-aminopentyl)amine; bis[2-(N-methylamino)-ethyl]amine; 2-N-butyl-bis(2-aminoethyl)amine; bis[3-(N-methylamino)propyl]amine; N-(3-aminopropyl)-1,4-diaminobutane; N-(3-aminopropyl)-1,5-diaminopentane; N-(4-aminobutyl)-1,5-diaminopentane; tris(2-aminoethyl)amine; tris(3-aminopropyl)-amine; tris(4-aminobutyl)amine; tris[2-(N-ethylamino)ethyl]-amine; N,N'-bis(2-aminoethyl)-1,2-diaminoethane; N,N'-bis(3-aminopropyl)-1,3-diaminopropane; N,N'-bis(2-aminoethyl)-1,3-diaminopropane; N,N'-bis(3-aminopropyl)-1,2-diaminoethane; N,N'-bis(3-aminopropyl)-1,4-diaminobutane; bis[2-(2-aminoethyl)aminoethyl]amine;N,N'-bis[2-(2-aminoethyl)aminoethyl]-1,2-diaminoethane;N,N'-bis[3-(2-aminoethyl)aminopropyl]-1,2-diaminoethane; N,N,N',N'-tetrakis(2-aminoethyl)-1,2-diaminoethane.

Specific compounds covered by general formula (I) are given in the examples following the present description and in Table 1 below.

Compounds of general formula (I) can be prepared from, e.g., the intermediates of general formula (XIV): 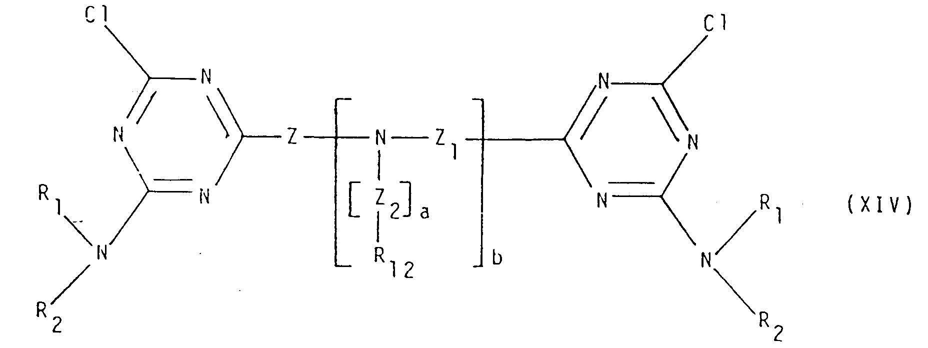
wherein R₁, R₂, Z, Z₁, Z₂, a and b are as defined above and R₁₂ is hydrogen or a group of formula 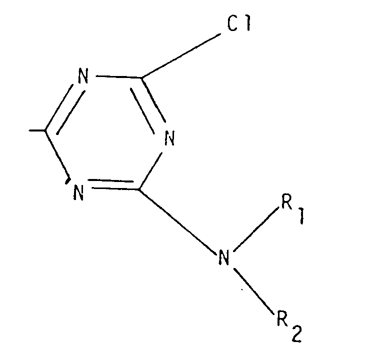
and its meaning may vary within each repeating unit; according to the following procedures:
a) when R is hydrogen: by hydrolysis with either an acid (such as hydrochloric acid, hydrobromic acid, sulfuric acid, phosphoric acid, etc.) at temperatures of from about 60 to about 100°C or a base (such as sodium hydroxide, potassium hydroxide, etc.) at temperatures of from about 100 to about 180°C;
b) when R is different from hydrogen: by condensation with a reagent of general formula (XV):

   R-OH (XV)

   wherein R is as defined above (R ≠ H); in a suitable solvent (such as toluene, xylene, ortho-dichlorobenzene, etc.) and/or in an excess of reagent (XV) if the latter can act as solvent (as in the case of, e.g., methanol, ethanol etc.) and in the presence of a base (such as sodium hydroxide, potassium hydroxide, sodium metal, etc.) at temperatures of from about 60 to about 150°C.

The product formed can easily be separated from the reaction mass by filtration.

Generally, products of general formula (I) of good quality are obtained in the form of a white, crystalline powder which can be used in self-extinguishing polymeric compositions without any further purification.

The intermediates of general formula (XIV) can easily be synthesized by reacting, at temperatures of from about 0 to about 10°C and at a pH of from about 5 to about 7, (2+b) or less moles of a cyanuric acid halide, for instance the chloride, in a suitable solvent (such as acetone, water, methylene chloride, etc.) with one mol of polyamine of general formula (XVI): 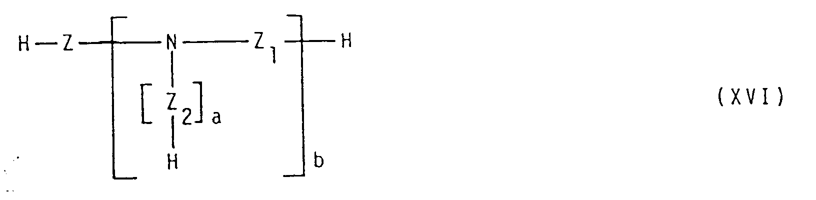
wherein Z, Z₁, Z₂, a and b are as defined above; to afford the intermediate of general formula (XVII): 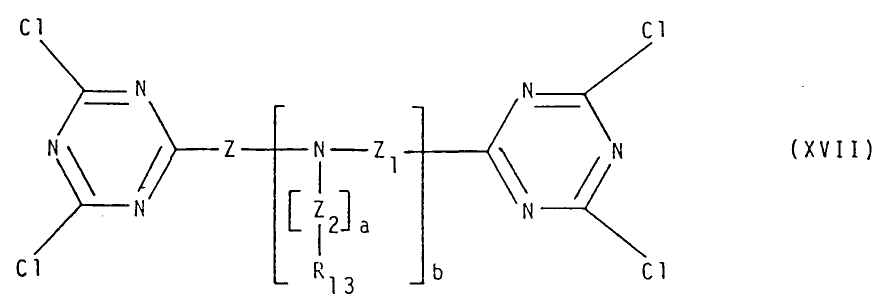
wherein R₁₃ is hydrogen or a group of formula 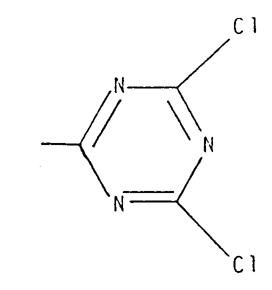
and its meaning can vary within each repeating unit.

This intermediate, either separated or not, is allowed to react again with (2+b) or less mols or an amine of general formula (XVIII): 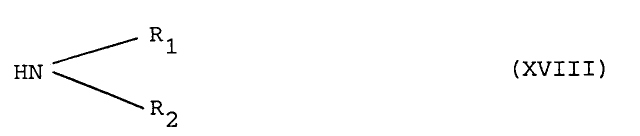
wherein R₁ and R₂ have the previously defined meanings; under conditions analogous to the preceding ones, but at temperatures of from about 10 to about 60°C.

A synthetic variant consists in inverting the order of addition of the reagents, i.e., in first reacting a cyanuric acid halide, for instance the chloride, under conditions analogous to the preceding ones, at temperatures of from about 0 to about 10°C, with an amine of general formula (XVIII) to yield the intermediate of general formula (XIX): 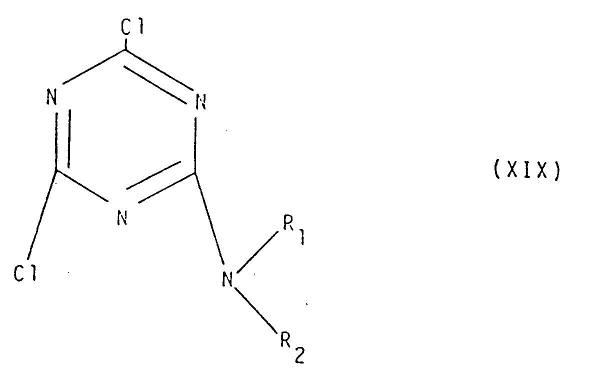
(2+b) moles or less of which, either separated or not, are then allowed to react, with one mol of a polyamine of general formula (XVI), under conditions analogous to the preceding ones, but at temperatures of from about 10 to about 60°C.

An alternative method for obtaining compunds of general formula (I) wherein R is different from hydrogen, comprises the reaction of a cyanuric acid halide, for instance the chloride, with a reagent of general formula (XV), at temperatures of from about 10 to about 110°C, in a suitable solvent (such as acetone, methylene chloride, toluene, xylene, etc.) and/or in an excess of the reagent (XV) if the latter can act as solvent (as in the case of, e.g., methanol, ethanol, etc.), and in the presence of an acidity acceptor (such as NaHCO₃, NaOH, Na₂CO₃, triethylamine, collidine, etc.) thereby obtaining the intermediate of general formula (XX): 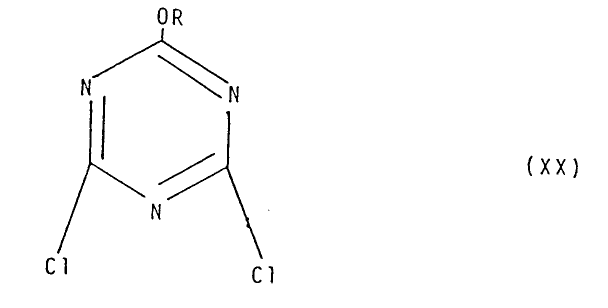
wherein R has the previously defined meanings.

This intermediate, either separated or not, is allowed to react with an amine of general formula (XVIII) under conditions analogous to the preceding ones, but at temperatures of from about -5 to about 30°C, to give the intermediate of general formula (XXI): 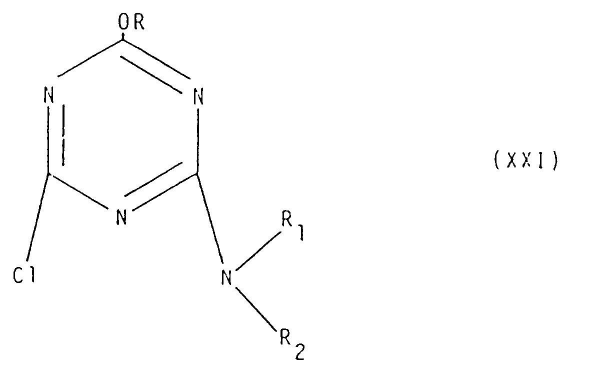
wherein R, R₁ and R₂ have the previously defined meanings.

(2+b) or less mols of this intermediate, either separated or not, are reacted with one mol of a polyamine of general formula (XVI), under conditions analogous to the preceding ones, but at temperatures of from about 40 to about 140°C.

From compounds of general formula (I) wherein R is different from hydrogen (and preferably is a (C₁-C₄)-alkyl group, it is possible to obtain the corresponding compounds wherein R is hydrogen by hydrolysis, either with an acid at temperatures of from about 80 to about 140°C or with a base at temperatures of from about 100 to about 180°C, using the same reagents as given for the hydrolysis of intermediates of general formula (XIV).

Furthermore, the present invention provides self-extinguishing polymeric compositions comprising:
a) from 91 to 40 (preferably 88 to 50) parts by weight of thermoplastic polymer and/or of polymer endowed with elastomeric properties;
b) from 6 to 33 (preferably 8 to 30) parts by weight of one or more ammonium and/or amine phosphates and/or phosphonates;
c) from 3 to 27 (preferably 4 to 20) parts by weight of one or more compounds of general formula (I) as defined above.

Particularly preferred are compounds of general formula (I) wherein R is hydrogen.

Among the phosphates, ammonium polyphosphates of the general formula

(NH₄)ₙ₊₂PₙO₃ₙ₊₁

wherein n is an integer equal to, or greater than, 2, are preferred. The molecular weight of the polyphosphates should be high enough to secure a low solubility in water.

For exemplary purposes, n preferably is comprised within the range of from 2 to 500.

The composition of the polyphosphates having the formula indicated hereinabove, in which n is a high enough number, and preferably ranges from 5 to 500, practically corresponds to a metaphosphate of formula

(NH₄PO₃)ₙ.

An example of said polyphosphates is the product known under the trade name "Exolit ® 422" (Hoechst), having the composition (NH₄PO₃)ₙ in which n is greater than 50. Another example is the product known under the name "Phos-Chek ® P/30" (Monsanto Chemical) which has a similar composition.

Another polyphosphate which can be used advantageously, above all thanks to its low solubility in water, is the product known under the trade name "Exolit ® 462" (Hoechst) which is "Exolit ® 422" microencapsulated in melamine-formaldehyde resin.

Other phosphates which can be used are those which are derived from amines, such as, e.g., dimethyl ammonium phosphate or diethyl ammonium phosphate, ethylene diamine phosphate, melamine orthophosphate or melamine pyrophosphate.

Good results may also be obtained by using mono- or polyammonium phosphonates selected from the salts derived from mono-or polyphosphonic acids.

Examples of said acids are:
ethane-1,1,2-triphosphonic acid, 2-hydroxy-ethane-1,1,2-triphosphonic acid, propane-1,2,3-triphosphonic acid, methylphosphonic acid, ethylphosphonic acid, n-propylphosphonic acid, n-butylphosphonic acid, phenylphosphonic acid, 1-amino-ethane-1,1-diphosphonic acid, 1-hydroxy-ethane-1,1-diphosphonic acid, 1-hydroxy-dodecane-1,1-diphosphonic acid, phosphonoacetic acid, 2-phosphonopropionic acid, 3-phosphonopropionic acid, 2-phosphonobutyric acid, 4-phosphonobutyric acid, amine tri(methylenephosphonic) acid, ethylenediamine tetra(methylenephosphonic) acid, hexamethylene diamine tetra(methylenephosphonic) acid, diethylene triamine penta(methylenephosphonic) acid.

Among the polymers which can be used in the compositions according to the present invention, preferred are polymers and copolymers of olefins of general formula

R' - CH = CH₂

wherein R' is hydrogen or a (C₁₋₈)-alkyl or (C₆₋₁₀)-aryl radical, in particular:
(1) isotactic or prevailingly isotactic polypropylene;
(2) HDPE, LLDPE and LDPE polyethylene;
(3) crystalline copolymers of propylene and minor amounts of ethylene and/or of other alpha-olefins, such as, e.g., 1-butene, 1-hexene, 1-octene, 4-methyl-1-pentene;
(4) heterophasic compositions comprising (A) a fraction constituted by a propylene homopolymer, or by one of the copolymers defined under (3); and (B) a copolymeric fraction constituted by elastomeric copolymers of ethylene and an alpha-olefin, optionally containing minor proportions of one or more dienes, wherein the alpha-olefin is preferably selected from propylene and 1-butene; and
(5) elastomeric copolymers of ethylene and alpha-olefin(s), optionally containing minor proportions of one or more dienes.

Examples of dienes more commonly contained in said elastomeric copolymers are butadiene, ethylidene-norbornene and hexadiene-1,4.

Among the polymers of olefins of general formula R'-CH=CH₂ wherein R' is an aryl radical, "crystal" and impact-resistant polystyrene are preferred.

Other examples of polymers which can be used are acrylonitrile/butadiene/styrene terpolymers (ABS); styrene/acrylonitrile copolymers (SAN); polyurethanes (of polyester or polyether grade); poly(ethylene terephthalate); poly(butylene terephthalate); polyamides.

The self-extinguishing compositions according to the present invention can be prepared according to methods known in the art. For example, ammonium and/or amine phosphate and/or phosphonate is first intimately mixed with one or more finely ground nitrogen-containing compounds of general formula (I) (the particles of which generally are smaller than 70 µm) and the thus obtained mixture is added to the polymer in a turbo-mixer, in order to generate a homogeneous mixture which is either extruded or granulated. The thus obtained granular product can be transformed into various finished articles according to any of the well-known moulding techniques.

The fire-retardant additives according to the present invention are also suitable for use in the field of flame-retardant paints.

Examples of ameline derivatives covered by general formula (I), which are not mentioned in the examples but are equally advantageously useable in self-extinguishing polymeric compositions of the present invention are those reported in Table 1 below, wherein R₃, when present, is a triazine ring of formula

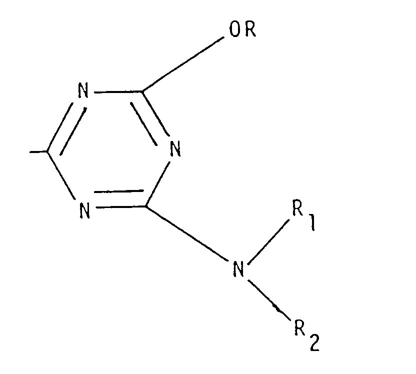

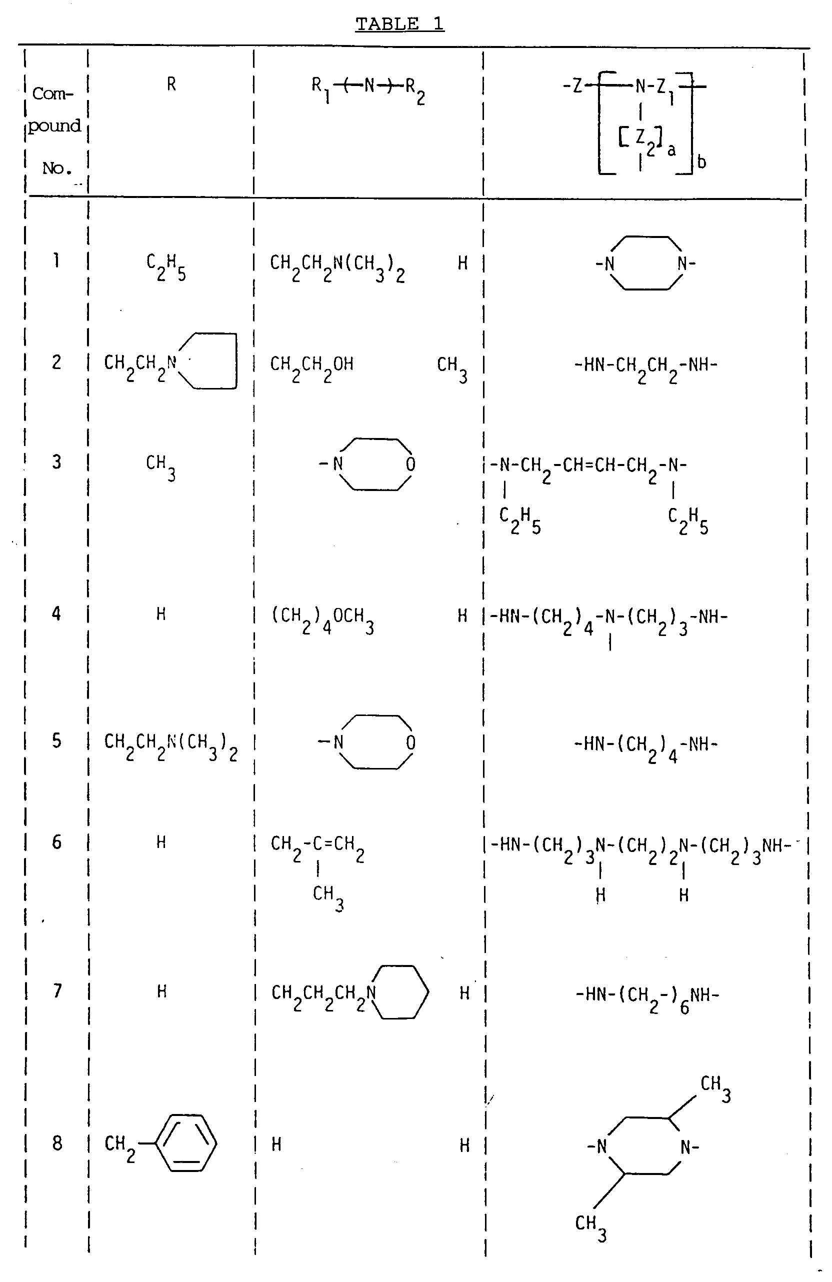

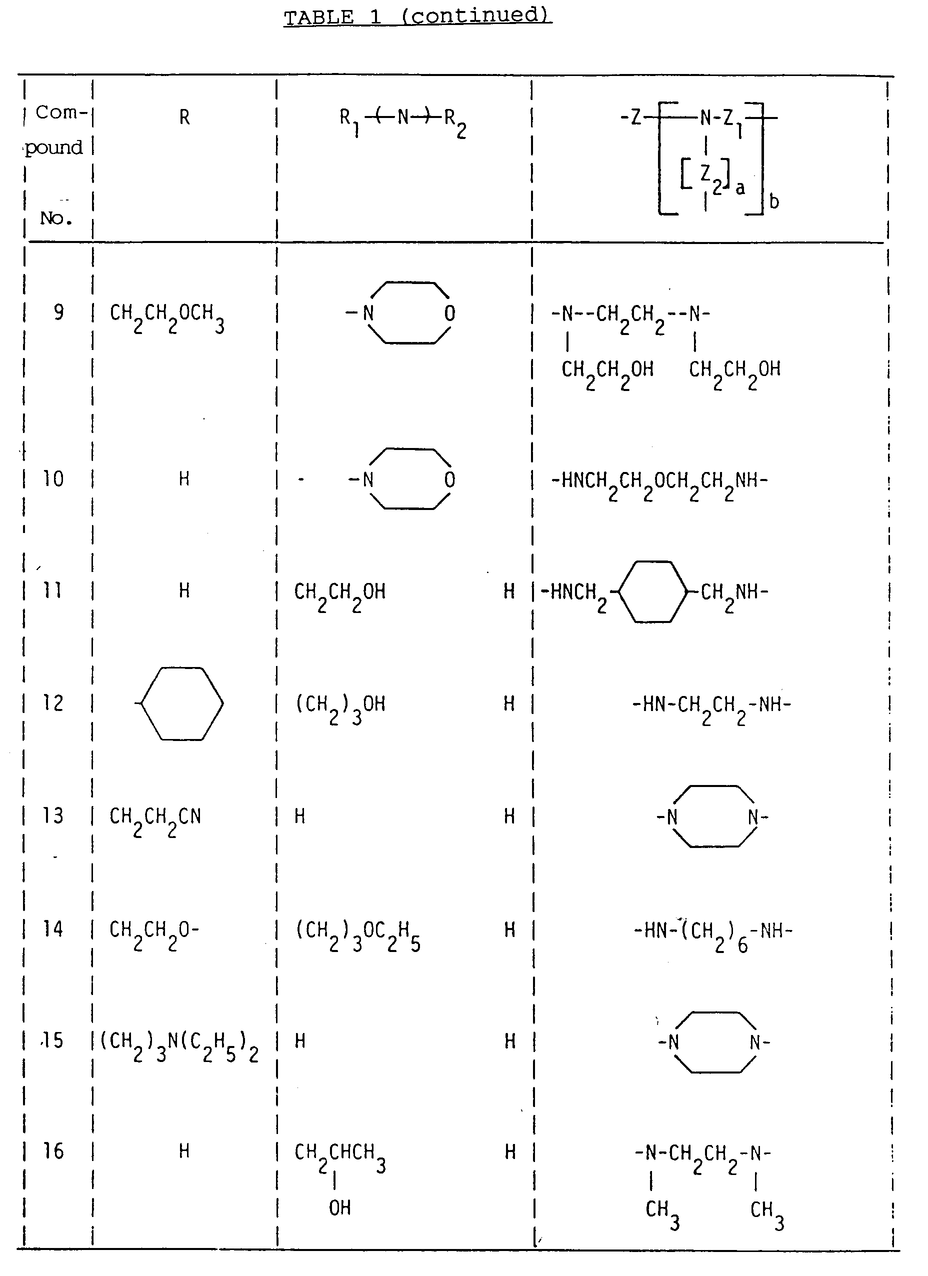

### EXAMPLE 1

Into a 2 l-reactor equipped with stirrer, thermometer, feeding funnel, condenser and cooling bath, 600 ml of acetone and 184.5 g of cyanuric acid chloride are introduced.

While externally cooling to 0-5°C, 42.6 g of piperazine, dissolved in 400 ml of acetone, are added within one hour.

Always at 0-5°C, 40 g of sodium hydroxide in 200 ml of water are then added within about 2 hours.

The resulting mixture is kept under stirring for a further 4 hours at 5°C, then 400 ml of cold water are added and the precipitate formed is filtered and washed on the filter with water.

Upon drying of the filter cake in an oven at 100°C, 177.4 g of the intermediate of formula (XXII): 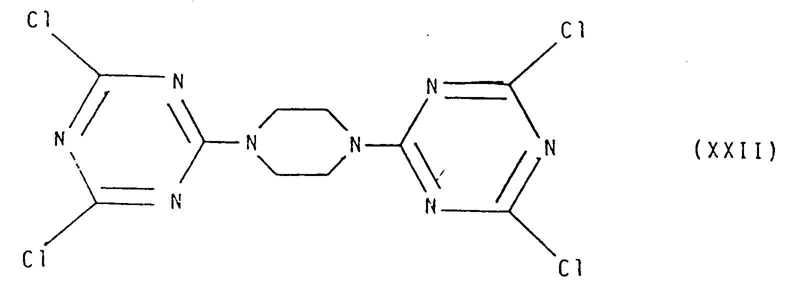
are obtained in the form of a white crystalline powder having a melting point (m.p.) of >300°C and a chlorine content of 37.35% (theor.: 37.17%).

In the same 2 l-reactor, but equipped with heating bath, 800 ml of xylene and 152.8 g of the intermediate of formula (XXII) are introduced.

The mixture is heated to 50°C and thereafter, within 4 hours, first 48.8 g of 2-hydroxyethylamine and then 32 g of sodium hydroxide in 100 ml of water are added.

The resulting mixture is kept under stirring at 50-55°C for a further 4 hours and then is cooled to room temperature. The product formed is filtered and washed thoroughly on the filter with water.

After drying of the filter cake in an oven at 100°C, 146.7 g of the intermediate of formula (XXIII): 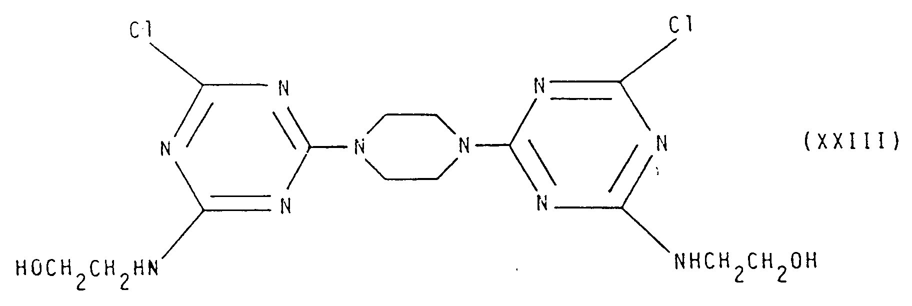
are obtained in the form of a white crystalline powder having a m.p. of >300°C and a chlorine content of 16.62% (theor.: 16.47%).

The structure of intermediates (XXII) and (XXIII) was confirmed by IR spectroscopic analysis.

In a 1 l-reactor, equipped as the preceding one, 500 ml of water, 86.2 g of the intermediate (XXIII) and 79.0 g of a 37% by weight hydrochloric acid solution are introduced.

The mass is heated to 95°C and is kept at this temperature for 6 hours, whereafter it is cooled to 80°C and 40 g of sodium hydroxide, dissolved in 100 ml of water, are added.

The resulting mixture is allowed to cool to room temperature and the product formed is filtered and washed on the filter with water.

By drying the cake in an oven at 100°C, 70.2 g of the product of formula 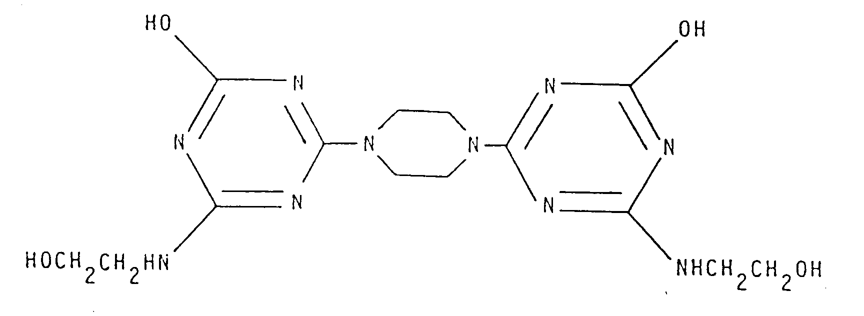
are obtained in the form of a white crystalline powder having a m.p. of >300°C.

### EXAMPLE 2

In the 2 l-reactor described in example 1, there are placed 500 ml of acetone and 300 ml of water.

After cooling to 0-5°C, 92.2 g of cyanuric acid chloride are introduced and thereafter, keeping the temperature at 3-5°C, 15 g of ethylene diamine dissolved in 100 ml of water are added within 2 hours.

The mixture is kept at 5°C for a further hour and then, within one hour and keeping the temperature at 5-7°C, 42 g of sodium bicarbonate are introduced. The resulting mixture is kept under stirring at 7-10°C for 2 hours and then is heated to 35°C and at this temperature, within one hour, 37.5 g of 2-methoxyethylamine dissolved in 100 ml of water are added.

Thereafter the temperature is raised to 40-45°C and within 2 hours 20 g of sodium hydroxide dissolved in 50 ml of water are added. After stirring the whole mixture for a further hour, the acetone is distilled off.

The resulting mass is subsequently heated to 80°C and 54.2 g of a 37% by weight hydrochloric acid solution are introduced.

The reaction mixture is heated to boiling and is maintained under reflux for 6 hours, whereafter it is cooled to 80°C and 42 g of sodium hydroxide, dissolved in 100 ml of water, are introduced.

Upon cooling to 50°C, the product formed is filtered and washed on the filter with water at 50°C.

By drying the filter cake in an oven at 100°C, 71.5 g of the product of formula 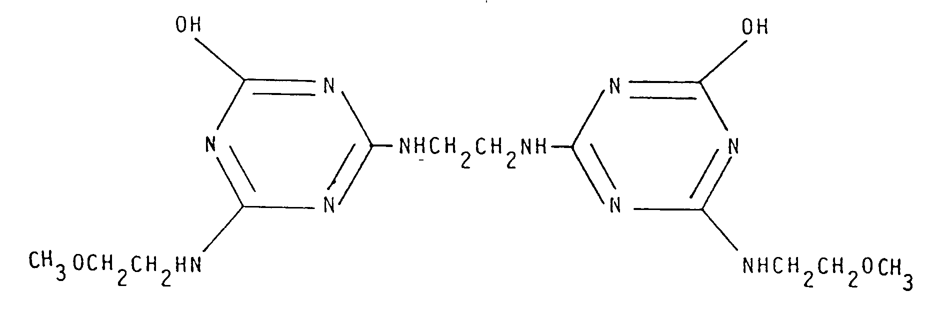
are obtained in the form of a white crystalline powder having a m.p. of >300°C.

### EXAMPLE 3

In the 2 l-reactor of example 1, 600 ml of methyl alcohol, 80 ml of water and 100.8 g of sodium bicarbonate are placed.

The mixture is cooled to 10°C and 110.7 g of the cyanuric acid chloride are added, whereafter the temperature is allowed to rise up to 30°C and is maintained at this value for about one hour, until the evolution of carbon dioxide is complete.

The heat of reaction is sufficient to maintain the desired temperature.

THe resulting mixture is cooled to 5°C and then 700 ml of cold water are added. The product formed is filtered and washed on the filter with cold water.

By drying the cake in an oven under vacuum at 50°C, 92.1 g of the intermediate of formula (XXIV): 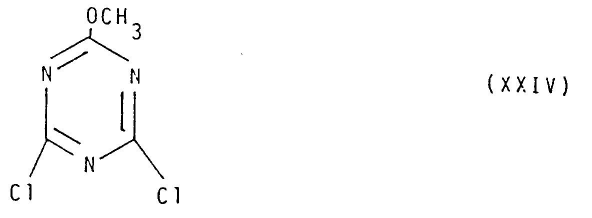
are obtained in the form of a white crystalline powder; m.p. = 90-92°C; chlorine content 39.27% (theor.: 39.44%).

In a 1 l-reactor equipped as the preceding one, 500 ml of methylene chloride and 90 g of the intermediate (XXIV) are introduced.

The mixture is externally cooled to 0-2°C and, within 2 hours, 43.2 g of morpholine are added.

Subsequently, always at 0-2°C and within 3 hours, 20 g of sodium hydroxide dissolved in 70 ml of water are introduced.

After having stirred the mixture for another hour, the aqueous phase is separated.

By distilling the methylene chloride, 112.8 g of the intermediate of formula (XXV): 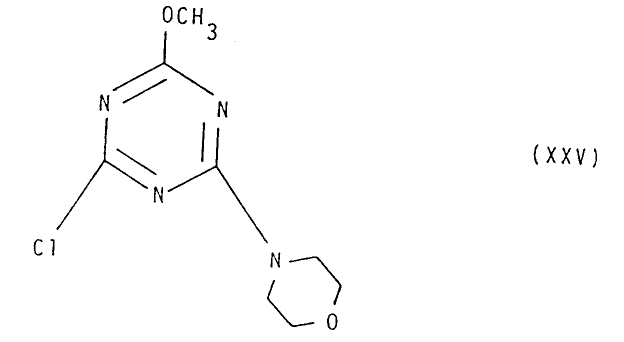
are obtained in the form of a white crystalline powder; m.p. = 107-108°C; chlorine content 15.28% (theor.: 15.40%).

The structure of intermediates (XXIV) and (XXV) was further confirmed by IR spectroscopic analysis.

In the same 1 l-reactor, 500 ml of acetonitrile, 69.2 g of the intermediate (XXV) and 10.3 g of diethylene triamine are placed.

The resulting mixture is heated to 80°C and within about 4 hours 31.8 g of sodium carbonate dissolved in 100 ml of water are added.

The whole mass is kept under reflux for 8 hours and thereafter the solvent is evaporated and 500 ml of water are added.

After stirring the mixture for 30 minutes the product formed is filtered and washed on the filter with water.

By drying the cake in an oven at 100°C, 61.2 g of the product of formula 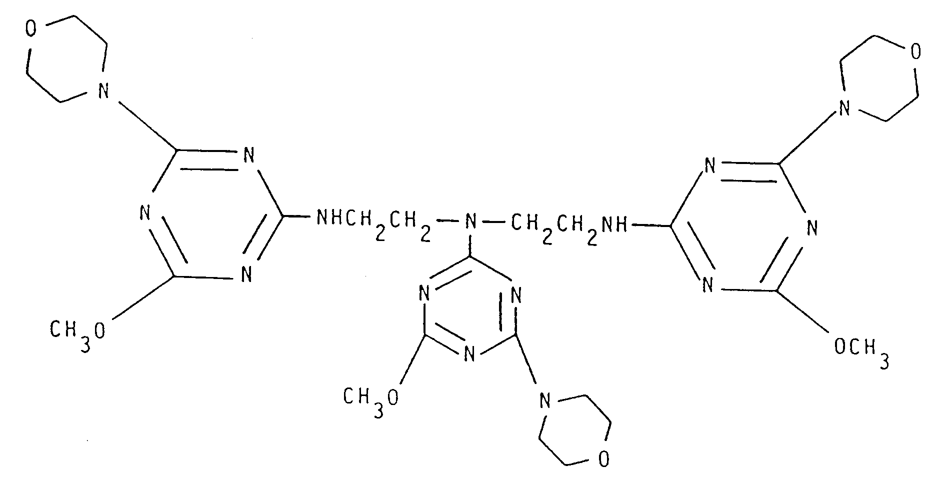
are obtained in the form of a white crystalline powder; m.p. = 212-214°C.

### EXAMPLE 4

In a 0.5 l-reactor, equipped as in the preceding example, 250 ml of water, 54.8 of the product of example 3 and 47.3 g of a 37% by weight hydrochloric acid solution are introduced.

After heating the resulting mixture to the boiling point it is maintained under reflux for 15 hours.

Then the whole mass is cooled to room temperature and 40.3 g of sodium bicarbonate are added, whereupon the product formed is filtered and washed on the filter with water.

By drying the cake in an oven at 100°C, 46.9 g of the product of formula: 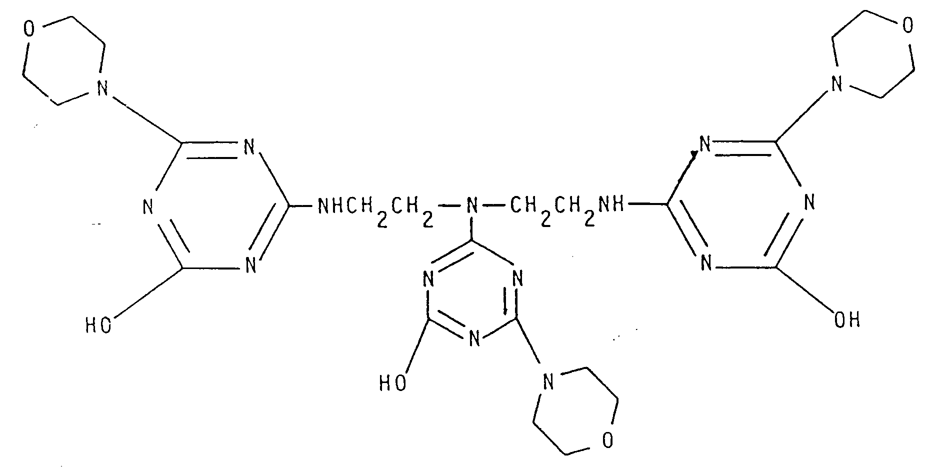
are obtained in the form of a white crystalline powder having a m.p. of 284-286°C.

### EXAMPLE 5

In a 1-liter reactor equipped as in the preceding examples, 600 ml of acetonitrile and 108 g of the intermediate (XXIV) are placed.

The resulting mixture is cooled to 5°C and within 2 hours, 102 g of of an aqueous ammonia solution (30% by weight) are added.

The whole mass is kept under agitation for another hour and then the product formed is filtered and washed on the filter with water.

After drying in an oven at 100°C, 84.2 g of the intermediate of formula (XXVI): 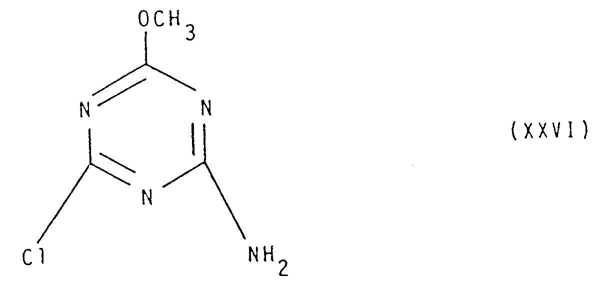
are obtained in the form of a white crystalline powder having a m.p. of >300°C and a chlorine content of 21.96% (theor. 22.12%).

The structure of the intermediate (XXVI) was confirmed by IR spectroscopic analysis.

In a 2 l-reactor equipped with stirrer, thermometer, feeding funnel, cooler and heating bath, 700 ml of acetonitrile, 80.3 g of the intermediate (XXVI) and 21.5 g of piperazine are introduced.

The resulting mixture is heated to boiling and within 4 hours a solution of 53 g of sodium carbonate in 200 ml of water is added.

The whole mass is kept under reflux for a further 5 hours, then 350 ml of water are added and the mixture is allowed to cool to room temperature.

The product formed is filtered and washed on the filter with water.

By drying the cake in an oven at 100°C, 83.2 g of the product of formula 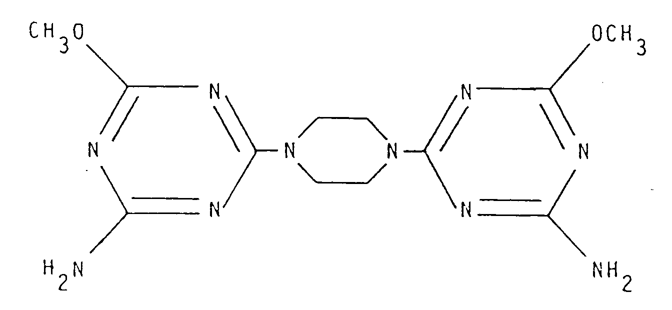
are obtained in the form of a white crystalline powder having a m.p. of >300°C.

### EXAMPLE 6

In the 2 l-reactor described in example 1, 184.5 g of cyanuric acid chloride and 1300 ml of methylene chloride are introduced.

While externally cooling, 87.2 g of morpholine and 40 g of sodium hydroxide dissolved in 150 g of water are simultaneously added within 3 hours, keeping the pH in the range of from 5 to 7 and the temperature in the range of from 0 to 3°C.

The whole mixture is kept at 0-3°C for a further 3 hours and thereafter the aqueous phase is separated.

By distilling the methylene chloride, 230 g of the intermediate of formula (XXVII): 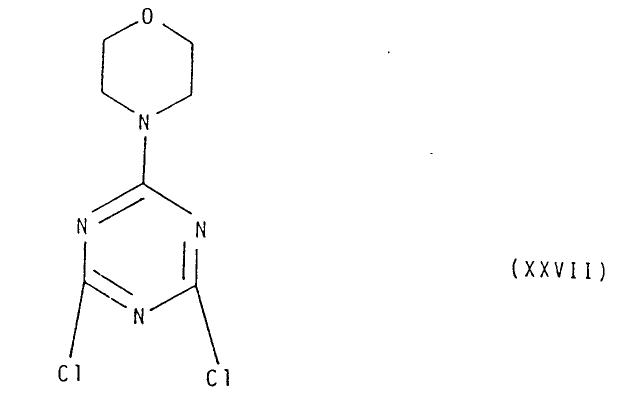
are obtained in the form of a white crystalline powder; m.p. = 155-157°C; chlorine content 30.12% (theor.: 30.21%).

In the same 2 l-reactor as above, but equipped with heating bath, 800 ml of xylene, 141 g of the intermediate (XXVII) and 25.8 g of piperazine are placed.

The mixture is heated to 60°C and within 3 hours 24 g of sodium hydroxide in 100 ml of water are added.

The resulting mixture is stirred at 60°C for a further 3 hours and thereafter is cooled to room temperature.

The product formed is filtered and the cake is thoroughly washed with water.

By drying the cake in an oven at 100°C, 126.4 g of the intermediate of formula (XXVIII): 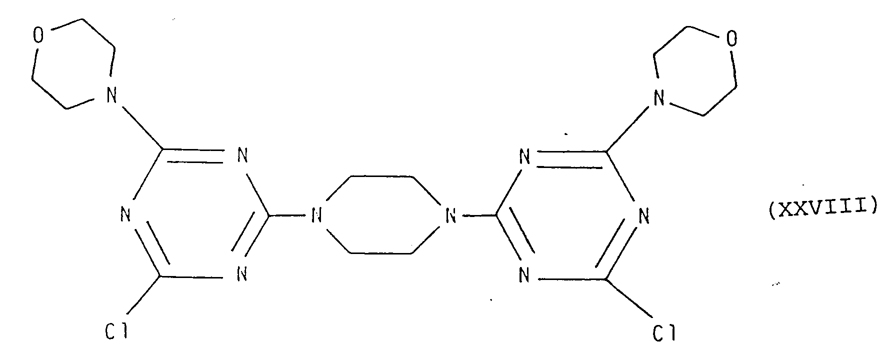
are obtained in the form of a white crystalline powder having a m.p. of >300°C and a chlorine content of 14.56% (theor.: 14.7%).

The structures of intermediates (XXVII) and (XXVIII) were further confirmed by IR spectroscopic analysis.

In a 1 l-reactor, equipped as described above, 500 ml of methanol are introduced and, while keeping the temperature at 15-20°C, 17.6 g of sodium hydroxide are added.

The resulting mixture is stirred until the dissolution of the sodium hydroxide is complete; thereafter 96.6 g of the intermediate (XXVIII) are added.

The resulting mixture is heated to boiling and is kept under reflux for about 10 hours, whereafter most of the solvent is distilled off (about 400 ml) and the distillation residue is treated with 300 ml of water.

The product formed is filtered and washed on the filter with water.

By drying the cake in an oven at 100°C. 87.4 g of the product of formula 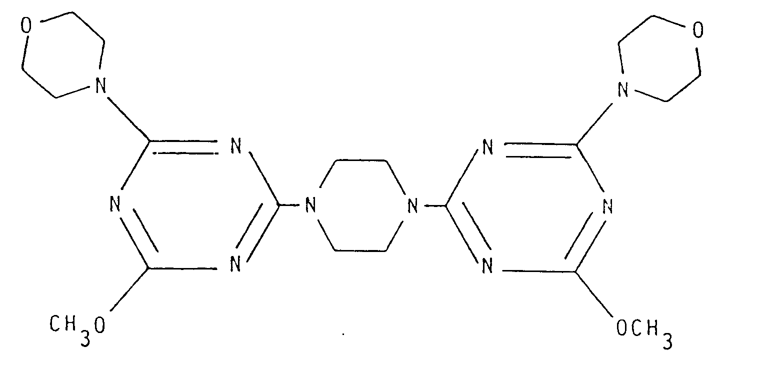
are obtained in the form of a white crystalline powder; m.p. = 268-270°C.

### EXAMPLE 7

In the same 2 l-reactor used in the preceding examples 500 ml of acetone and 300 ml of water are introduced.

While externally cooling to 0-5°C, first 110.7 g of cyanuric acid chloride and thereafter, within 2 hours, 29.2 g of tris-(2-aminoethyl)amine. dissolved in 100 ml of water, are added.

The resulting mixture is maintained at 5°C for a further hour and thereafter, within one hour and while keeping the temperature at 5-7°C, 50.4 g of sodium bicarbonate are added.

The resulting mixture is stirred for a further 3 hours at 7-10°C and then is heated to 35°C and at this temperature 61.8 g of thiomorpholine, dissolved in 100 ml of water, are introduced within one hour.

Then the temperature is raised to 40-45°C and a solution of 24 g of sodium hydroxide in 50 ml of water is added within about 2 hours. The whole mass is stirred for a further 2 hours and then the acetone is distilled off.

The distillation residue is transferred to a 1 liter steel reactor and then 26.4 g of sodium hydroxide are added thereto.

The resulting mixture is heated to 140°C and maintained at this temperature for about 12 hours, whereafter it is cooled to room temperature. The product formed is filtered and washed on the filter with water.

By drying the cake in an oven at 100°C, 132.8 g of the product of formula 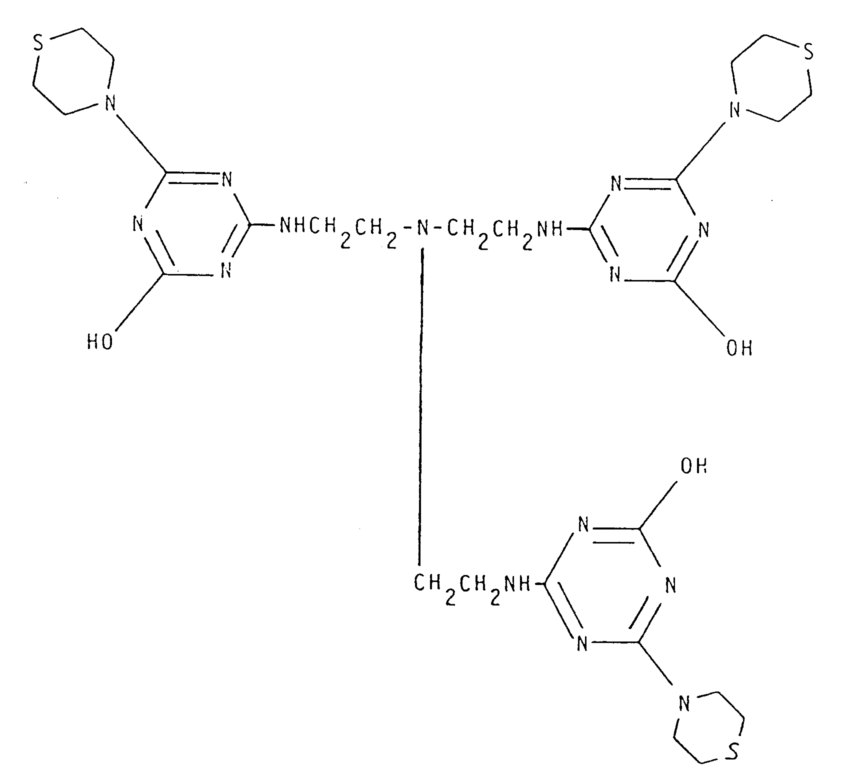
are obtained in the form of a white crystalline powder; m.p. = 237-239°C.

### EXAMPLES 8-34

By working under conditions analogous to those described in examples 1 to 7, the compounds of general formula (I) reported in Table 2 below are prepared. In these structures R₃, if present, is a triazine ring of the formula

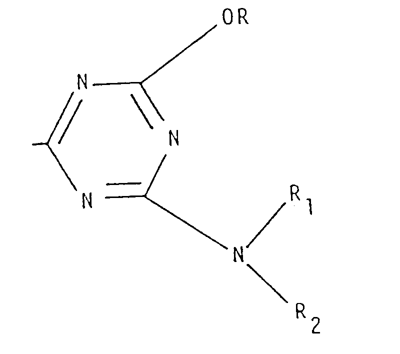

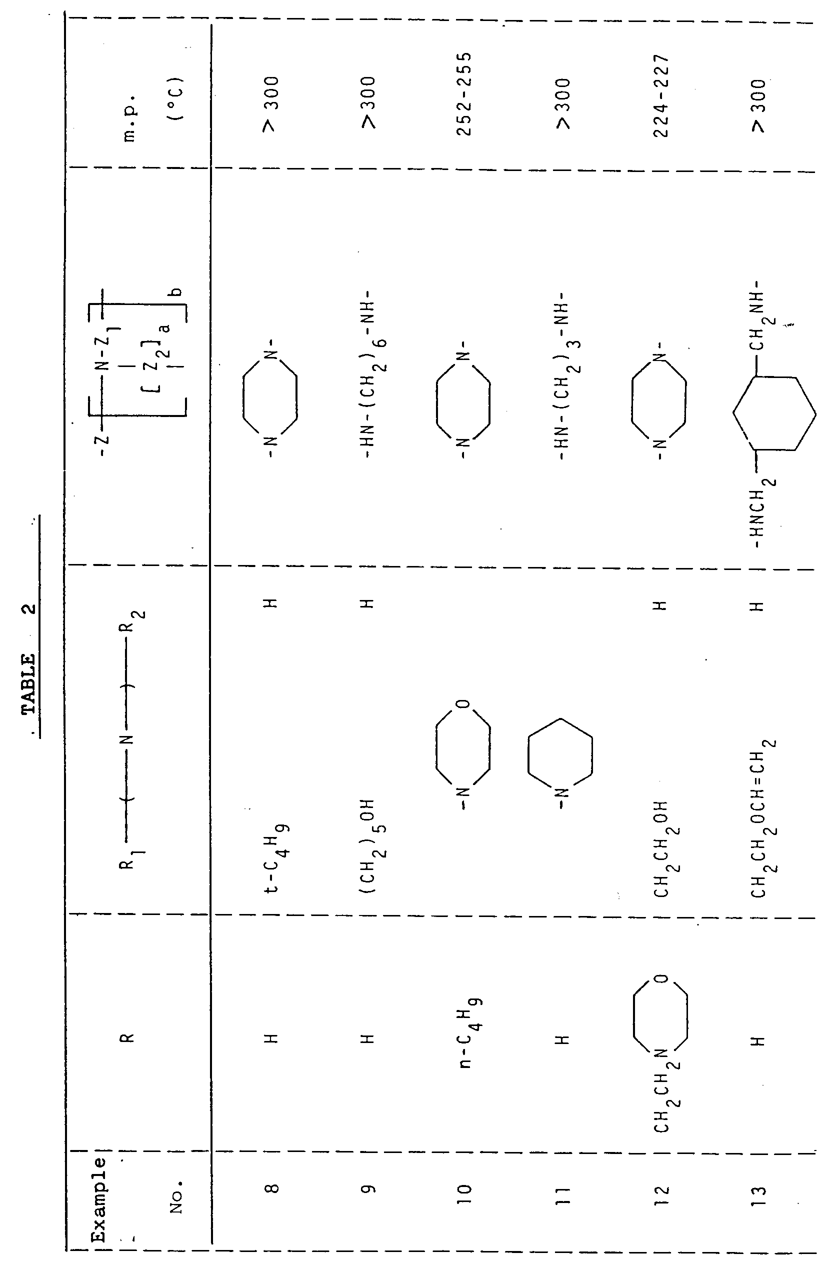

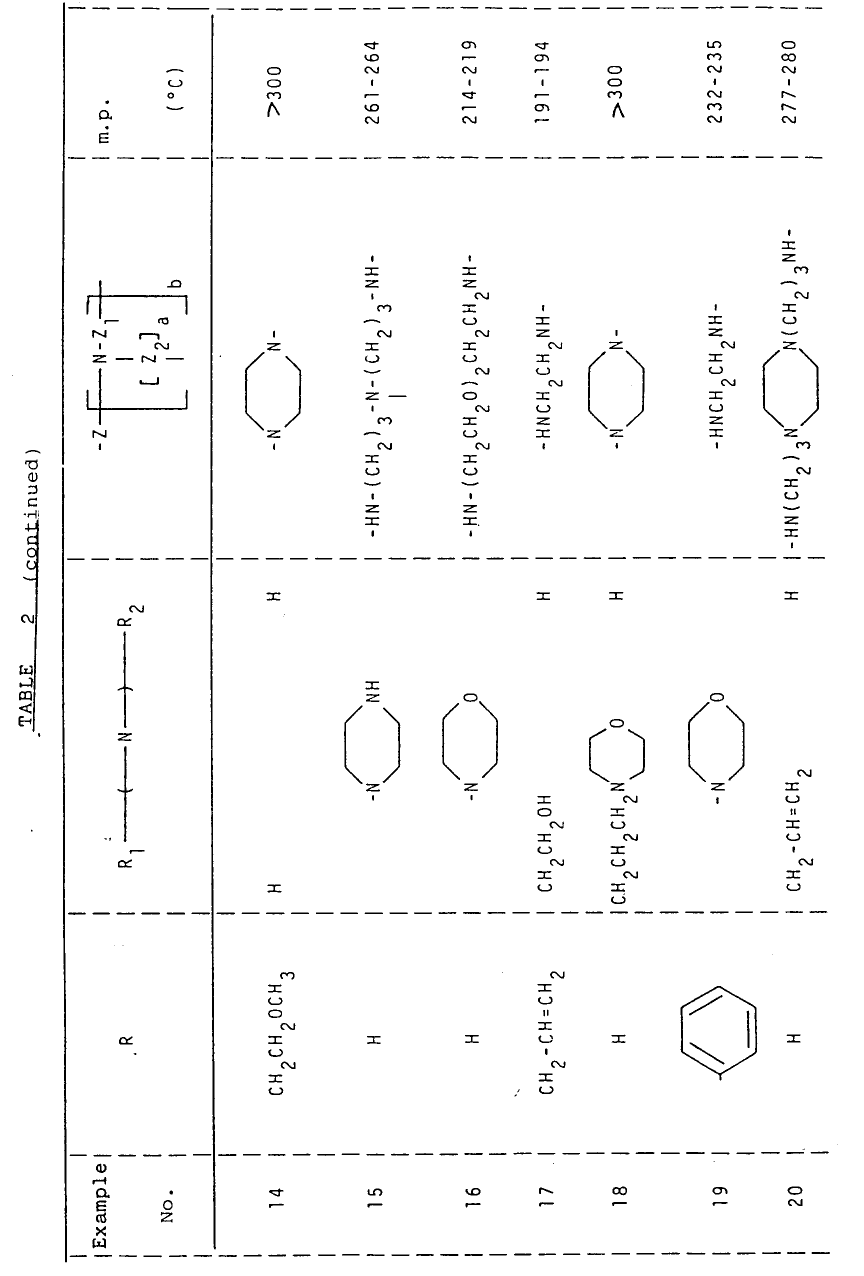

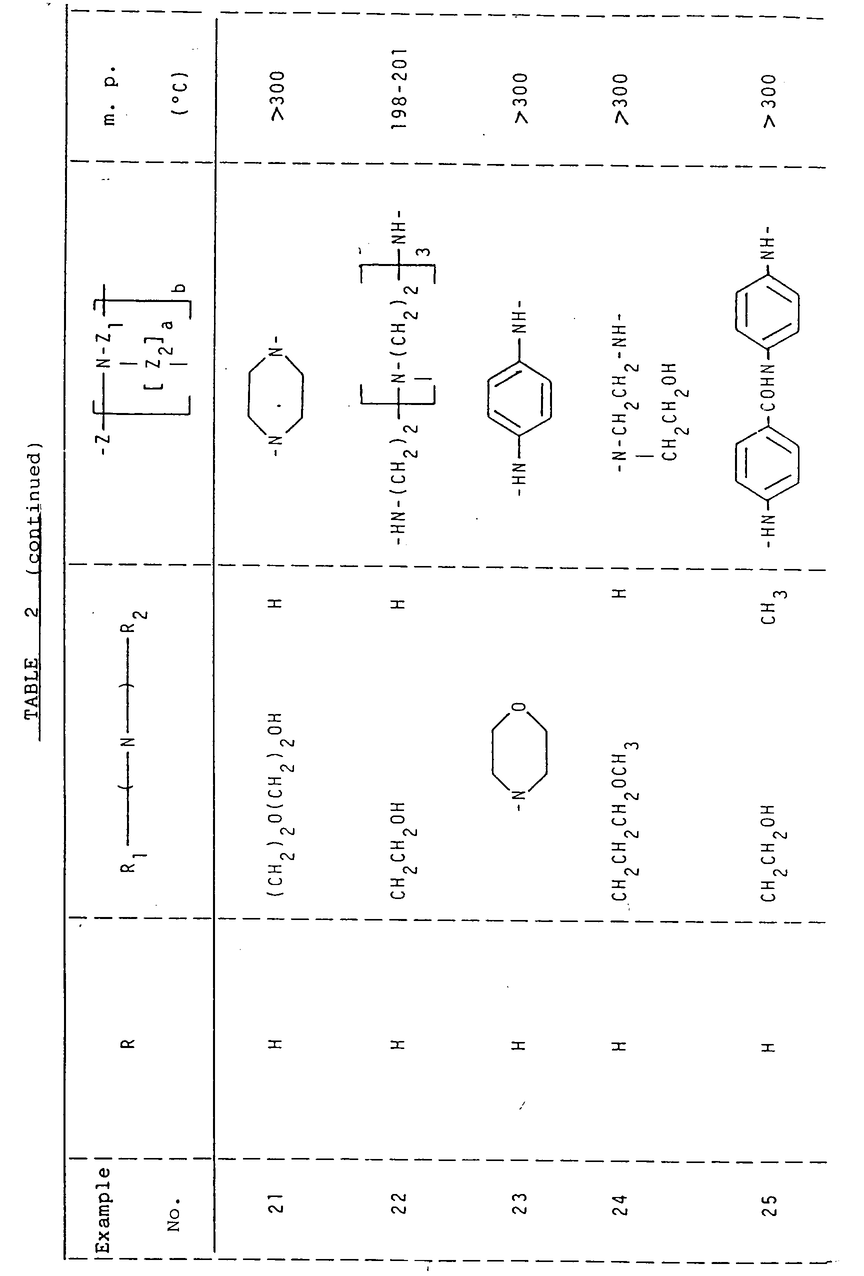

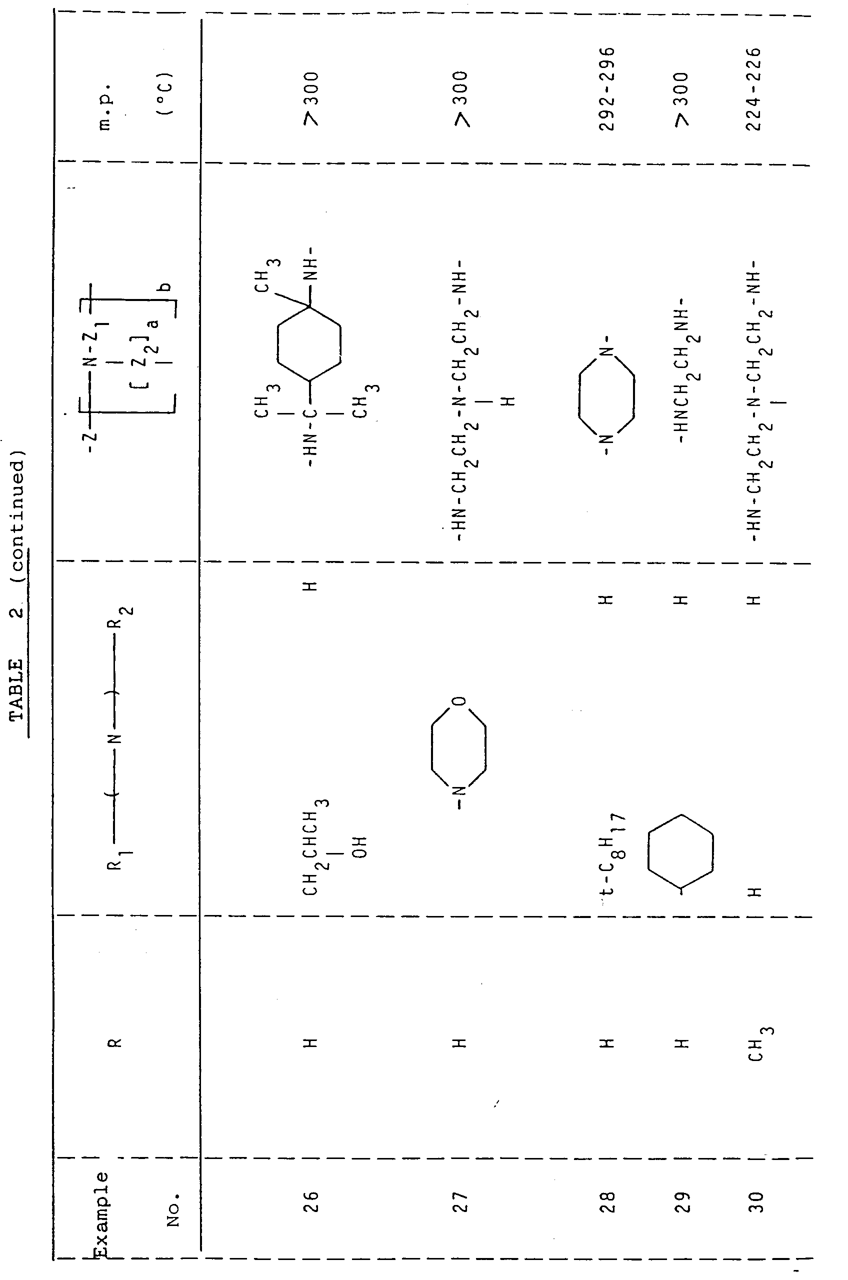

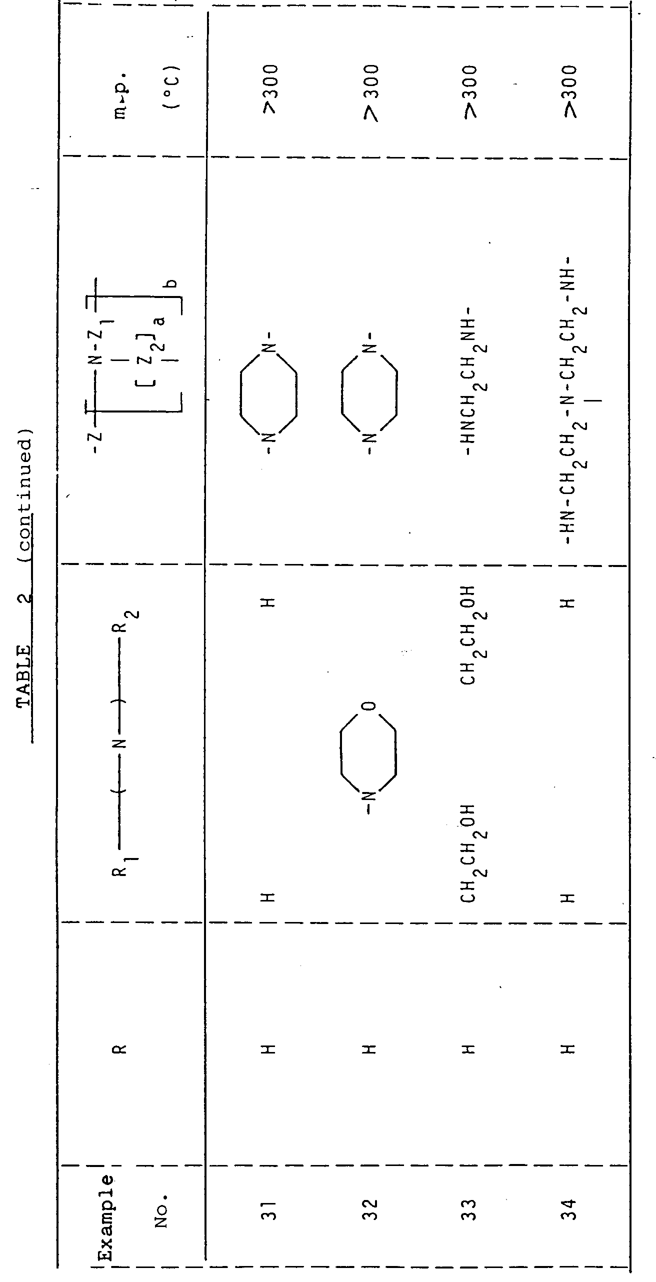

### EXAMPLES 35 TO 80

The tests reported in the following Tables 3 and 4 relate to polymeric compositions containing the products of general formula (I) prepared according to the preceding examples.

Specimen having the shape of small slabs of about 3 mm of thickness were prepared by moulding mixtures of granular polymer and additives on a MOORE platen press, with a moulding cycle of 7 minutes and a moulding pressure of about 40 kg/cm².

On the slab specimen thus obtained the level of self-extinguishment was determined by measuring the oxygen index (L.O.I. according to ASTM D-2863-77 in a Stanton Redcroft instrument) and by applying the "Vertical Burning Test" which makes it possible to classify the material according to three rating levels (94 V-O, 94 V-1 and 94 V-2) according to the UL 94 standard (issued by Underwriters Laboratories - U.S.A.).

In Table 3 the values which were obtained by using an isotactic polypropylene in flake form and having a melt flow index of 12 and an insoluble fraction in boiling n-heptane of 96% by weight are reported.

In Table 4 there are reported the values which were obtained by using low-density polyethylene in granular form with a melt flow index of 7; polystyrene granules containing 5% by weight of butadiene rubber and having a melt flow index of 9; thermoplastic polyurethane granules, of polyester grade (ESTANE 54600® by Goodrich) or of polyether grade (ESTANE 58300® by Goodrich), having respective specific gravities of 1.19 and 1.10 g/cm³; an ethylene-propylene elastomeric copolymer containing 45% by weight of propylene; and an acrylonitrile-butadiene-styrene terpolymer having a specific gravity of 1.06 g/cm³ and a melt flow index of 1.6, and containing about 40% of each acrylonitrile and styrene and 20% butadiene.

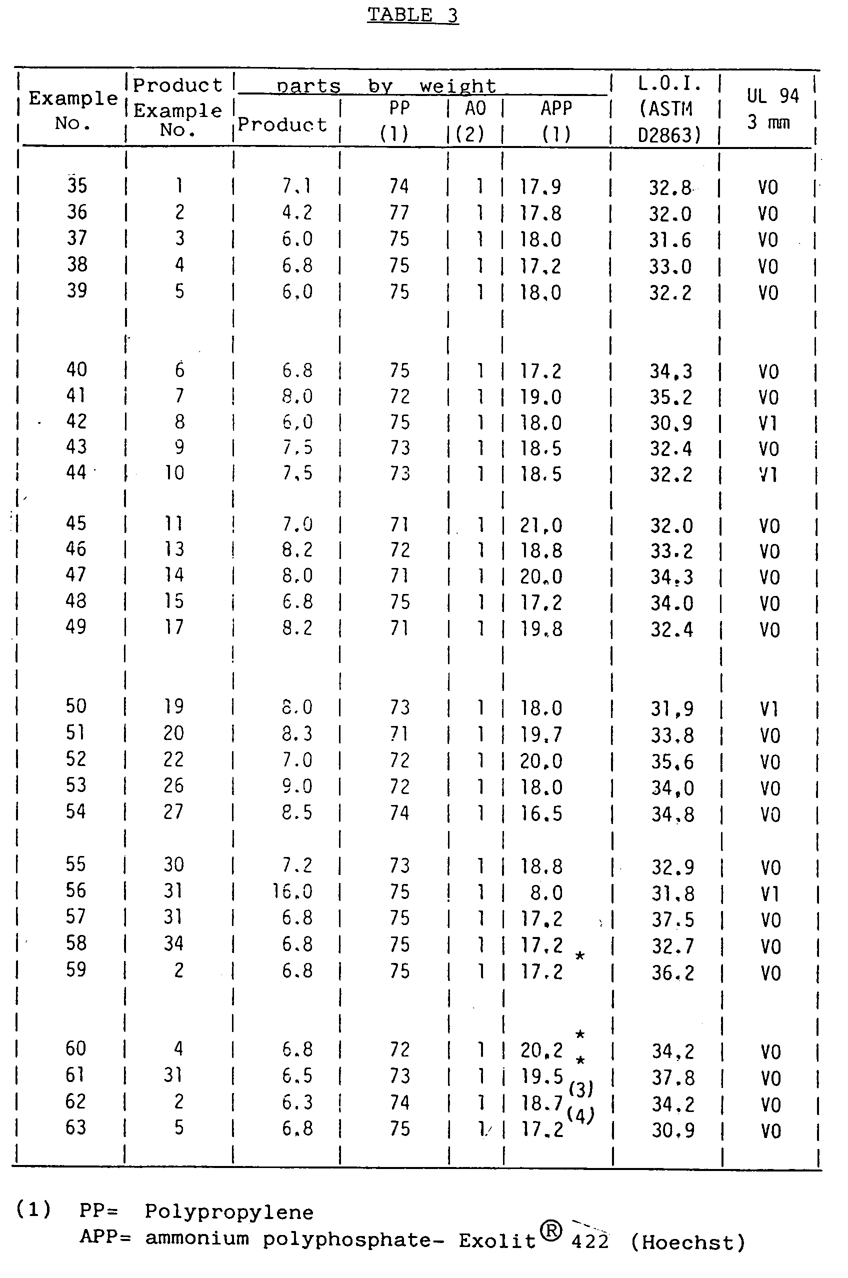

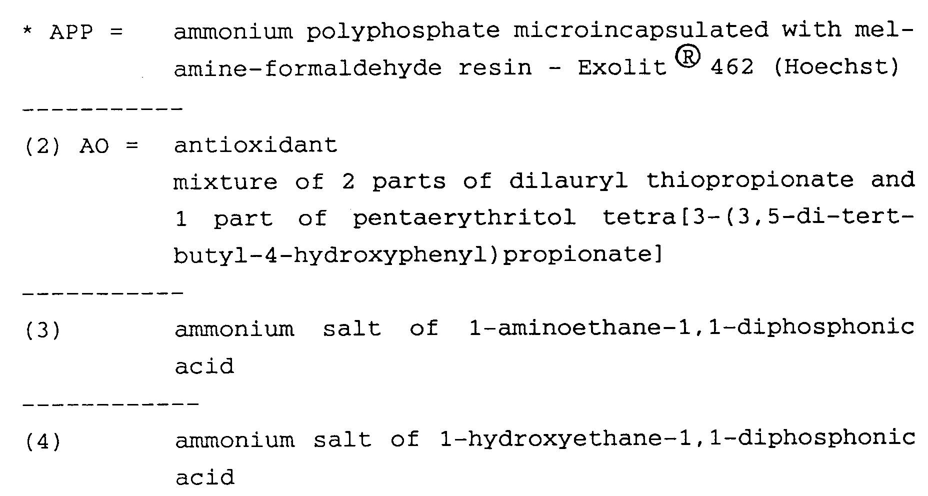

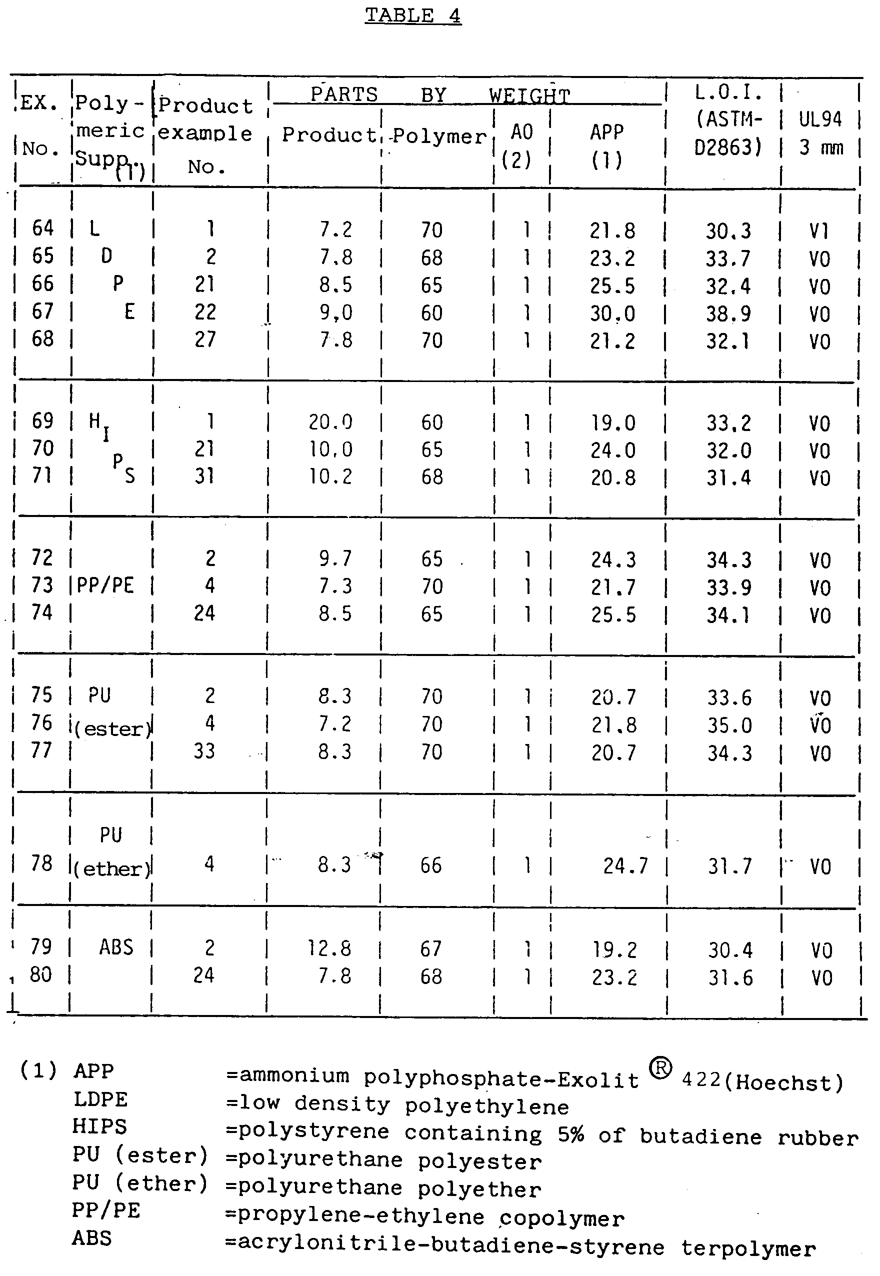

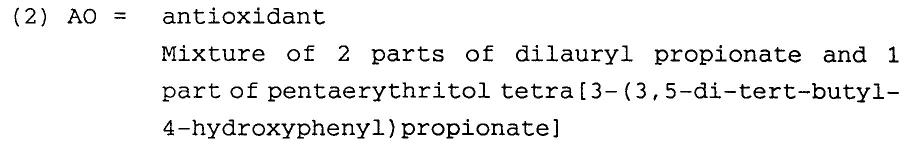

### EXAMPLE 81 (comparison example)

By working according to the procedure used in examples 35 to 63, but using as nitrogen containing compound 2,4-diamino-6-hydroxy-1,3,5-triazine, the composition specified hereinafter is prepared:

| | |
|---|---|
| Polypropylene | 72 parts by weight |
| Antioxidant | 1 part by weight |
| Ammonium polyphosphate | 19.3 parts by weight |
| 2,4-diamino-6-hydroxy-1,3,5-triazine | 7.7 parts by weight |

Using the above mentioned composition specimens are prepared which are subjected to the self-extinguishing tests described above.

The following results are obtained:
- L.O.I.: = 23.8
- UL 94 (3 mm):: class B (the specimen burns).

## Claims (Claims for the following Contracting State(s): AT, BE, CH, DE, FR, GB, LI, NL, SE)

1. Ameline derivatives of general formula (I): 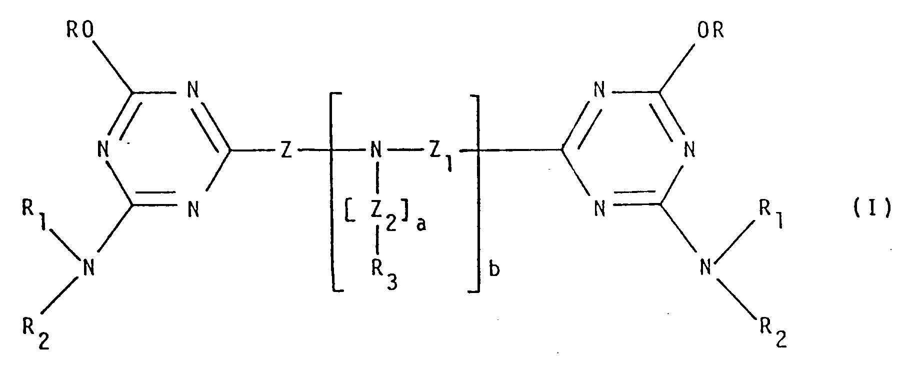 wherein:
R = H; (C₂-C₆)-alkenyl; (C₆-C₁₂)-cycloalkyl; (C₆-C₁₂)-alkyl-cycloalkyl; (C₆-C₁₂)-aryl; (C₇-C₁₂)-aralkyl; or ⁅CₙH₂ₙ⁆Y,
n being an integer of from 1 to 8 and Y representing H; CN; -O(C₁-C₄)-alkyl; -O-(C₂-C₄)-alkenyl; (C₆-C₁₂)-cycloalkyl; (C₆-C₁₂)-alkylcycloalkyl; -O-(C₆-C₁₂)-aryl;or -N(R₄)₂, wherein the groups R₄, the same or different from each other, are (C₁-C₄)-alkyl or (C₃-C₄)-alkenyl or the moiety -N(R₄)₂ is replaced by an N-heterocyclic radical which is linked to the alkyl chain through the nitrogen atom and is selected from pyrrolidyl; piperidyl; morpholyl; thiomorpholyl; piperazyl; 4-methylpiperazyl and 4-ethylpiperazyl;
the groups R₁ and R₂, the same or different from each other and having the same or different meanings in each triazine ring, are selected from H; (C₁-C₁₈)-alkyl; (C₂-C₈)-alkenyl; (C₆-C₁₆)cycloalkyl; (C₆-C₁₆)-alkylcycloalkyl;
⁅CₘH₂ₘ⁆O-R₅; and 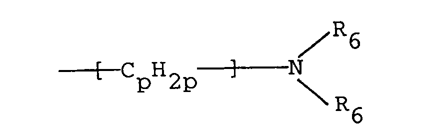 wherein:
m = integer of from 2 to 8;
p = integer of from 2 to 6;
R₅ = H; (C₁-C₈)-alkyl; (C₂-C₆)-alkenyl;
⁅C_{q}H_{2q}⁆O-R₇, q being an integer of from 1 to 4 and R₇ being H or (C₁-C₄)-alkyl; (C₆-C₁₂)-cycloalkyl; and (C₆-C₁₂)-alkylcycloalkyl;
the groups R₆, the same or different from each other, are selected from H; (C₁-C₈)-alkyl; (C₂-C₆)-alkenyl; (C₆-C₁₂)-cycloalkyl; (C₆-C₁₂)-alkylcycloalkyl; and (C₁-C₄)-hydroxyalkyl; or the moiety -N(R₆)₂ is replaced by an N-heterocyclic radical which is linked to the alkyl chain through the nitrogen atom and is
selected from aziridyl; pyrrolidyl; piperidyl; morpholyl; thiomorpholyl; piperazyl; 4-methylpiperazyl and 4-ethylpiperazyl;
or in general formula (I) one or both of the moieties -NR₁R₂ are replaced by an N-heterocyclic radical which is linked to the triazine ring through the nitrogen atom and is selected from aziridyl; pyrrolidyl; piperidyl; morpholyl; thiomorpholyl; piperazyl; 4-methylpiperazyl; 4-ethylpiperazyl; 2-methylpiperazyl; 2,5-dimethylpiperazyl; 2,3,5,6-tetramethylpiperazyl; 2,2,5,5-tetramethylpiperazyl; 2-ethylpiperazyl; and 2,5-diethylpiperazyl;
a is 0 or 1;
b is 0 or an integer of from 1 to 5;
R₃ is hydrogen or a group of formula 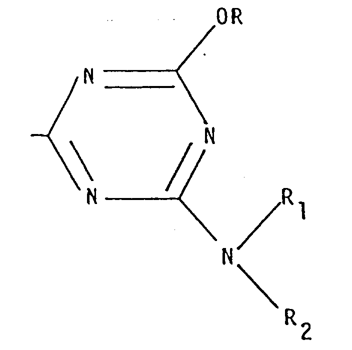 and its meaning may vary within each repeating unit; when b is O:
Z is a divalent radical selected from those of the following formulae: 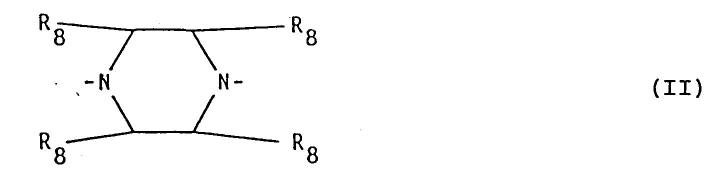 wherein the groups R₈, the same or different from each other, represent hydrogen or (C₁-C₄)-alkyl; 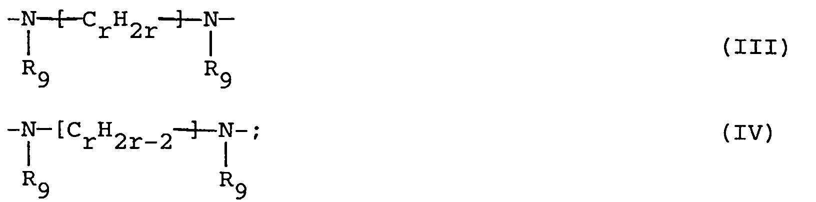 wherein r is an integer of from 2 to 14 and R₉ is selected from hydrogen; (C₁-C₄)-alkyl; (C₂-C₆)-alkenyl; (C₁-C₄)-hydroxyalkyl; 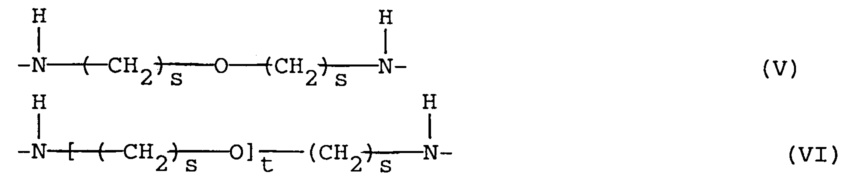 wherein s is an integer of from 2 to 5 and t is an integer of from 1 to 3; 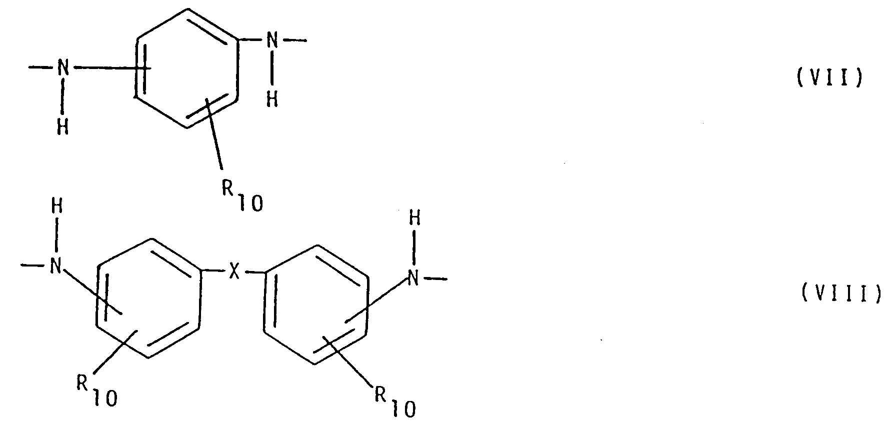 wherein X is a direct carbon carbon bond; O; S; S-S; SO; SO₂; NH; NHSO₂; NHCO; N=N; or CH₂; and R₁₀ is hydrogen; hydroxy; (C₁-C₄)-alkyl or (C₁-C₄)-alkoxy; 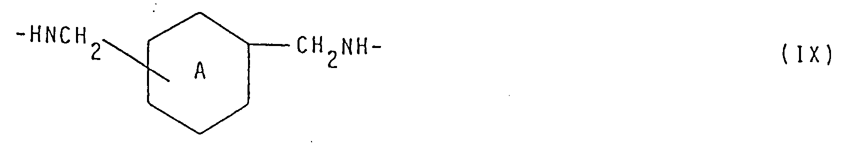 wherein A is an either saturated or unsaturated ring; 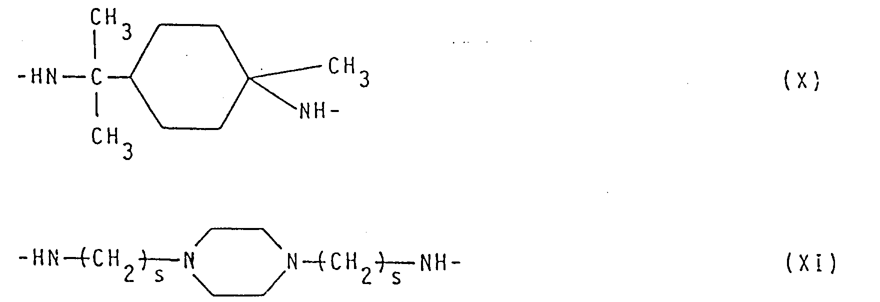 wherein s is an integer of from 2 to 5; with the proviso that when R represents (C₁-C₈)-alkyl, (C₆-C₁₂)-cycloalkyl, (C₇-C₁₂)-aralkyl or a phenyl or naphthyl group optionally substituted with inert radicals, and R¹ and R² are (C₁-C₃)-alkyl or (C₆-C₁₂)-cycloalkyl groups, or -NR¹R² represents a piperidinyl or morpholinyl group, Z cannot be of formula (II) wherein all four of the groups R₈ are hydrogen;
when b is an integer of from 1 to 5:
the moiety of formula 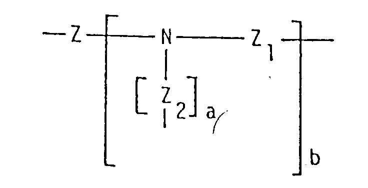 is a polyvalent radical selected from those of the following formulae; 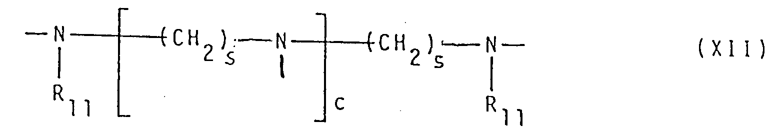 wherein R₁₁ is hydrogen or (C₁-C₄)-alkyl;
c is an integer of from 1 to 5;
the subscripts s, the same or different from each other, are integers of from 2 to 5; 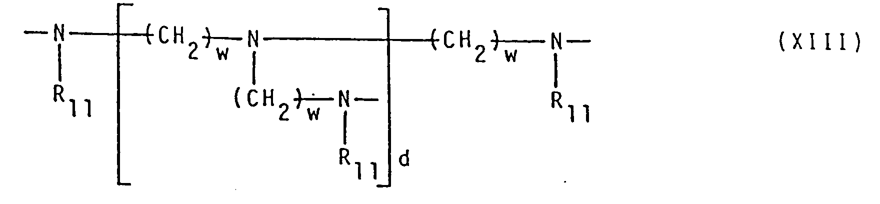 wherein R₁₁ is hydrogen or (C₁-C₄) alkyl;
w is an integer of from 2 to 4; and
d is 1 or 2.

2. Compounds according to claim 1, wherein at least one of the groups R₁ and R₂ in general formula (I) is a group of formula
⁅CₘH₂ₘ⁆O-R₅;
wherein:
m is an integer of from 2 to 4; and
R₅ is hydrogen or (C₁-C₄)-alkyl.

3. Compounds according to any one of claims 1 and 2 wherein R in general formula (I) is hydrogen or a group of formula
⁅CₙH₂ₙ⁆Y
wherein n is an integer of form 1 to 4 and Y is hydrogen.

4. Process for the preparation of compounds of general formula (I) according to any one of claims 1 to 3, comprising the hydrolysis of intermediates of general formula (XIV): 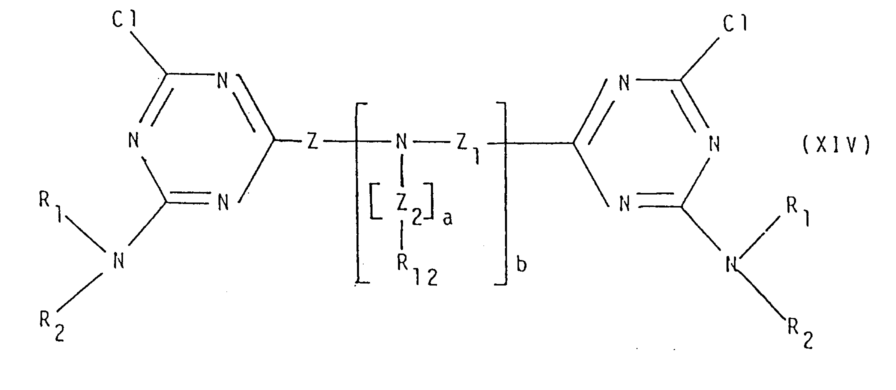 wherein R₁, R₂, Z, Z₁, Z₂, a and b are as defined in claim 1 and R₁₂ is hydrogen or a group of formula 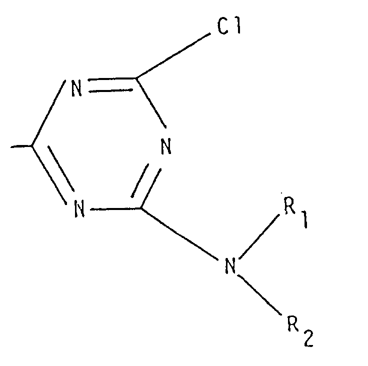 and its meaning may vary within each repeating unit; or the condensation of intermediates of general formula (XIV) with a reagent of general formula (XV):
R-OH (XV)
wherein R has the meanings given in claim 1 but cannot be hydrogen.

5. Process according to claim 4, wherein the hydrolysis reaction is carried out either in the presence of an acid at temperatures of from about 60 to about 100°C, or in the presence of a base at temperatures of from about 100 to about 180°C.

6. Process according to claim 4, wherein the condensation reaction is carried out, in a solvent and/or in an excess of the reagent of general formula (XV), in the presence of a base at temperatures of from about 60 to about 150°C.

7. Process for the preparation of compounds of general formula (I) according to any one of claims 1 to 3, R being different from hydrogen, comprising the reaction of one mol of a polyamine of general formula (XVI): 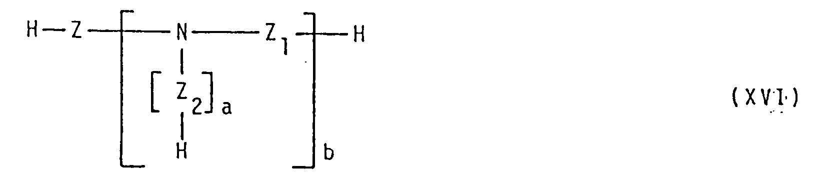 wherein Z, Z₁, Z₂, a and b are as defined in claim 1; with (2+b) moles or less of the intermediate of general formula (XXI): 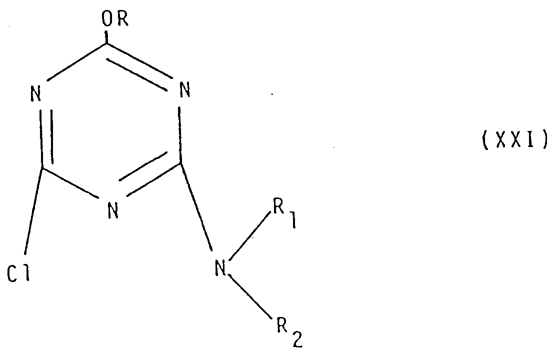 wherein R, R₁ and R₂ have the meanings given in claim 1, R being different from hydrogen.

8. Self-extinguishing polymeric compositions, comprising:
a) from 91 to 40 parts by weight of thermoplastic polymer and/or of polymer showing elastomeric properties;
b) from 6 to 33 parts by weight of one or more ammonium and/or amine phosphates and/or phosphonates;
c) from 3 to 27 parts by weight of one or more compounds of general formula (I) as defined in any one of claims 1 to 4.

9. Compositions according to claim 8, wherein the ammonium phosphates (b) have the general formula (NH₄)ₙ₊₂PₙO₃ₙ₊₁ wherein n is an integer of at least 2; or have the general formula (NH₄PO₃)ₙ wherein n ranges from 50 to 500.

10. Compositions according to any one of claims 8 and 9, wherein the amine phosphates (b) are selected from dimethylammonium phosphate; diethylammonium phosphate; ethylenediamine phosphate; melamine ortho-phosphate; and melamine pyrophosphate.

11. Compositions according to any one of claims 8 to 10 wherein the ammonium phosphonates (b) are the mono- and polysubstituted ones and are selected from salts derived from mono- and polyphosphonic acids.

12. Compositions according to any one of claims 8 to 11, wherein the polymer (a) is selected from acrylonitrile/butadiene/styrene (ABS) terpolymers; styrene/acrylonitrile (SAN) copolymers; polyurethanes; poly(ethyleneterephthalate); poly(butylene terephthalate); polyamides; and polymers and copolymers of olefins of general formula R'-CH=CH₂ wherein R' is hydrogen, a (C₁-C₈)-alkyl or (C₆-C₁₀)-aryl group, particularly:
(1) isotactic or prevailingly isotactic polypropylene;
(2) HDPE, LLDPE and LDPE polyethylene;
(3) crystalline copolymers of propylene and minor amounts of ethylene and/or other alpha-olefins, such as butene-1, hexene-1, octene-1 and 4-methylpentene-1;
(4) heterophasic compositions comprising (A) a fraction of propylene homopolymer or of propylene copolymers as specified under (3) and (B) a copolymeric fraction composed of elastomeric copolymers of ethylene and an alpha-olefin, optionally containing minor amounts of a diene, wherein the alpha-olefin is preferably selected from propylene and butene-1;
(5) elastomeric copolymers of ethylene and alpha-olefin(s) optionally containing minor amounts of diene.

13. Molded articles, obtained from compositions according to any one of claims 8 to 12.

## Claims (Claims for the following Contracting State(s): DK)

1. Ameline derivatives of general formula (I): 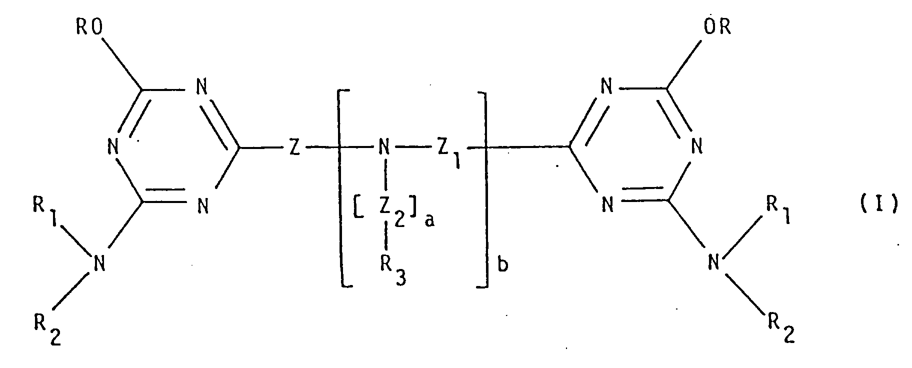 wherein:
R = H; (C₂-C₆)-alkenyl; (C₆-C₁₂)-cycloalkyl; (C₆-C₁₂)-alkyl-cycloalkyl; (C₆-C₁₂)-aryl; (C₇-C₁₂)-aralkyl; or ⁅CₙH₂ₙ⁆Y,
n being an integer of from 1 to 8 and Y representing H; CN; -O(C₁-C₄)-alkyl; -O-(C₂-C₄)-alkenyl; (C₆-C₁₂)-cycloalkyl; (C₆-C₁₂)-alkylcycloalkyl; -O-(C₆-C₁₂)-aryl;or
-N(R₄)₂, wherein the groups R₄, the same or different from each other, are (C₁-C₄)-alkyl or (C₃-C₄)-alkenyl or the moiety -N(R₄)₂ is replaced by an N-heterocyclic radical which is
linked to the alkyl chain through the nitrogen atom and is selected from pyrrolidyl; piperidyl; morpholyl; thiomorpholyl; piperazyl; 4-methylpiperazyl and 4-ethylpiperazyl;
the groups R₁ and R₂, the same or different from each other and having the same or different meanings in each triazine ring, are selected from H; (C₁-C₁₈)-alkyl; (C₂-C₈)-alkenyl; (C₆-C₁₆)-cycloalkyl; (C₆-C₁₆)-alkylcycloalkyl;
⁅CₘH₂ₘ⁆O-R₅; and 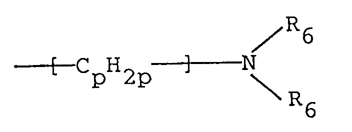 wherein:
m = integer of from 2 to 8;
p = integer of from 2 to 6;
R₅ = H; (C₁-C₈)-alkyl; (C₂-C₆)-alkenyl;
⁅C_{q}H_{2q}⁆O-R₇, q being an integer of from 1 to 4 and R₇ being H or (C₁-C₄)-alkyl; (C₆-C₁₂)-cycloalkyl; and (C₆-C₁₂)-alkylcycloalkyl;
the groups R₆, the same or different from each other, are selected from H; (C₁-C₈)-alkyl; (C₂-C₆)-alkenyl; (C₆-C₁₂)-cycloalkyl; (C₆-C₁₂)-alkylcycloalkyl; and (C₁-C₄)-hydroxyalkyl; or the moiety -N(R₆)₂ is replaced by an N-heterocyclic radical which is linked to the alkyl chain through the nitrogen atom and is
selected from aziridyl; pyrrolidyl; piperidyl; morpholyl; thiomorpholyl; piperazyl; 4-methylpiperazyl and 4-ethylpiperazyl;
or in general formula (I) one or both of the moieties -NR₁R₂ are replaced by an N-heterocyclic radical which is linked to the triazine ring through the nitrogen atom and is selected from aziridyl; pyrrolidyl; piperidyl; morpholyl; thiomorpholyl; piperazyl; 4-methylpiperazyl; 4-ethylpiperazyl; 2-methylpiperazyl; 2,5-dimethylpiperazyl; 2,3,5,6-tetramethylpiperazyl; 2,2,5,5-tetramethylpiperazyl; 2-ethylpiperazyl; and 2,5-diethylpiperazyl;
a is 0 or 1;
b is 0 or an integer of from 1 to 5;
R₃ is hydrogen or a group of formula 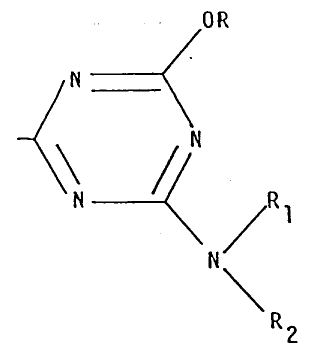 and its meaning may vary within each repeating unit;
when b is O:
Z is a divalent radical selected from those of the following formulae: 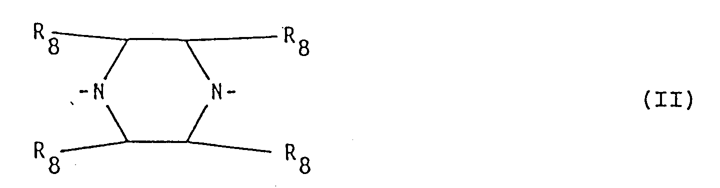 wherein the groups R₈, the same or different from each other, represent hydrogen or (C₁-C₄)-alkyl; 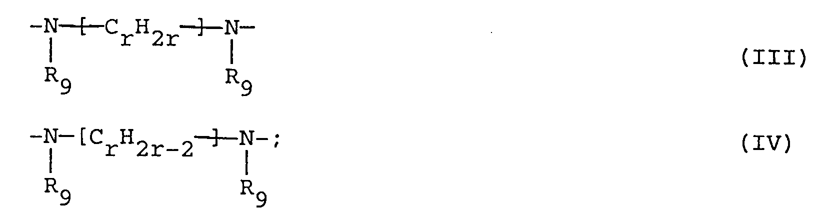 wherein r is an integer of from 2 to 14 and R₉ is selected from hydrogen; (C₁-C₄)-alkyl; (C₂-C₆)-alkenyl; (C₁-C₄)-hydroxyalkyl; 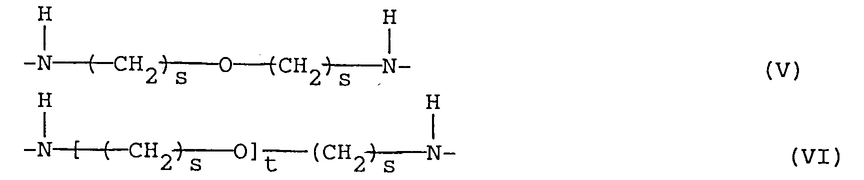 wherein s is an integer of from 2 to 5 and t is an integer of from 1 to 3; 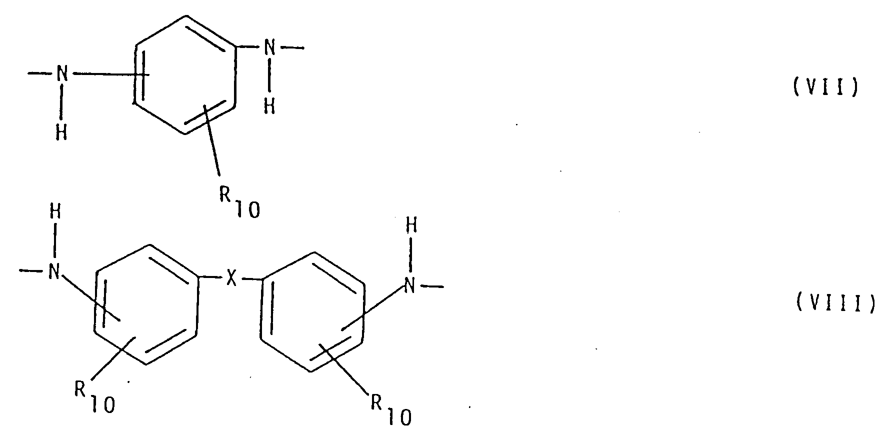 wherein X is a direct carbon carbon bond; O; S; S-S; SO; SO₂; NH; NHSO₂; NHCO; N=N; or CH₂; and R₁₀ is hydrogen; hydroxy; (C₁-C₄)-alkyl or (C₁-C₄)-alkoxy; 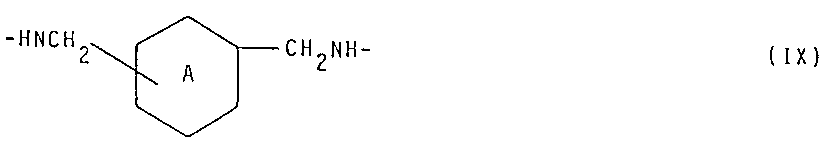 wherein A is an either saturated or unsaturated ring; 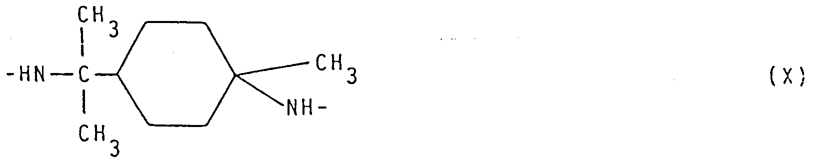 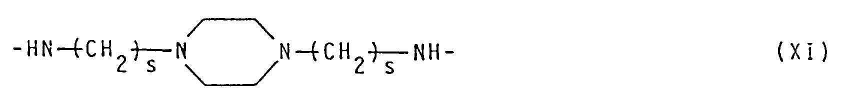 wherein s is an integer of from 2 to 5;
when b is an integer of from 1 to 5:
the moiety of formula 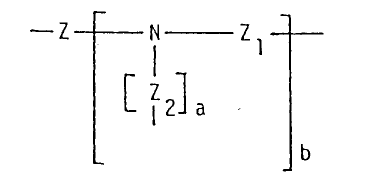 is a polyvalent radical selected from those of the following formulae; 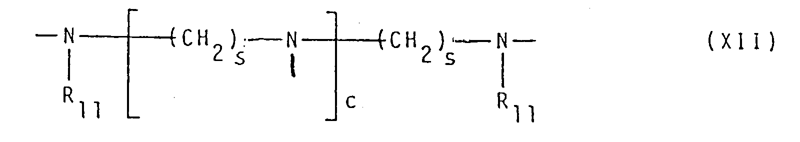 wherein R₁₁ is hydrogen or (C₁-C₄)-alkyl;
c is an integer of from 1 to 5;
the subscripts s, the same or different from each other, are integers of from 2 to 5; 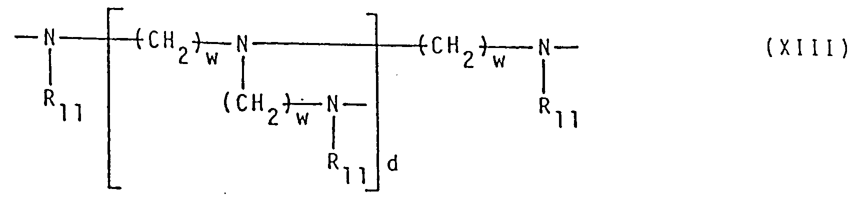 wherein R₁₁ is hydrogen or (C₁-C₄) alkyl;
w is an integer of from 2 to 4; and
d is 1 or 2.

2. Compounds according to claim 1, wherein at least one of the groups R₁ and R₂ in general formula (I) is a group of formula
⁅CₘH₂ₘ⁆O-R₅;
wherein: m is an integer of from 2 to 4; and
R₅ is hydrogen or (C₁-C₄)-alkyl.

3. Compounds according to any one of claims 1 and 2 wherein R in general formula (I) is hydrogen or a group of formula
⁅CₙH₂ₙ⁆Y
wherein n is an integer of form 1 to 4 and Y is hydrogen.

4. Process for the preparation of compounds of general formula (I) according to any one of claims 1 to 3, comprising the hydrolysis of intermediates of general formula (XIV): 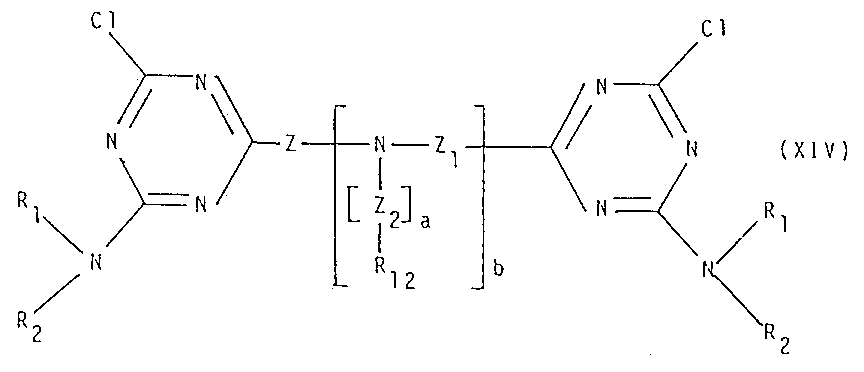 wherein R₁, R₂, Z, Z₁, Z₂, a and b are as defined in claim 1 and R₁₂ is hydrogen or a group of formula 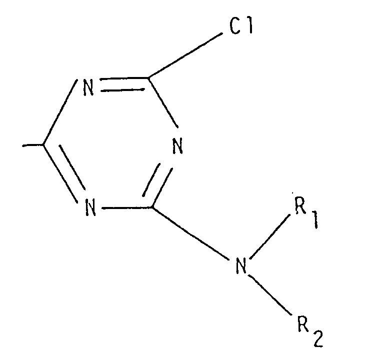 and its meaning may vary within each repeating unit; or the condensation of intermediates of general formula (XIV) with a reagent of general formula (XV):
R-OH (XV)
wherein R has the meanings given in claim 1 but cannot be hydrogen.

5. Process according to claim 4, wherein the hydrolysis reaction is carried out either in the presence of an acid at temperatures of from about 60 to about 100°C, or in the presence of a base at temperatures of from about 100 to about 180°C.

6. Process according to claim 4, wherein the condensation reaction is carried out, in a solvent and/or in an excess of the reagent of general formula (XV), in the presence of a base at temperatures of from about 60 to about 150°C.

7. Process for the preparation of compounds of general formula (I) according to any one of claims 1 to 3, R being different from hydrogen, comprising the reaction of one mol of a polyamine of general formula (XVI): 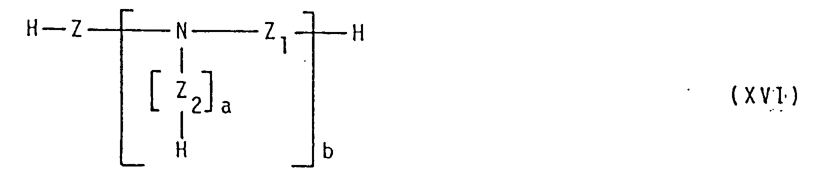 wherein Z, Z₁, Z₂, a and b are as defined in claim 1; with (2+b) moles or less of the intermediate of general formula (XXI): 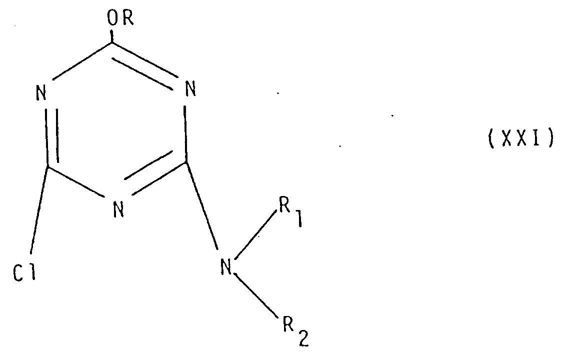 wherein R, R₁ and R₂ have the meanings given in claim 1, R being different from hydrogen.

8. Self-extinguishing polymeric compositions, comprising:
a) from 91 to 40 parts by weight of thermoplastic polymer and/or of polymer showing elastomeric properties;
b) from 6 to 33 parts by weight of one or more ammonium and/or amine phosphates and/or phosphonates;
c) from 3 to 27 parts by weight of one or more compounds of general formula (I) as defined in any one of claims 1 to 4.

9. Compositions according to claim 8, wherein the ammonium phosphates (b) have the general formula (NH₄)ₙ₊₂PₙO₃ₙ₊₁ wherein n is an integer of at least 2; or have the general formula (NH₄PO₃)ₙ wherein n ranges from 50 to 500.

10. Compositions according to any one of claims 8 and 9, wherein the amine phosphates (b) are selected from dimethylammonium phosphate; diethylammonium phosphate; ethylenediamine phosphate; melamine ortho-phosphate; and melamine pyrophosphate.

11. Compositions according to any one of claims 8 to 10 wherein the ammonium phosphonates (b) are the mono- and polysubstituted ones and are selected from salts derived from mono- and polyphosphonic acids.

12. Compositions according to any one of claims 8 to 11, wherein the polymer (a) is selected from acrylonitrile/butadiene/styrene (ABS) terpolymers; styrene/acrylonitrile (SAN) copolymers; polyurethanes; poly(ethyleneterephthalate); poly(butylene terephthalate); polyamides; and polymers and copolymers of olefins of general formula R'-CH=CH₂ wherein R' is hydrogen, a (C₁-C₈)-alkyl or (C₆-C₁₀)-aryl group, particularly:
(1) isotactic or prevailingly isotactic polypropylene;
(2) HDPE, LLDPE and LDPE polyethylene;
(3) crystalline copolymers of propylene and minor amounts of ethylene and/or other alpha-olefins, such as butene-1, hexene-1, octene-1 and 4-methylpentene-1;
(4) heterophasic compositions comprising (A) a fraction of propylene homopolymer or of propylene copolymers as specified under (3) and (B) a copolymeric fraction composed of elastomeric copolymers of ethylene and an alpha-olefin, optionally containing minor amounts of a diene, wherein the alpha-olefin is preferably selected from propylene and butene-1;
(5) elastomeric copolymers of ethylene and alpha-olefin(s) optionally containing minor amounts of diene.

13. Molded articles, obtained from compositions according to any one of claims 8 to 12.

## Claims (Claims for the following Contracting State(s): ES)

1. Process for the preparation of ameline derivatives of general formula (I): 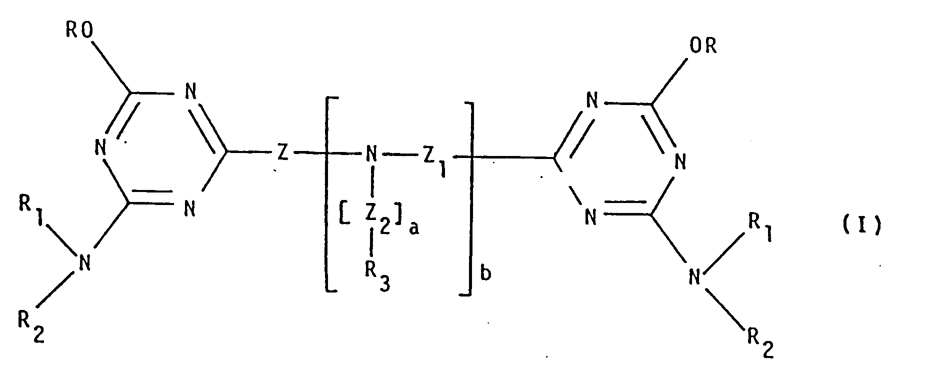 wherein:
R = H; (C₂-C₆)-alkenyl; (C₆-C₁₂)cycloalkyl; (C₆-C₁₂)-alkyl-cycloalkyl; (C₆-C₁₂)-aryl; (C₇-C₁₂)-aralkyl; or ⁅CₙH₂ₙ⁆Y,
n being an integer of from 1 to 8 and Y representing H; CN; -O(C₁-C₄)-alkyl; -O-(C₂-C₄)-alkenyl; (C₆-C₁₂)-cycloalkyl; (C₆-C₁₂)-alkylcycloalkyl; -O-(C₆-C₁₂)-aryl;or
-N(R₄)₂, wherein the groups R₄, the same or different from each other, are (C₁-C₄)-alkyl or (C₃-C₄)-alkenyl or the moiety -N(R₄)₂ is replaced by an N-heterocyclic radical which is linked to the alkyl chain through the nitrogen atom and is selected from pyrrolidyl; piperidyl; morpholyl; thiomorpholyl; piperazyl; 4-methylpiperazyl and 4-ethylpiperazyl;
the groups R₁ and R₂, the same or different from each other and having the same or different meanings in each triazine ring, are selected from H; (C₁-C₁₈)-alkyl; (C₂-C₈)-alkenyl; (C₆-C₁₆)-cycloalkyl; (C₆-C₁₆)-alkylcycloalkyl;
⁅CₘH₂ₘ⁆O-R₅; and 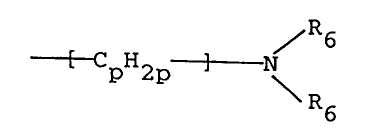 wherein:
m = integer of from 2 to 8;
p = integer of from 2 to 6;
R₅ = H; (C₁-C₈)-alkyl; (C₂-C₆)-alkenyl;
⁅C_{q}H_{2q}⁆O-R₇, q being an integer of from 1 to 4 and R₇ being H or (C₁-C₄)-alkyl; (C₆-C₁₂)-cycloalkyl; and (C₆-C₁₂)-alkylcycloalkyl;
the groups R₆, the same or different from each other, are selected from H; (C₁-C₈)-alkyl; (C₂-C₆)-alkenyl; (C₆-C₁₂)-cycloalkyl; (C₆-C₁₂)-alkylcycloalkyl; and (C₁-C₄)-hydroxyalkyl; or the moiety -N(R₆)₂ is replaced by an N-heterocyclic radical which is linked to the alkyl chain through the nitrogen atom and is selected from aziridyl; pyrrolidyl; piperidyl; morpholyl; thiomorpholyl; piperazyl; 4-methylpiperazyl and 4-ethylpiperazyl;
or in general formula (I) one or both of the moieties -NR₁R₂ are replaced by an N-heterocyclic radical which is linked to the triazine ring through the nitrogen atom and is selected from aziridyl; pyrrolidyl; piperidyl; morpholyl; thiomorpholyl; piperazyl; 4-methylpiperazyl; 4-ethylpiperazyl; 2-methylpiperazyl; 2,5-dimethylpiperazyl; 2,3,5,6-tetramethylpiperazyl; 2,2,5,5-tetramethylpiperazyl; 2-ethylpiperazyl; and 2,5-diethylpiperazyl;
a is 0 or 1;
b is 0 or an integer of from 1 to 5;
R₃ is hydrogen or a group of formula 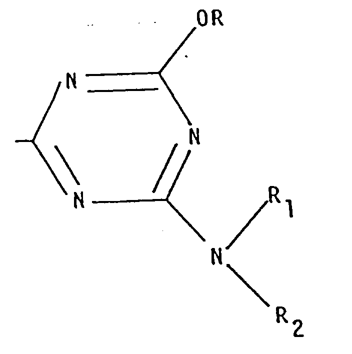 and its meaning may vary within each repeating unit;
when b is O:
Z is a divalent radical selected from those of the following formulae: 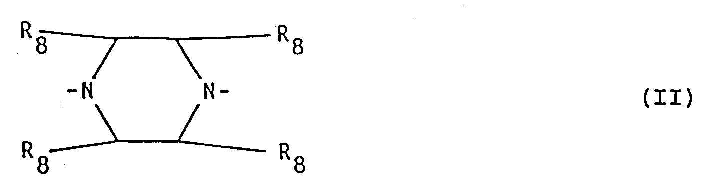 wherein the groups R₈, the same or different from each other, represent hydrogen or (C₁-C₄)-alkyl; 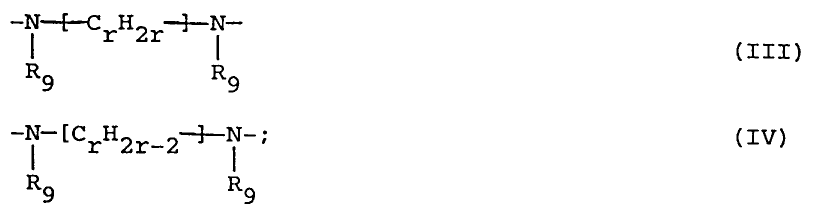 wherein r is an integer of from 2 to 14 and R₉ is selected from hydrogen; (C₁-C₄)-alkyl; (C₂-C₆)-alkenyl; (C₁-C₄)-hydroxyalkyl; 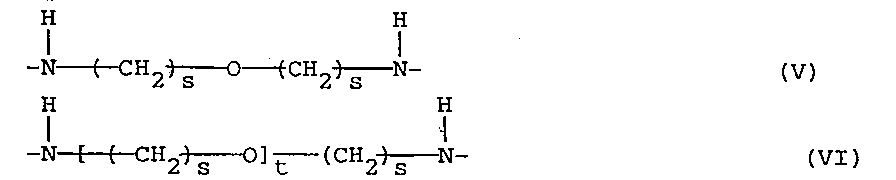 wherein s is an integer of from 2 to 5 and t is an integer of from 1 to 3; 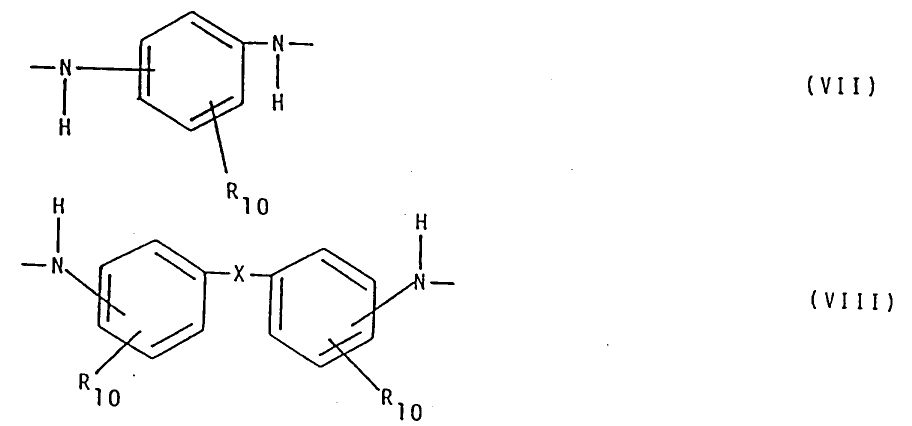 wherein X is a direct carbon carbon bond; O; S; S-S; SO; SO₂; NH; NHSO₂; NHCO; N=N; or CH₂; and
R₁₀ is hydrogen; hydroxy; (C₁-C₄)-alkyl or (C₁-C₄)-alkoxy; 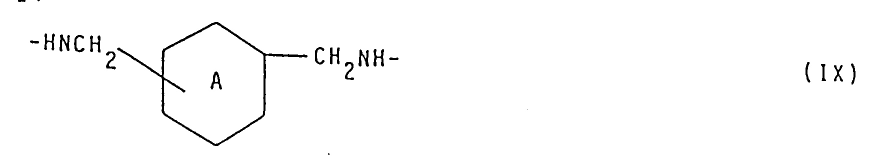 wherein A is an either saturated or unsaturated ring; 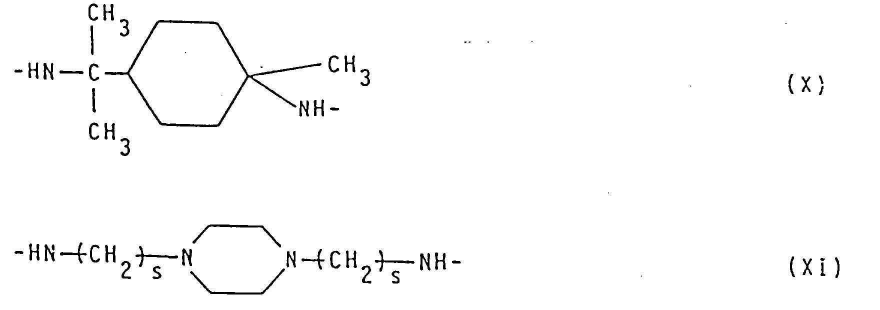 wherein s is an integer of from 2 to 5; with the proviso that when R represents (C₁-C₈)-alkyl, (C₆-C₁₂)-cycloalkyl, (C₇-C₁₂)-aralkyl or a phenyl or naphthyl group optionally substituted with inert radicals, and R¹ and R² are (C₁-C₃)-alkyl or (C₆-C₁₂)-cycloalkyl groups, or -NR¹R² represents a piperidinyl or morpholinyl group, Z cannot be of formula (II) wherein all four of the groups R₈ are hydrogen;
when b is an integer of from 1 to 5:
the moiety of formula 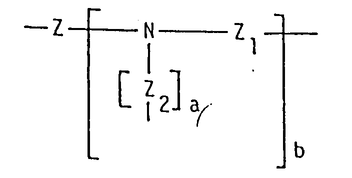 is a polyvalent radical selected from those of the following formulae; 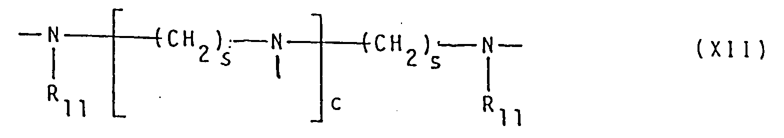 wherein R₁₁ is hydrogen or (C₁-C₄)-alkyl;
c is an integer of from 1 to 5;
the subscripts s, the same or different from each other, are integers of from 2 to 5; 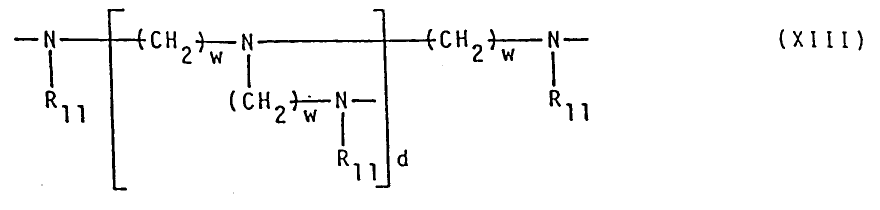
wherein R₁₁ is hydrogen or (C₁-C₄)-alkyl;
w is an integer of from 2 to 4; and
d is 1 or 2;
said process comprising
(a) for R = H, the hydrolysis of intermediates of general formula (XIV): 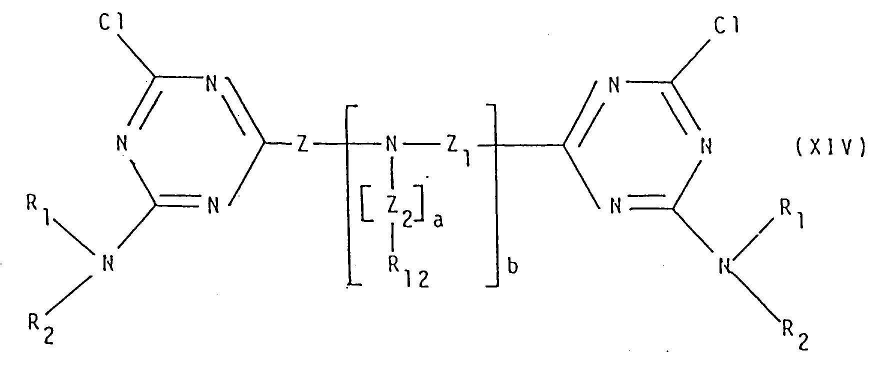 wherein R₁, R₂, Z, Z₁, Z₂, a and b are as defined above and R₁₂ is hydrogen or a group of formula 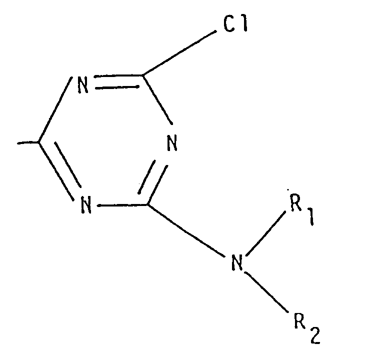 and its meaning may vary within each repeating unit; or
(b) for R ≠ H, the condensation of intermediates of general formula (XIV) with a reagent of general formula (XV):
R-OH (XV)
wherein R has the meanings given above but cannot be hydrogen; or
(c) for R ≠ H, the reaction of one mole of a polyamine of general formula (XVI): 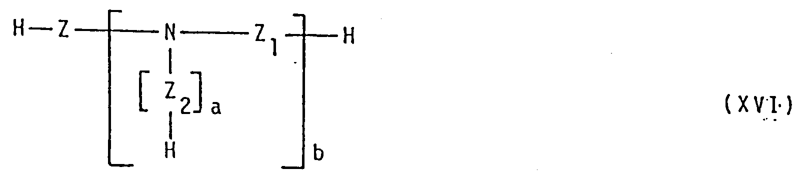 wherein Z, Z₁, Z₂, a and b are as defined above; with (2+b) moles or less of the intermediate of general formula (XXI): 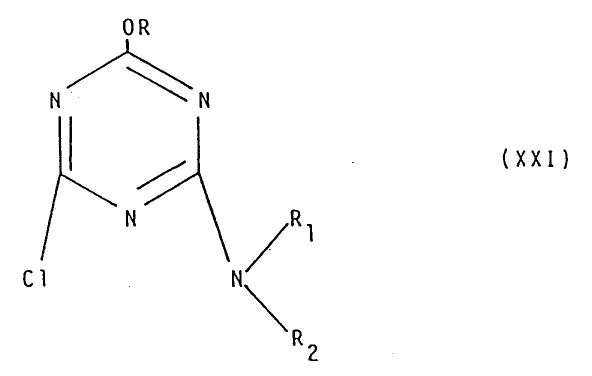 wherein R, R₁ and R₂ have the meanings given above, R being different from hydrogen.

2. Process according to claim 1 wherein compounds are prepared in which at least one of the groups R₁ and R₂ in general formula (I) is a group of formula
⁅CₘH₂ₘ⁆O-R₅;
wherein:
m is an integer of from 2 to 4; and
R₅ is hydrogen or (C₁-C₄)-alkyl.

3. Process according to any one of claims 1 and 2 wherein R in general formula (I) is hydrogen or a group of formula
⁅CₙH₂ₙ⁆Y
wherein n is an integer of from 1 to 4 and Y is hydrogen.

4. Process according to any one of claims 1 to 3, alternative (a), wherein the hydrolysis reaction is carried out in the presence of an acid at temperatures of from about 60 to about 100°C, or in the presence of a base at temperatures of from about 100 to about 180°C.

5. Process according to any one of claims 1 to 3, alternative (b), wherein the condensation reaction is carried out, in a solvent and/or in an excess of the reagent of general formula (XV), in the presence of a base at temperatures of from about 60 to about 150°C.

6. Self-extinguishing polymeric compositions, comprising:
(a) from 91 to 40 parts by weight of thermoplastic polymer and/or of polymer showing elastomeric properties;
(b) form 6 to 33 parts by weight of one or more ammonium and/or amine phosphates and/or phosphonates;
(c) from 3 to 27 parts by weight of one or more compounds of general formula (I) as defined in any one of claims 1 to 3.

7. Compositions according to claim 6, wherein the ammonium phosphates (b) have the general formula (NH₄)ₙ₊₂PₙO₃ₙ₊₁ wherein n is an integer of at least 2; or have the general formula (NH₄PO₃)ₙ wherein n ranges from 50 to 500.

8. Compositions according to any one of claims 6 and 7, wherein the amine phosphates (b) are selected from dimethylammonium phosphate; diethylammonium phosphate; ethylenediamine phosphate; melamine ortho-phosphate; and melamine pyrophosphate.

9. Compositions according to any one of claims 6 to 8, wherein the ammonium phosphonates (b) are the mono- and polysubstituted ones and are selected from salts derived from mono- and polyphosphonic acids.

10. Compositions according to any one of claims 6 to 9, wherein the polymer (a) is selected from acrylonitrile/butadiene/styrene (ABS) terpolymers; styrene/acrylonitrile (SAN) copolymers; polyurethanes; poly(ethyleneterephthalate); poly(butylene terephthalate); polyamides; and polymers and copolymers of olefins of general formula R'-CH=CH₂ wherein R' is hydrogen, a (C₁-C₈)-alkyl or (C₆-C₁₀)-aryl group, particularly:
(1) isotactic or prevailingly isotactic polypropylene;
(2) HDPE, LLDPE and LDPE polyethylene;
(3) crystalline copolymers of propylene and minor amounts of ethylene and/or other alpha-olefins, such as butene-1, hexene-1, octene-1 and 4-methylpentene-1;
(4) heterophasic compositions comprising (A) a fraction of propylene homopolymer or of propylene copolymers as specified under (3) and (B) a copolymeric fraction composed of elastomeric copolymers of ethylene and an alpha-olefin, optionally containing minor amounts of a diene, wherein the alpha-olefin is preferably selected from propylene and butene-1;
(5) elastomeric copolymers of ethylene and alpha-olefin(s) optionally containing minor amounts of diene.

11. Molded articles, obtained from compositions according to any one of claims 6 to 10.

## Patentansprüche (Patentansprüche für folgende(n) Vertragsstaat(en): AT, BE, CH, DE, FR, GB, LI, NL, SE)

1. Amelin-Derivate der allgemeinen Formel (I): 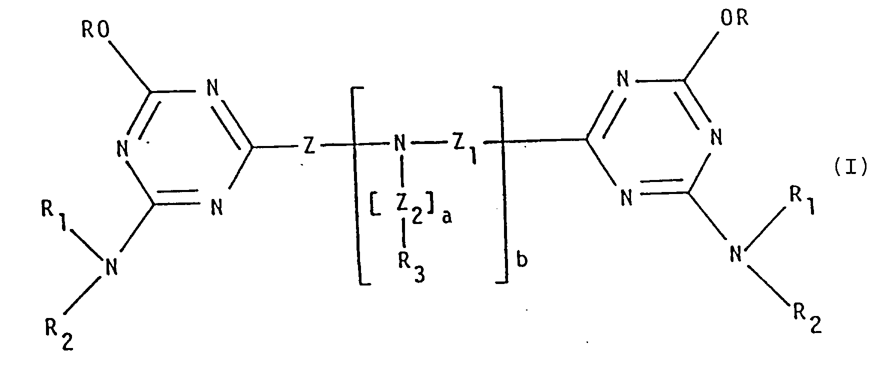 worin:
R = H; (C₂-C₆)-Alkenyl; (C₆-C₁₂)-Cycloalkyl; (C₆-C₁₂)-Alkylcycloalkyl; (C₆-C₁₂)-Aryl; (C₇-C₁₂)-Aralkyl; oder ⁅CₙH₂ₙ⁆Y,
wobei n eine ganze Zahl von 1 bis 8 ist und Y H; CN; -O-(C₁-C₄)-Alkyl; -O-(C₂-C₄)-Alkenyl; (C₆-C₁₂)-Cycloalkyl; (C₆-C₁₂)-Alkylcycloalkyl; -O-(C₆-C₁₂)-Aryl; oder -N(R₄)₂ darstellt, worin die Gruppen R₄, gleich oder verschieden voneinander, für (C₁-C₄)-Alkyl oder (C₃-C₄)-Alkenyl stehen oder die Einheit -N(R₄)₂ durch einen N-heterocyclischen Rest ersetzt ist, der über das Stickstoffatom an die Alkylkette gebunden ist und aus Pyrrolidyl; Piperidyl; Morpholyl; Thiomorpholyl; Piperazyl; 4-Methylpiperazyl und 4-Ethylpiperazyl ausgewählt ist;
die Gruppen R₁ und R₂, gleich oder verschieden voneinander und dieselben oder verschiedene Bedeutungen in jedem Triazin-Ring aufweisend, ausgewählt sind aus H; (C₁-C₁₈)-Alkyl; (C₂-C₈)-Alkenyl; (C₆-C₁₆)-Cycloalkyl; (C₆-C₁₆)-Alkylcycloalkyl; ⁅CₘH₂ₘ⁆O-R₅; und 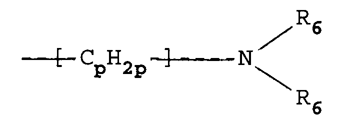 worin:
m = ganze Zahl von 2 bis 8;
p = ganze Zahl von 2 bis 6;
R₅ = H; (C₁-C₈)-Alkyl; (C₂-C₆)-Alkenyl;
⁅C_{q}H_{2q}⁆O-R₇, wobei q eine ganze Zahl von 1 bis 4 ist und R₇ H oder (C₁-C₄)-Alkyl; (C₆-C₁₂)-Cycloalkyl; und (C₆-C₁₂)-Alkylcycloalkyl darstellt;
die Gruppen R₆, gleich oder verschieden voneinander, ausgewählt sind aus H; (C₁-C₈)-Alkyl; (C₂-C₆)-Alkenyl; (C₆-C₁₂)-Cycloalkyl; (C₆-C₁₂)-Alkylcycloalkyl; und (C₁-C₄)-Hydroxyalkyl; oder die Einheit -N(R₆)₂ durch einen N-heterocyclischen Rest ersetzt ist, der über das Stickstoffatom an die Alkylkette gebunden ist und ausgewählt ist aus Aziridyl; Pyrrolidyl; Piperidyl; Morpholyl; Thiomorpholyl; Piperazyl; 4-Methylpiperazyl und 4-Ethylpiperazyl;
oder in der allgemeinen Formel (I) eine oder beide der Einheiten -NR₁R₂ durch einen N-heterocyclischen Rest ersetzt sind, der über das Stickstoffatom an den Triazin-Ring gebunden ist und ausgewählt ist aus Aziridyl; Pyrrolidyl; Piperidyl; Morpholyl; Thiomorpholyl; Piperazyl; 4-Methylpiperazyl; 4-Ethylpiperazyl; 2-Methylpiperazyl; 2,5-Dimethylpiperazyl; 2,3,5,6-Tetramethylpiperazyl; 2,2,5,5-Tetramethylpiperazyl; 2-Ethylpiperazyl; und 2,5-Diethylpiperazyl;
a 0 oder 1 ist;
b 0 oder eine ganze Zahl von 1 bis 5 ist;
R₃ Wasserstoff oder eine Gruppe der Formel 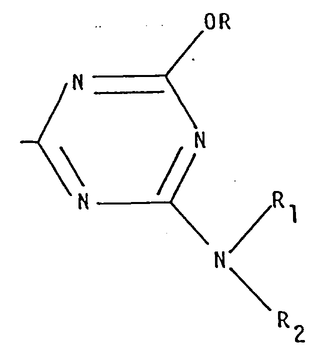 darstellt und seine Bedeutung in jeder wiederkehrenden Einheit verschieden sein kann;
wenn b 0 ist:
Z ein zweiwertiger Rest ist, der aus denjenigen mit den folgenden Formeln ausgewählt ist: 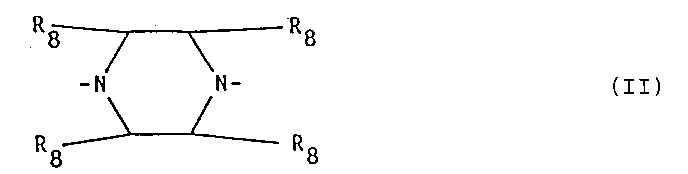 worin die Gruppen R₈, gleich oder verschieden voneinander, für Wasserstoff oder (C₁-C₄)-Alkyl stehen; 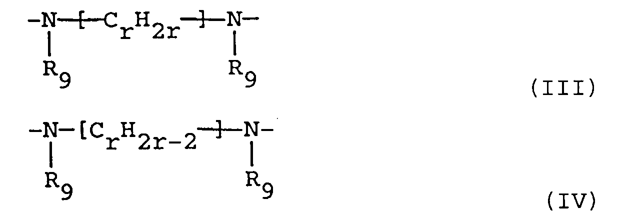 worin r eine ganze Zahl von 2 bis 14 ist und R₉ aus Wasserstoff; (C₁-C₄)-Alkyl; (C₂-C₆)-Alkenyl; (C₁-C₄)-Hydroxyalkyl ausgewählt ist; 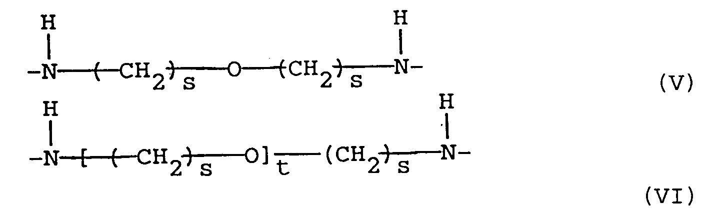 worin s eine ganze Zahl von 2 bis 5 ist und t eine ganze Zahl von 1 bis 3 darstellt; 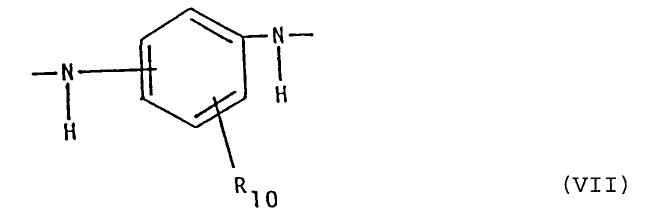 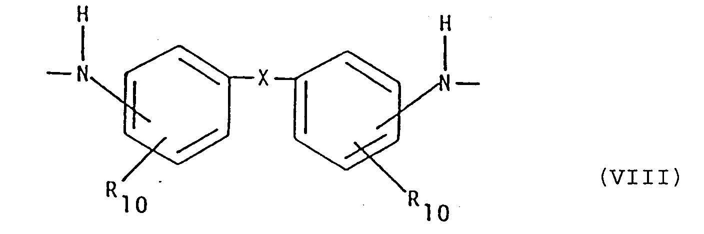 worin X eine direkte Kohlenstoff-Kohlenstoff-Bindung; O; S; S-S; SO; SO₂; NH; NHSO₂; NHCO; N=N; oder CH₂ darstellt; und
R₁₀ für Wasserstoff; Hydroxy; (C₁-C₄)-Alkyl oder (C₁-C₄)-Alkoxy steht; 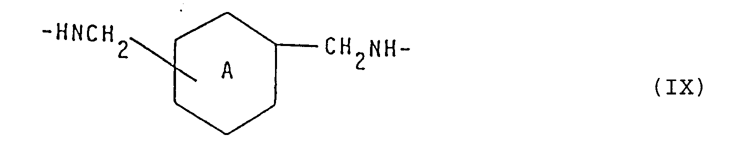 worin A entweder ein gesättigter oder ein ungesättigter Ring ist; 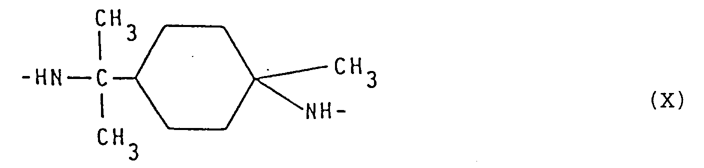 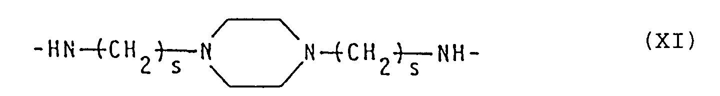 worin s eine ganze Zahl von 2 bis 5 darstellt; mit der Maßgabe, daß wenn R für (C₁-C₈)-Alkyl, (C₆-C₁₂)-Cycloalkyl, (C₇-C₁₂)-Aralkyl oder eine Phenyl- oder Naphthylgruppe, gegebenenfalls mit inerten Resten substituiert, steht und R¹ und R² (C₁-C₃) -Alkyl- oder (C₆-C₁₂)-Cycloalkylgruppen darstellen oder -NR¹R² eine Piperidinyl- oder Morpholinylgruppe repräsentiert, Z nicht die Formel (II), in der alle vier Gruppen R₈ Wasserstoff sind, repräsentieren kann;
wenn b eine ganze Zahl von 1 bis 5 ist:
die Einheit der Formel 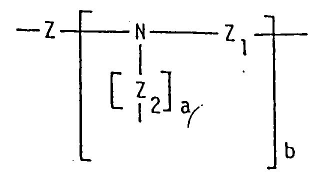
ein mehrwertiger Rest ist, der aus denjenigen mit den folgenden Formeln ausgewählt ist; 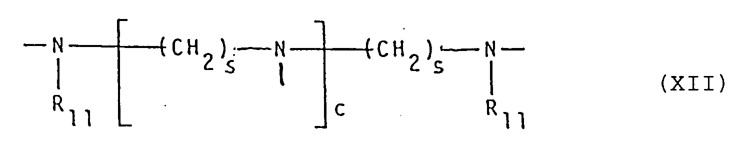 worin R₁₁ Wasserstoff oder (C₁-C₄)-Alkyl bedeutet;
c eine ganze Zahl von 1 bis 5 ist;
die Indices s, gleich oder verschieden voneinander, ganze Zahlen von 2 bis 5 sind; 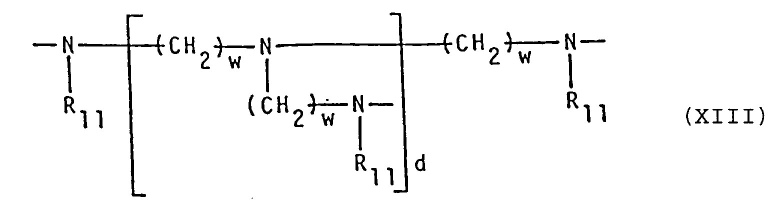 worin R₁₁ für Wasserstoff oder (C₁-C₄)-Alkyl steht;
w eine ganze Zahl von 2 bis 4 ist; und
d 1 oder 2 darstellt.

2. Verbindungen nach Anspruch 1, in denen mindestens eine der Gruppen R₁ und R₂ in der allgemeinen Formel (I) eine Gruppe der Formel
⁅CₘH₂ₘ⁆O-R₅
ist, worin:
m eine ganze Zahl von 2 bis 4 darstellt; und
R₅ für Wasserstoff oder (C₁-C₄)-Alkyl steht.

3. Verbindungen nach irgendeinem der Ansprüche 1 und 2, worin R in der allgemeinen Formel (I) Wasserstoff oder eine Gruppe der Formel
⁅CₙH₂ₙ⁆Y
darstellt, worin n eine ganze Zahl von 1 bis 4 ist und Y Wasserstoff repräsentiert.

4. Verfahren zur Herstellung von Verbindungen der allgemeinen Formel (I) gemäß irgendeinem der Ansprüche 1 bis 3, umfassend die Hydrolyse von Zwischenprodukten der allgemeinen Formel (XIV): 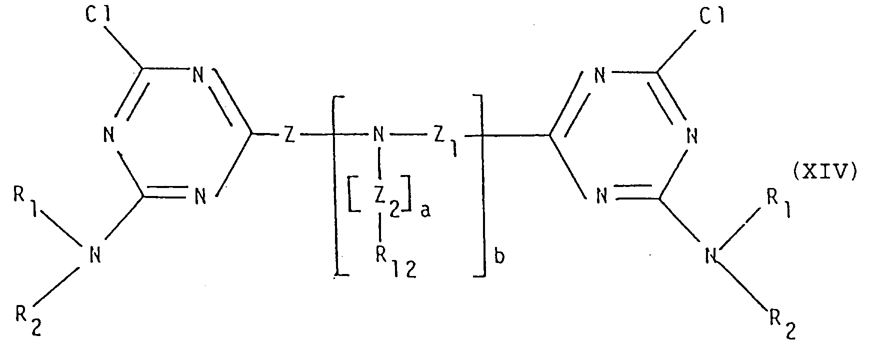 worin R₁, R₂, Z, Z₁, Z₂, a und b wie in Anspruch 1 definiert sind und R₁₂ für Wasserstoff oder eine Gruppe der Formel 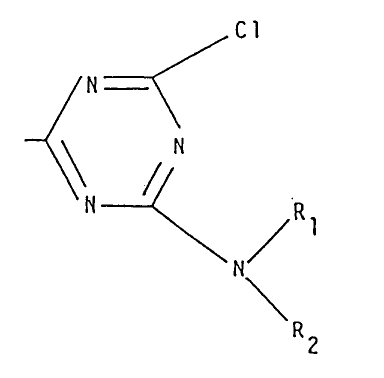 steht und seine Bedeutung in jeder wiederkehrenden Einheit verschieden sein kann; oder die Kondensation von Zwischenprodukten der allgemeinen Formel (XIV) mit einem Reagenz der allgemeinen Formel (XV):
R-OH (XV)
worin R die in Anspruch 1 angegebenen Bedeutungen aufweist, aber nicht Wasserstoff sein kann.

5. Verfahren nach Anspruch 4, in welchem die Hydrolysereaktion entweder in Anwesenheit einer Säure bei Temperaturen von etwa 60 bis etwa 100°C oder in Anwesenheit einer Base bei Temperaturen von etwa 100 bis etwa 180°C durchgeführt wird.

6. Verfahren nach Anspruch 4, in welchem die Kondensationsreaktion in einem Lösungsmittel und/oder in einem Überschuß an Reagenz der allgemeinen Formel (XV) in Anwesenheit einer Base bei Temperaturen von etwa 60 bis etwa 150°C durchgeführt wird.

7. Verfahren zur Herstellung von Verbindungen der allgemeinen Formel (I) nach irgendeinem der Ansprüche 1 bis 3, wobei R von Wasserstoff verschieden ist, umfassend die Umsetzung von einem Mol Polyamin der allgemeinen Formel (XVI): 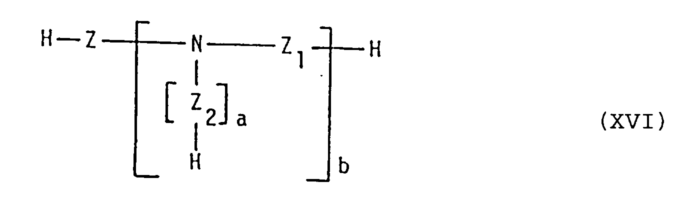 worin Z, Z₁, Z₂, a und b wie in Anspruch 1 definiert sind; mit (2+b) Mol oder weniger des Zwischenproduktes der allgemeinen Formel (XXI): 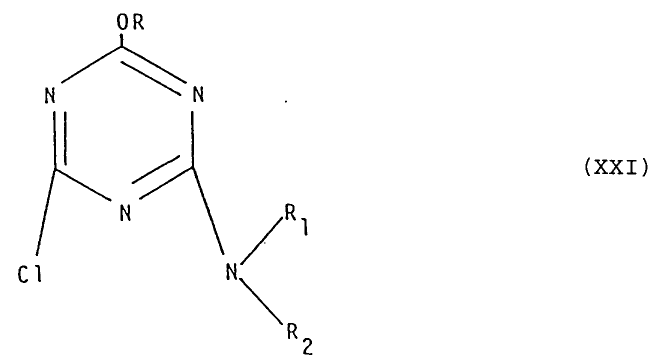 worin R, R₁ und R₂ die in Anspruch 1 angegebenen Bedeutungen aufweisen, wobei R von Wasserstoff verschieden ist.

8. Selbstverlöschende polymere Zusammensetzungen, umfassend:
a) 91 bis 40 Gewichtsteile thermoplastisches Polymer und/oder elastomere Eigenschaften zeigendes Polymer;
b) 6 bis 33 Gewichtsteile eines oder mehrerer Ammonium- und/oder Aminphosphate und/oder -phosphonate;
c) 3 bis 27 Gewichtsteile einer oder mehrerer Verbindungen der allgemeinen Formel (I) wie in irgendeinem der Ansprüche 1 bis 4 definiert.

9. Zusammensetzungen nach Anspruch 8, in denen die Ammoniumphosphate (b) die allgemeine Formel (NH₄)ₙ₊₂PₙO₃ₙ₊₁ aufweisen, worin n eine ganze Zahl von mindestens 2 darstellt; oder die allgemeine Formel (NH₄PO₃)ₙ aufweisen, worin n im Bereich von 50 bis 500 liegt.

10. Zusammensetzungen nach irgendeinem der Ansprüche 8 und 9, in denen die Aminphosphate (b) aus Dimethylammoniumphosphat; Diethylammoniumphosphat; Ethylendiaminphosphat; Melaminorthophosphat; und Melaminpyrophosphat ausgewählt sind.

11. Zusammensetzungen nach irgendeinem der Ansprüche 8 bis 10, in denen die Ammoniumphosphonate (b) die mono- und polysubstituierten sind und aus von Mono- und Polyphosphonsäuren abgeleiteten Salzen ausgewählt sind.

12. Zusammensetzungen nach irgendeinem der Ansprüche 8 bis 11, in denen das Polymer (a) ausgewählt ist aus Acrylnitril/Butadien/Styrol (ABS)-Terpolymeren; Styrol/Acrylnitril (SAN)-Copolymeren; Polyurethanen; Poly(ethylenterephthalat); Poly(butylenterephthalat); Polyamiden; und Polymeren und Copolymeren von Olefinen der allgemeinen Formel R'-CH=CH₂, worin R' für Wasserstoff, eine (C₁-C₈)-Alkyl- oder (C₆-C₁₀)-Arylgruppe steht, insbesondere:
(1) isotaktischem oder überwiegend isotaktischem Polypropylen;
(2) HDPE-, LLDPE- und LDPE-Polyethylen;
(3) kristallinen Copolymeren von Propylen und kleineren Mengen an Ethylen und/oder anderen α-Olefinen wie beispielsweise Buten-1, Hexen-1, Octen-1 und 4-Methylpenten-1;
(4) heterophasischen Zusammensetzungen, die umfassen (A) eine Fraktion aus Propylen-Homopolymer oder aus Propylen-Copolymeren wie unter (3) angegeben und (B) eine Copolymer-Fraktion, die aus elastomeren Copolymeren von Ethylen und einem α-Olefin, gegebenenfalls kleinere Mengen eines Diens enthaltend, zusammengesetzt ist, wobei das α-Olefin vorzugsweise aus Propylen und Buten-1 ausgewählt ist;
(5) elastomeren Copolymeren von Ethylen und α-Olefin(en), gegebenenfalls kleinere Mengen an Dien enthaltend.

13. Formkörper, erhältlich aus Zusammensetzungen gemäß irgendeinem der Ansprüche 8 bis 12.

## Patentansprüche (Patentansprüche für folgende(n) Vertragsstaat(en): DK)

1. Amelin-Derivate der allgemeinen Formel (I): 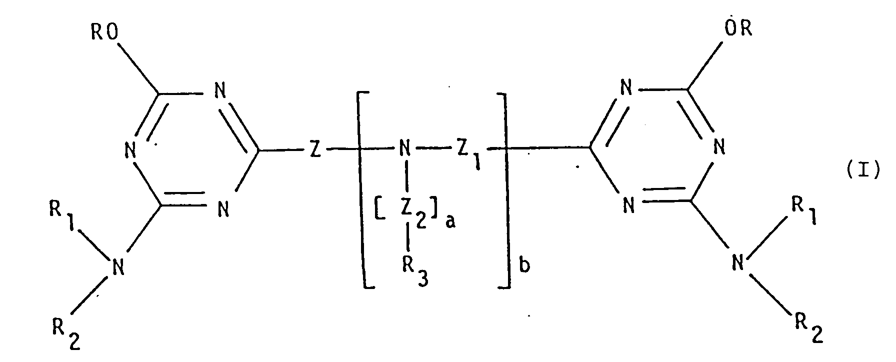 worin:
R = H; (C₂-C₆)-Alkenyl; (C₆-C₁₂)-Cycloalkyl; (C₆-C₁₂)-Alkylcycloalkyl; (C₆-C₁₂)-Aryl; (C₇-C₁₂)-Aralkyl; oder ⁅CₙH₂ₙ⁆Y,
worin n eine ganze Zahl von 1 bis 8 ist und Y H; CN; -O-(C₁-C₄)-Alkyl; -O-(C₂-C₄)-Alkenyl; (C₆-C₁₂)-Cycloalkyl; (C₆-C₁₂)-Alkylcycloalkyl; -O-(C₆-C₁₂)-Aryl; oder -N(R₄)₂ darstellt, worin die Gruppen R₄, gleich oder verschieden voneinander, für (C₁-C₄)-Alkyl oder (C₃-C₄)-Alkenyl stehen oder die Einheit -N(R₄)₂ durch einen N-heterocyclischen Rest ersetzt ist, der über das Stickstoffatom an die Alkylkette gebunden ist und aus Pyrrolidyl; Piperidyl; Morpholyl; Thiomorpholyl; Piperazyl; 4-Methylpiperazyl und 4-Ethylpiperazyl ausgewählt ist;
die Gruppen R₁ und R₂, gleich oder verschieden voneinander und dieselben oder verschiedene Bedeutungen in jedem Triazin-Ring aufweisend, ausgewählt sind aus H; (C₁-C₁₈)-Alkyl; (C₂-C₈)-Alkenyl; (C₆-C₁₆)-Cycloalkyl; (C₆-C₁₆)-Alkylcycloalkyl; ⁅CₘH₂ₘ⁆O-R₅; und 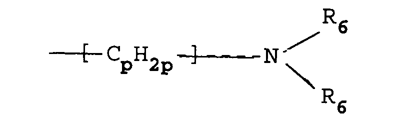 worin:
m = ganze Zahl von 2 bis 8;
p = ganze Zahl von 2 bis 6;
R₅ = H; (C₁-C₈)-Alkyl; (C₂-C₆)-Alkenyl;
⁅C_{q}H_{2q}⁆O-R₇, wobei q eine ganze Zahl von 1 bis 4 ist und R₇ H oder (C₁-C₄)-Alkyl; (C₆-C₁₂)-Cycloalkyl; und (C₆-C₁₂)-Alkylcycloalkyl darstellt;
die Gruppen R₆, gleich oder verschieden voneinander, ausgewählt sind aus H; (C₁-C₈)-Alkyl; (C₂-C₆)-Alkenyl; (C₆-C₁₂)-Cycloalkyl; (C₆-C₁₂)-Alkylcycloalkyl; und (C₁-C₄)-Hydroxyalkyl; oder die Einheit -N(R₆)₂ durch einen N-heterocyclischen Rest ersetzt ist, der über das Stickstoffatom an die Alkylkette gebunden ist und ausgewählt ist aus Aziridyl; Pyrrolidyl; Piperidyl; Morpholyl; Thiomorpholyl; Piperazyl; 4-Methylpiperazyl und 4-Ethylpiperazyl;
oder in der allgemeinen Formel (I) eine oder beide der Einheiten -NR₁R₂ durch einen N-heterocyclischen Rest ersetzt sind, der über das Stickstoffatom an den Triazin-Ring gebunden ist und ausgewählt ist aus Aziridyl; Pyrrolidyl; Piperidyl; Morpholyl; Thiomorpholyl; Piperazyl; 4-Methylpiperazyl; 4-Ethylpiperazyl; 2-Methylpiperazyl; 2,5-Dimethylpiperazyl; 2,3,5,6-Tetramethylpiperazyl; 2,2,5,5-Tetramethylpiperazyl; 2-Ethylpiperazyl; und 2,5-Diethylpiperazyl;
a 0 oder 1 ist;
b 0 oder eine ganze Zahl von 1 bis 5 ist;
R₃ Wasserstoff oder eine Gruppe der Formel 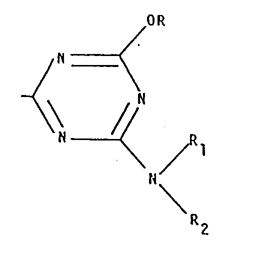 darstellt und seine Bedeutung in jeder wiederkehrenden Einheit verschieden sein kann;
wenn b 0 ist:
Z ein zweiwertiger Rest ist, der aus denjenigen mit den folgenden Formeln ausgewählt ist: 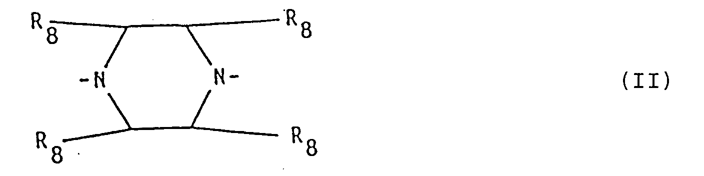 worin die Gruppen R₈, gleich oder verschieden voneinander, für Wasserstoff oder (C₁-C₄)-Alkyl stehen; 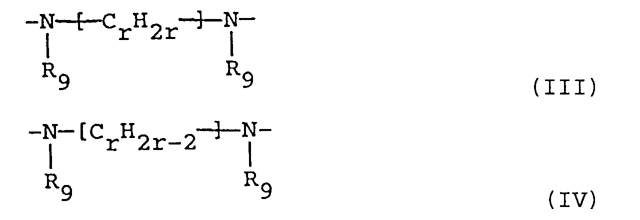 worin r eine ganze Zahl von 2 bis 14 ist und R₉ aus Wasserstoff; (C₁-C₄)-Alkyl; (C₂-C₆)-Alkenyl; (C₁-C₄)-Hydroxyalkyl ausgewählt ist; 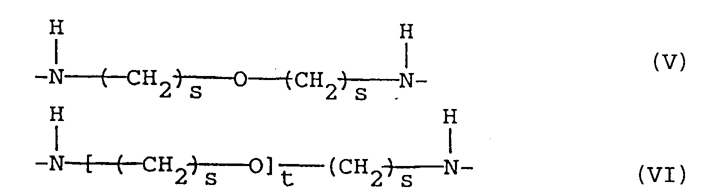 worin s eine ganze Zahl von 2 bis 5 ist und t eine ganze Zahl von 1 bis 3 darstellt; 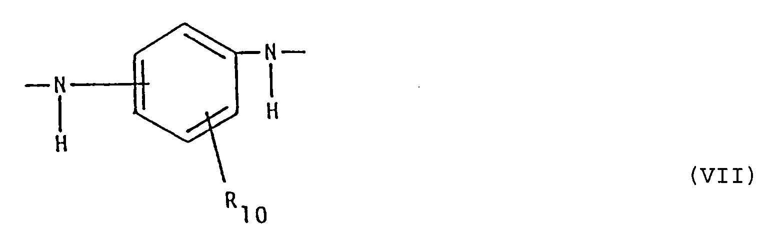 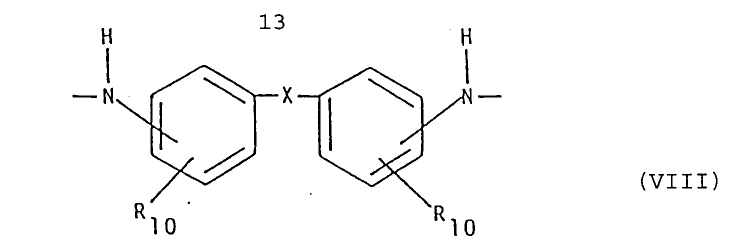 worin X eine direkte Kohlenstoff-Kohlenstoff-Bindung; O; S; S-S; SO; SO₂; NH; NHSO₂; NHCO; N=N; oder CH₂ darstellt; und
R₁₀ für Wasserstoff; Hydroxy; (C₁-C₄)-Alkyl oder (C₁-C₄)-Alkoxy steht; 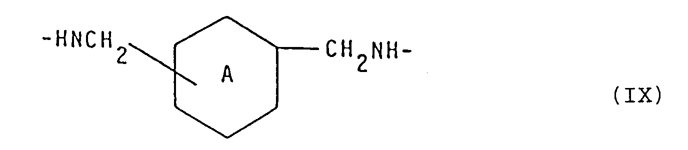 worin A entweder ein gesättigter oder ein ungesättigter Ring ist; 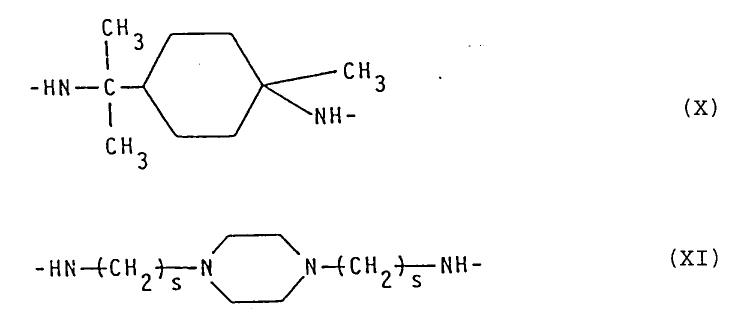 worin s eine ganze Zahl von 2 bis 5 darstellt;
wenn b eine ganze Zahl von 1 bis 5 ist:
die Einheit der Formel 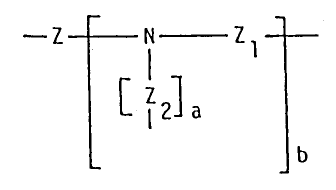 ein mehrwertiger Rest ist, der aus denjenigen mit den folgenden Formeln ausgewählt ist; 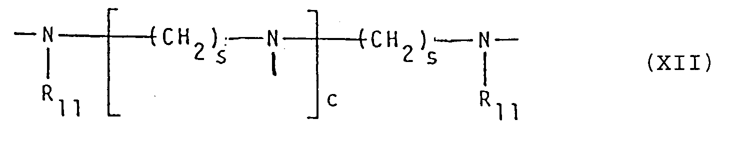
worin R₁₁ Wasserstoff oder (C₁-C₄)-Alkyl bedeutet;
c eine ganze Zahl von 1 bis 5 ist;
die Indices s, gleich oder verschieden voneinander, ganze Zahlen von 2 bis 5 sind; 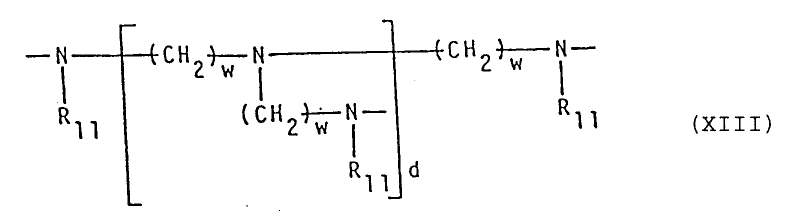 worin R₁₁ für Wasserstoff oder (C₁-C₄)-Alkyl steht;
w eine ganze Zahl von 2 bis 4 ist; und
d 1 oder 2 darstellt.

2. Verbindungen nach Anspruch 1, in denen mindestens eine der Gruppen R₁ und R₂ in der allgemeinen Formel (I) eine Gruppe der Formel
⁅CₘH₂ₘ⁆O-R₅
ist, worin:
m eine ganze Zahl von 2 bis 4 darstellt; und
R₅ für Wasserstoff oder (C₁-C₄)-Alkyl steht.

3. Verbindungen nach irgendeinem der Ansprüche 1 und 2, worin R in der allgemeinen Formel (I) Wasserstoff oder eine Gruppe der Formel
⁅CₙH₂ₙ⁆Y
darstellt, worin n eine ganze Zahl von 1 bis 4 ist und Y Wasserstoff repräsentiert.

4. Verfahren zur Herstellung von Verbindungen der allgemeinen Formel (I) gemäß irgendeinem der Ansprüche 1 bis 3, umfassend die Hydrolyse von Zwischenprodukten der allgemeinen Formel (XIV): 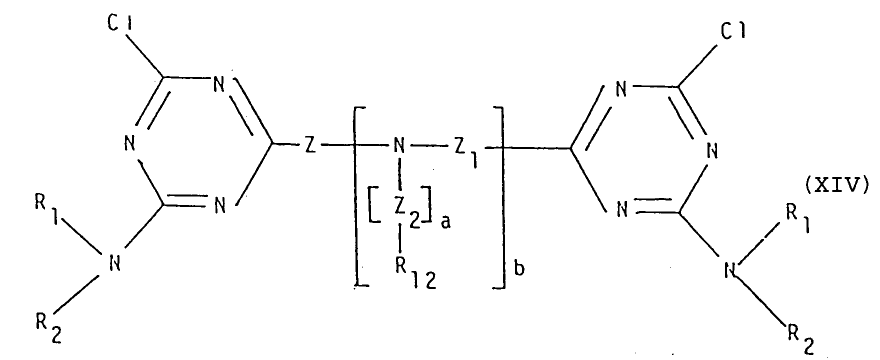 worin R₁, R₂, Z, Z₁, Z₂, a und b wie in Anspruch 1 definiert sind und R₁₂ für Wasserstoff oder eine Gruppe der Formel 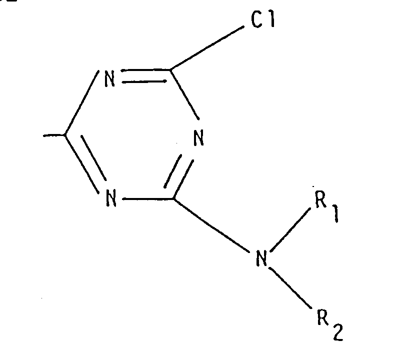 steht und seine Bedeutung in jeder wiederkehrenden Einheit verschieden sein kann; oder die Kondensation von Zwischenprodukten der allgemeinen Formel (XIV) mit einem Reagenz der allgemeinen Formel (XV):
R-OH (XV)
worin R die in Anspruch 1 angegebenen Bedeutungen aufweist, aber nicht Wasserstoff sein kann.

5. Verfahren nach Anspruch 4, in welchem die Hydrolysereaktion entweder in Anwesenheit einer Säure bei Temperaturen von etwa 60 bis etwa 100°C oder in Anwesenheit einer Base bei Temperaturen von etwa 100 bis etwa 180°C durchgeführt wird.

6. Verfahren nach Anspruch 4, in welchem die Kondensationsreaktion in einem Lösungsmittel und/oder in einem Überschuß an Reagenz der allgemeinen Formel (XV) in Anwesenheit einer Base bei Temperaturen von etwa 60 bis etwa 150°C durchgeführt wird.

7. Verfahren zur Herstellung von Verbindungen der allgemeinen Formel (I) nach irgendeinem der Ansprüche 1 bis 3, wobei R von Wasserstoff verschieden ist, umfassend die Umsetzung von einem Mol Polyamin der allgemeinen Formel (XVI): 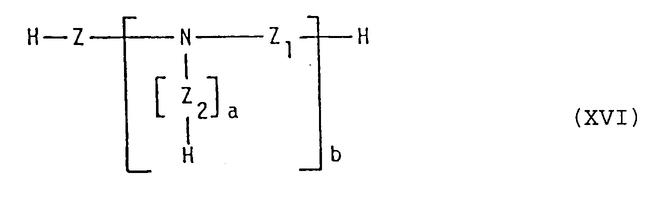 worin Z, Z₁, Z₂, a und b wie in Anspruch 1 definiert sind; mit (2+b) Mol oder weniger des Zwischenproduktes der allgemeinen Formel (XXI): 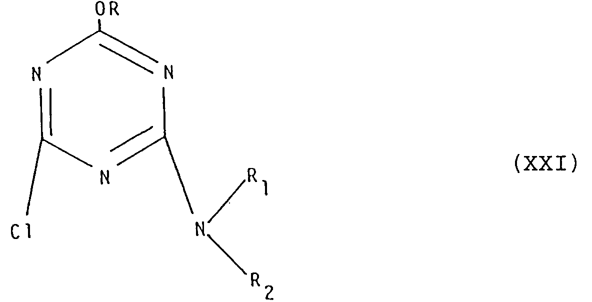 worin R, R₁ und R₂ die in Anspruch 1 angegebenen Bedeutungen aufweisen, wobei R von Wasserstoff verschieden ist.

8. Selbstverlöschende polymere Zusammensetzungen, umfassend:
a) 91 bis 40 Gewichtsteile thermoplastisches Polymer und/oder elastomere Eigenschaften zeigendes Polymer;
b) 6 bis 33 Gewichtsteile eines oder mehrerer Ammonium- und/oder Aminphosphate und/oder -phosphonate;
c) 3 bis 27 Gewichtsteile einer oder mehrerer Verbindungen der allgemeinen Formel (I) wie in irgendeinem der Ansprüche 1 bis 4 definiert.

9. Zusammensetzungen nach Anspruch 8, in denen die Ammoniumphosphate (b) die allgemeine Formel (NH₄)ₙ₊₂PₙO₃ₙ₊₁ aufweisen, worin n eine ganze Zahl von mindestens 2 darstellt; oder die allgemeine Formel (NH₄PO₃)ₙ aufweisen, worin n im Bereich von 50 bis 500 liegt.

10. Zusammensetzungen nach irgendeinem der Ansprüche 8 und 9, in denen die Aminphosphate (b) aus Dimethylammoniumphosphat; Diethylammoniumphosphat; Ethylendiaminphosphat; Melaminorthophosphat; und Melaminpyrophosphat ausgewählt sind.

11. Zusammensetzungen nach irgendeinem der Ansprüche 8 bis 10, in denen die Ammoniumphosphonate (b) die mono- und polysubstituierten sind und aus von Mono- und Polyphosphonsäuren abgeleiteten Salzen ausgewählt sind.

12. Zusammensetzungen nach irgendeinem der Ansprüche 8 bis 11, in denen das Polymer (a) ausgewählt ist aus Acrylnitril/Butadien/Styrol (ABS)-Terpolymeren; Styrol/Acrylnitril (SAN)-Copolymeren; Polyurethanen; Poly(ethylenterephthalat); Poly(butylenterephthalat); Polyamiden; und Polymeren und Copolymeren von Olefinen der allgemeinen Formel R'-CH=CH₂, worin R' für Wasserstoff, eine (C₁-C₈)-Alkyl- oder (C₆-C₁₀)-Arylgruppe steht, insbesondere:
(1) isotaktischem oder überwiegend isotaktischem Polypropylen;
(2) HDPE-, LLDPE- und LDPE-Polyethylen;
(3) kristallinen Copolymeren von Propylen und kleineren Mengen an Ethylen und/oder anderen α-Olefinen wie beispielsweise Buten-1, Hexen-1, Octen-1 und 4-Methylpenten-1;
(4) heterophasischen Zusammensetzungen, die umfassen (A) eine Fraktion aus Propylen-Homopolymer oder aus Propylen-Copolymeren wie unter (3) angegeben und (B) eine Copolymer-Fraktion, die aus elastomeren Copolymeren von Ethylen und einem α-Olefin, gegebenenfalls kleinere Mengen eines Diens enthaltend, zusammengesetzt ist, wobei das α-Olefin vorzugsweise aus Propylen und Buten-1 ausgewählt ist;
(5) elastomeren Copolymeren von Ethylen und α-Olefin(en), gegebenenfalls kleinere Mengen an Dien enthaltend.

13. Formkörper, erhältlich aus Zusammensetzungen gemäß irgendeinem der Ansprüche 8 bis 12.

## Patentansprüche (Patentansprüche für folgende(n) Vertragsstaat(en): ES)

1. Verfahren zur Herstellung von Amelin-Derivaten der allgemeinen Formel (I): 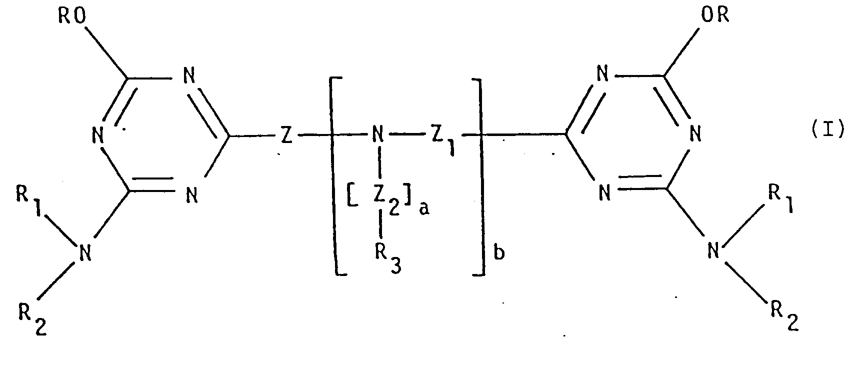 worin:
R = H; (C₂-C₆)-Alkenyl; (C₆-C₁₂)-Cycloalkyl; (C₆-C₁₂)-Alkylcycloalkyl; (C₆-C₁₂)-Aryl; (C₇-C₁₂)-Aralkyl; oder ⁅CₙH₂ₙ⁆Y,
worin n eine ganze Zahl von 1 bis 8 ist und Y H; CN; -O-(C₁-C₄)-Alkyl; -O-(C₂-C₄)-Alkenyl; (C₆-C₁₂)-Cycloalkyl; (C₆-C₁₂)-Alkylcycloalkyl; -O-(C₆-C₁₂)-Aryl; oder -N(R₄)₂ darstellt, worin die Gruppen R₄, gleich oder verschieden voneinander, für (C₁-C₄)-Alkyl oder (C₃-C₄)-Alkenyl stehen oder die Einheit -N(R₄)₂ durch einen N-heterocyclischen Rest ersetzt ist, der über das Stickstoffatom an die Alkylkette gebunden ist und aus Pyrrolidyl; Piperidyl; Morpholyl; Thiomorpholyl; Piperazyl; 4-Methylpiperazyl und 4-Ethylpiperazyl ausgewählt ist;
die Gruppen R₁ und R₂, gleich oder verschieden voneinander und dieselben oder verschiedene Bedeutungen in jedem Triazin-Ring aufweisend, ausgewählt sind aus H; (C₁-C₁₈)-Alkyl; (C₂-C₈)-Alkenyl; (C₆-C₁₆)-Cycloalkyl; (C₆-C₁₆)-Alkylcycloalkyl; ⁅CₘH₂ₘ⁆O-R₅; und 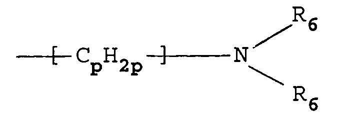 worin:
m = ganze Zahl von 2 bis 8;
p = ganze Zahl von 2 bis 6;
R₅ = H; (C₁-C₈)-Alkyl; (C₂-C₆)-Alkenyl;
⁅C_{q}H_{2q}⁆O-R₇, wobei q eine ganze Zahl von 1 bis 4 ist und R₇ H oder (C₁-C₄)-Alkyl; (C₆-C₁₂)-Cycloalkyl; und (C₆-C₁₂)-Alkylcycloalkyl darstellt;
die Gruppen R₆, gleich oder verschieden voneinander, ausgewählt sind aus H; (C₁-C₈)-Alkyl; (C₂-C₆)-Alkenyl; (C₆-C₁₂)-Cycloalkyl; (C₆-C₁₂)-Alkylcycloalkyl; und (C₁-C₄)-Hydroxyalkyl; oder die Einheit -N(R₆)₂ durch einen N-heterocyclischen Rest ersetzt ist, der über das Stickstoffatom an die Alkylkette gebunden ist und ausgewählt ist aus Aziridyl; Pyrrolidyl; Piperidyl; Morpholyl; Thiomorpholyl; Piperazyl; 4-Methylpiperazyl und 4-Ethylpiperazyl;
oder in der allgemeinen Formel (I) eine oder beide der Einheiten -NR₁R₂ durch einen N-heterocyclischen Rest ersetzt sind, der über das Stickstoffatom an den Triazin-Ring gebunden ist und ausgewählt ist aus Aziridyl; Pyrrolidyl; Piperidyl; Morpholyl; Thiomorpholyl; Piperazyl; 4-Methylpiperazyl; 4-Ethylpiperazyl; 2-Methylpiperazyl; 2,5-Dimethylpiperazyl; 2,3,5,6-Tetramethylpiperazyl; 2,2,5,5-Tetramethylpiperazyl; 2-Ethylpiperazyl; und 2,5-Diethylpiperazyl;
a 0 oder 1 ist;
b 0 oder eine ganze Zahl von 1 bis 5 ist;
R₃ Wasserstoff oder eine Gruppe der Formel 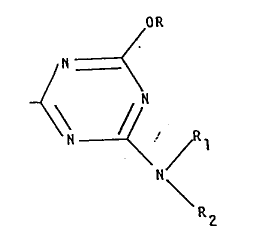 darstellt und seine Bedeutung in jeder wiederkehrenden Einheit verschieden sein kann;
wenn b 0 ist:
Z ein zweiwertiger Rest ist, der aus denjenigen mit den folgenden Formeln ausgewählt ist: 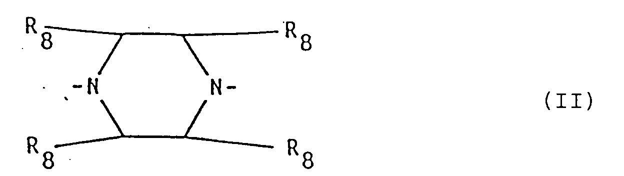 worin die Gruppen R₈, gleich oder verschieden voneinander, für Wasserstoff oder (C₁-C₄)-Alkyl stehen; 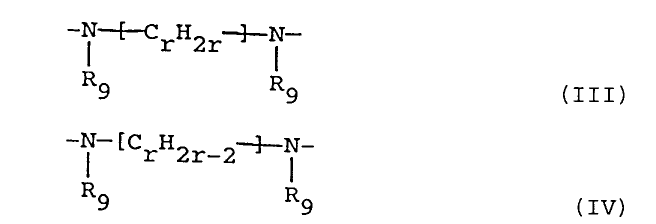 worin r eine ganze Zahl von 2 bis 14 ist und R₉ aus Wasserstoff; (C₁-C₄)-Alkyl; (C₂-C₆)-Alkenyl; (C₁-C₄)-Hydroxyalkyl ausgewählt ist; 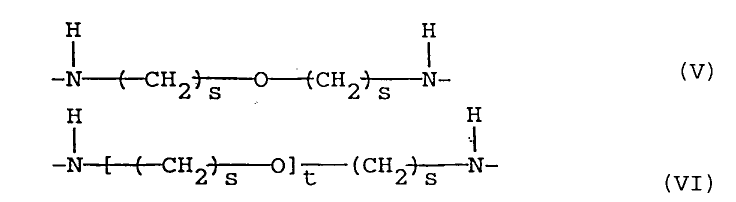 worin s eine ganze Zahl von 2 bis 5 ist und t eine ganze Zahl von 1 bis 3 darstellt; 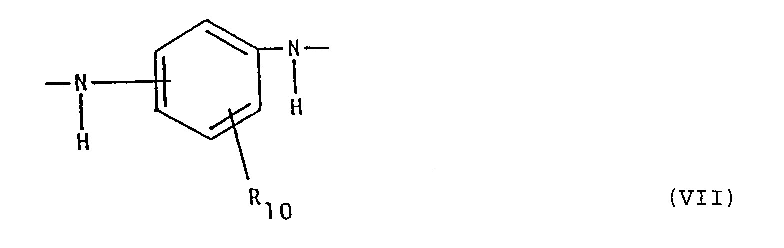 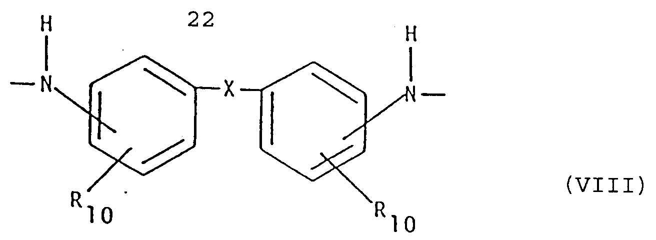 worin X eine direkte Kohlenstoff-Kohlenstoff-Bindung; O; S; S-S; SO; SO₂; NH; NHSO₂; NHCO; N=N; oder CH₂ darstellt; und
R₁₀ für Wasserstoff; Hydroxy; (C₁-C₄)-Alkyl oder (C₁-C₄)-Alkoxy steht; 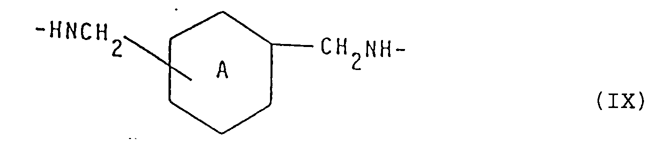 worin A entweder ein gesättigter oder ein ungesättigter Ring ist; 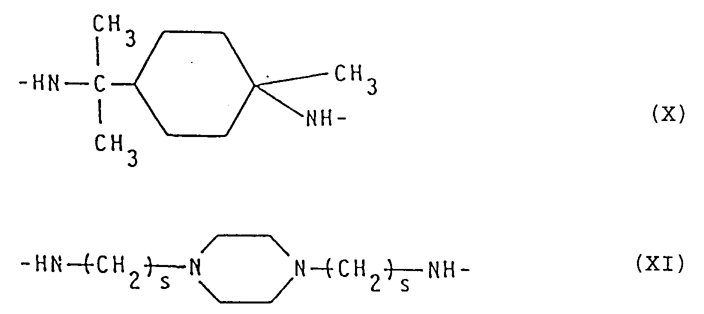 worin s eine ganze Zahl von 2 bis 5 darstellt; mit der Maßgabe, daß wenn R für (C₁-C₈)-Alkyl, (C₆-C₁₂)-Cycloalkyl, (C₇-C₁₂)-Aralkyl oder eine Phenyl- oder Naphthylgruppe, gegebenenfalls mit inerten Resten substituiert, steht und R¹ und R² (C₁-C₃)-Alkyl- oder (C₆-C₁₂)-Cycloalkylgruppen darstellen oder -NR¹R² eine Piperidinyl- oder Morpholinylgruppe repräsentiert, Z nicht die Formel (II), in der alle vier Gruppen R₈ Wasserstoff sind, repräsentieren kann;
wenn b eine ganze Zahl von 1 bis 5 ist:
die Einheit der Formel 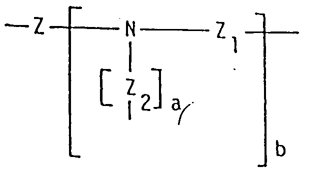 ein mehrwertiger Rest ist, der aus denjenigen mit den folgenden Formeln ausgewählt ist; 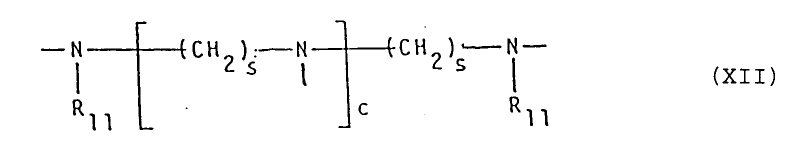 worin R₁₁ Wasserstoff oder (C₁-C₄)-Alkyl bedeutet;
c eine ganze Zahl von 1 bis 5 ist;
die Indices s, gleich oder verschieden voneinander, ganze Zahlen von 2 bis 5 sind; 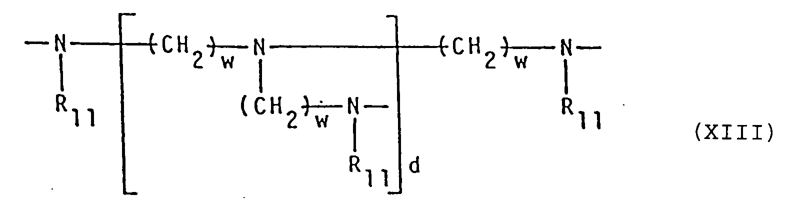 worin R₁₁ für Wasserstoff oder (C₁-C₄)-Alkyl steht;
w eine ganze Zahl von 2 bis 4 ist; und
d 1 oder 2 darstellt;
wobei das Verfahren umfaßt
(a) für R = H, die Hydrolyse von Zwischenprodukten der allgemeinen Formel (XIV): 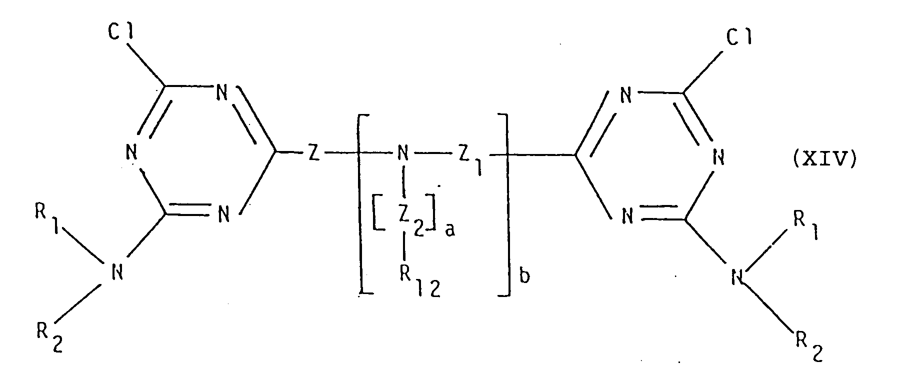 worin R₁, R₂, Z, Z₁, Z₂, a und b wie oben definiert sind und R₁₂ Wasserstoff oder eine Gruppe der Formel 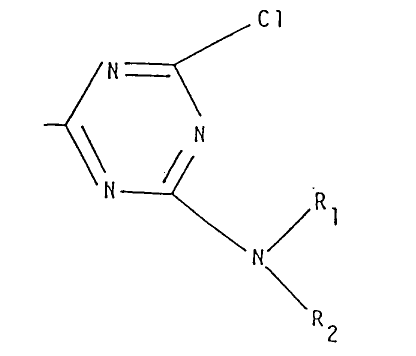 darstellt und seine Bedeutung in jeder wiederkehrenden Einheit verschieden sein kann; oder
(b) für R ≠ H, die Umsetzung von Zwischenprodukten der allgemeinen Formel (XIV) mit einem Reagenz der allgemeinen Formel (XV):
R-OH (XV)
worin R die oben angegebenen Bedeutungen aufweist, aber nicht Wasserstoff sein kann; oder
(c) für R ≠ H, die Umsetzung von einem Mol eines Polyamins der allgemeinen Formel (XVI): 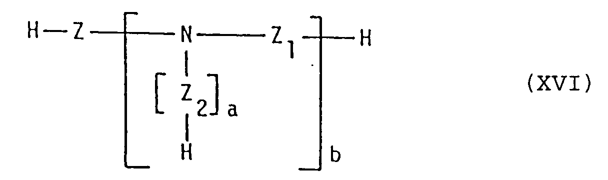 worin Z, Z₁, Z₂, a und b wie oben definiert sind; mit (2+b) Mol oder weniger des Zwischenproduktes der allgemeinen Formel (XXI): 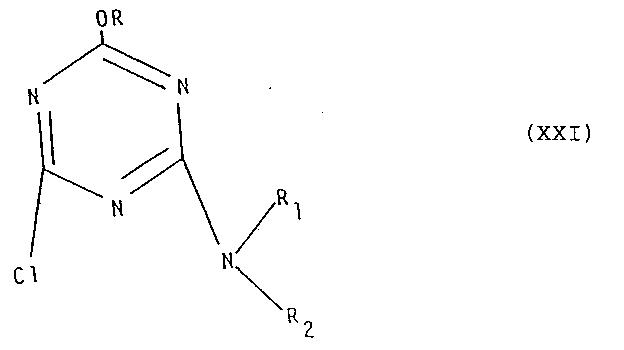 worin R, R₁ und R₂ die oben angegebenen Bedeutungen aufweisen, wobei R von Wasserstoff verschieden ist.

2. Verfahren nach Anspruch 1, in welchem Verbindungen hergestellt werden, in denen mindestens eine der Gruppen R₁ und R₂ in der allgemeinen Formel (I) eine Gruppe der Formel
⁅CₘH₂ₘ⁆O-R₅
ist;
worin:
m eine ganze Zahl von 2 bis 4 ist; und
R₅ für Wasserstoff oder (C₁-C₄)-Alkyl steht.

3. Verfahren nach irgendeinem der Ansprüche 1 und 2, in welchem R in der allgemeinen Formel (I) für Wasserstoff oder eine Gruppe der Formel
⁅CₙH₂ₙ⁆Y
steht, worin n eine ganze Zahl von 1 bis 4 ist und Y Wasserstoff darstellt.

4. Verfahren nach irgendeinem der Ansprüche 1 bis 3, Alternative (a), in welchem die Hydrolysereaktion in Anwesenheit einer Säure bei Temperaturen von etwa 60 bis etwa 100°C oder in Anwesenheit einer Base bei Temperaturen von etwa 100 bis etwa 180°C durchgeführt wird.

5. Verfahren nach irgendeinem der Ansprüche 1 bis 3, Alternative (b), in welchem die Kondensationsreaktion in einem Lösungsmittel und/oder in einem Überschuß des Reagenzes der allgemeinen Formel (XV) in Anwesenheit einer Base bei Temperaturen von etwa 60 bis etwa 150°C durchgeführt wird.

6. Selbstverlöschende polymere Zusammensetzungen, umfassend:
a) 91 bis 40 Gewichtsteile thermoplastisches Polymer und/oder elastomere Eigenschaften zeigendes Polymer;
b) 6 bis 33 Gewichtsteile eines oder mehrerer Ammonium- und/oder Aminphosphate und/oder -phosphonate;
c) 3 bis 27 Gewichtsteile einer oder mehrerer Verbindungen der allgemeinen Formel (I) wie in irgendeinem der Ansprüche 1 bis 3 definiert.

7. Zusammensetzungen nach Anspruch 6, in denen die Ammoniumphosphate (b) die allgemeine Formel (NH₄)ₙ₊₂PₙO₃ₙ₊₁ aufweisen, worin n eine ganze Zahl von mindestens 2 darstellt; oder die allgemeine Formel (NH₄PO₃)ₙ aufweisen, worin n im Bereich von 50 bis 500 liegt.

8. Zusammensetzungen nach irgendeinem der Ansprüche 6 und 7, in denen die Aminphosphate (b) aus Dimethylammoniumphosphat; Diethylammoniumphosphat; Ethylendiaminphosphat; Melaminorthophosphat; und Melaminpyrophosphat ausgewählt sind.

9. Zusammensetzungen nach irgendeinem der Ansprüche 6 bis 8, in denen die Ammoniumphosphonate (b) die mono- und polysubstituierten sind und aus von Mono- und Polyphosphonsäuren abgeleiteten Salzen ausgewählt sind.

10. Zusammensetzungen nach irgendeinem der Ansprüche 6 bis 9, in denen das Polymer (a) ausgewählt ist aus Acrylnitril/Butadien/Styrol (ABS)-Terpolymeren; Styrol/Acrylnitril (SAN)-Copolymeren; Polyurethanen; Poly(ethylenterephthalat); Poly(butylenterephthalat); Polyamiden; und Polymeren und Copolymeren von Olefinen der allgemeinen Formel R'-CH=CH₂, worin R' für Wasserstoff, eine (C₁-C₈)-Alkyl- oder (C₆-C₁₀)-Arylgruppe steht, insbesondere:
(1) isotaktischem oder überwiegend isotaktischem Polypropylen;
(2) HDPE-, LLDPE- und LDPE-Polyethylen;
(3) kristallinen Copolymeren von Propylen und kleineren Mengen an Ethylen und/oder anderen α-Olefinen wie beispielsweise Buten-1, Hexen-1, Octen-1 und 4-Methylpenten-1;
(4) heterophasischen Zusammensetzungen, die umfassen (A) eine Fraktion aus Propylen-Homopolymer oder aus Propylen-Copolymeren wie unter (3) angegeben und (B) eine Copolymer-Fraktion, die aus elastomeren Copolymeren von Ethylen und einem α-Olefin, gegebenenfalls kleinere Mengen eines Diens enthaltend, zusammengesetzt ist, wobei das α-Olefin vorzugsweise aus Propylen und Buten-1 ausgewählt ist;
(5) elastomeren Copolymeren von Ethylen und α-Olefin(en), gegebenenfalls kleinere Mengen an Dien enthaltend.

11. Formkörper, erhältlich aus Zusammensetzungen gemäß irgendeinem der Ansprüche 6 bis 10.

## Revendications (Revendications pour l'(les) Etat(s) contractant(s) suivant(s): AT, BE, CH, DE, FR, GB, LI, NL, SE)

1. Dérivés d'améline, de formule générale (I) : 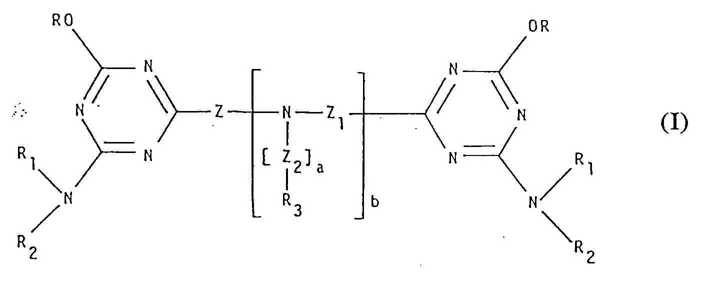 dans laquelle :
R représente un atome d'hydrogène, un groupe alcényle en C₂₋₆, cycloalkyle en C₆₋₁₂, alkyl-cycloalkyle en C₆₋₁₂, aryle en C₆₋₁₂ ou aralkyle en C₇₋₁₂, ou un groupe de formule -(CₙH₂ₙ)-Y où n représente un nombre entier valant de 1 à 8 et Y représente un atome d'hydrogène ou un groupe cyano, alcoxy en C₁₋₄, alcényloxy en C₂₋₄, cycloalkyle en C₆₋₁₂, alkyl-cycloalkyle en C₆₋₁₂ ou aryloxy en C₆₋₁₂, ou encore un groupe de formule -N(R₄)₂ où les symboles R₄ représentent des groupes alkyle en C₁₋₄ ou alcényle en C₃₋₄ qui peuvent être identiques ou différents l'un de l'autre, ou ce fragment -N(R₄)₂ est remplacé par un groupe hétérocyclique azoté, relié à la chaise alkyle par un atome d'azote et choisi parmi les groupes pyrrolidinyle, pipéridinyle, morpholino, thiomorpholino, pipérazinyle, 4-méthylpipérazinyle et 4-éthylpipérazinyle ;
les groupes représentés par R₁ et R₂, qui peuvent être identiques ou différents l'un de l'autre et identiques ou différents d'un cycle triazine à l'autre, sont choisis parmi les atomes d'hydrogène, les groupes alkyle en C₁₋₁₈, alcényle en C₂₋₈, cycloalkyle en C₆₋₁₆ ou alkyl-cycloalkyle en C₆₋₁₆, et les groupes de formule -(CₘH₂ₘ)-O-R₅ ou -(CₚH₂ₚ)-N(R₆)₂, où m représente un nombre entier valant de 2 à 8, p représente un nombre entier valant de 2 à 6, R₅ représente un atome d'hydrogène, un groupe alkyle en C₁₋₈ ou alcényle en C₂₋₆, ou un groupe de formule
-(C_{q}H_{2q})-O-R₇ où q représente un nombre entier valant de 1 à 4 et R₇ représente un atome d'hydrogène ou un groupe alkyle en C₁₋₄, cycloalkyle en C₆₋₁₂ ou alkyl-cycloalkyle en C₆₋₁₂, et les groupes représentés par R₆, qui peuvent être identiques ou différents l'un de l'autre, sont choisis parmi les atomes d'hydrogène et les groupes alkyle en C₁₋₈, alcényle en C₂₋₆, cycloalkyle en C₆₋₁₂, alkyl-cycloalkyle en C₆₋₁₂ ou hydroxyalkyle en C₁₋₄, ou encore le fragment -N(R₆)₂ est remplacé par un groupe hétérocyclique azoté, relié à la chaîne alkyle par un 'atome d'azote et choisi parmi les groupes aziridinyle, pyrrolidinyle, pipéridinyle, morpholino, thiomorpholino, pipérazinyle, 4-méthylpipérazinyle et 4-éthylpipérazinyle ;
ou bien, dans la formule générale (I), l'un des fragments -NR₁R₂ ou les deux sont remplacés chacun par un groupe hétérocyclique azoté, relié au cycle triazine par un atome d'azote et choisi parmi les groupes aziridinyle, pyrrolidinyle, pipéridinyle, morpholino, thiomorpholino, pipérazinyle, 4-méthylpipérazinyle, 4-éthylpipérazinyle, 2-méthylpipérazinyle, 2,5-diméthylpipérazinyle, 2,3,5,6-tétraméthylpipérazinyle, 2,2,5,5-tétraméthylpipérazinyle, 2-éthylpipérazinyle et 2,5-diéthylpipérazinyle ; a vaut 0 ou 1 ;
b vaut 0 ou représente un nombre entier valant de 1 à 5 ;
R₃ représente un atome d'hydrogène ou un groupe de formule 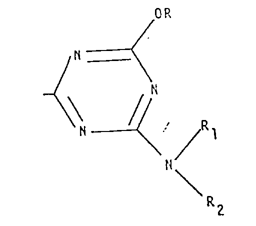 et sa signification peut varier d'un motif à l'autre ;
et si b vaut 0 :
Z représente un reste divalent, choisi parmi ceux qui correspondent aux formules suivantes : 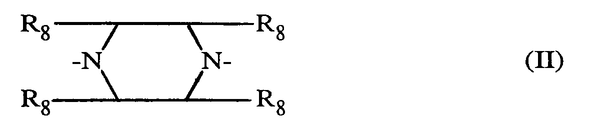 où les groupes représentés par R₈, qui peuvent être identiques ou différents les uns des autres, sont des atomes d'hydrogène ou des groupes alkyle en C₁₋₄ ; 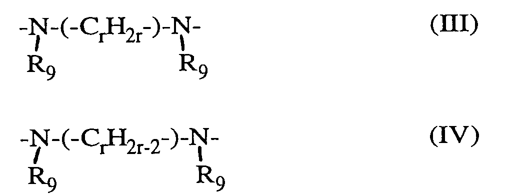 où r représente un nombre entier valant de 2 à 14 et R₉ est choisi parmi un atome d'hydrogène et un groupe alkyle en C₁₋₄, alcényle en C₂₋₆ ou hydroxyalkyle en C₁₋₄ ;
-NH-(-CH₂-)ₛ-O-(CH₂-)ₛ-NH- (V)
-NH-[(-CH₂-)ₛ-O]ₜ-(CH₂-)ₛ-NH- (VI)
où s représente un nombre entier valant de 2 à 5 et t représente un nombre entier valant de 1 à 3 ; 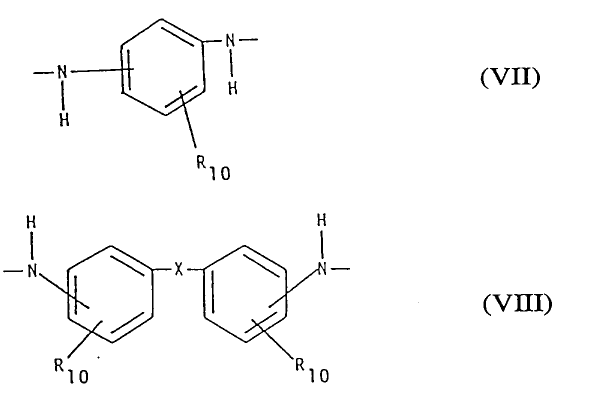 où X représente une liaison directe carbone-carbone, -O-, -S-, -S-S-, -SO-, -SO₂-, -NH-, -NH-SO₂-, -NH-CO-, -N=N-, ou -CH₂-, et R₁₀ représente un atome d'hydrogène ou un groupe hydroxy, alkyle en C₁₋₄ ou alcoxy en C₁₋₄ ; 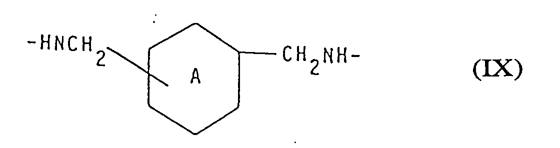 où A désigne un cycle saturé ou insaturé ; 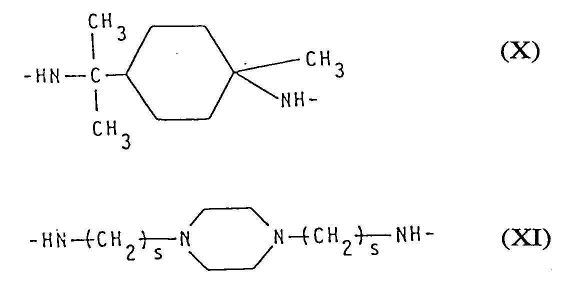 où s représente un nombre entier valant de 2 à 5 ;
sous réserve que, si R représente un groupe alkyle en C₁₋₈, cycloalkyle en C₆₋₁₂, aralkyle en C₇₋₁₂, phényle ou naphtyle, portant éventuellement des substituants inertes, et si R₁ et R₂ représentent des groupes alkyle en C₁₋₃ ou cycloalkyle en C₆₋₁₂ ou si -NR₁R₂ représente un groupe pipéridinyle ou morpholino, Z ne peut pas être un reste de formule (II) où les quatre symboles R₈ représenteraient tous des atomes d'hydrogène ;
si b représente un entier de 1 à 5 :
le fragment de formule 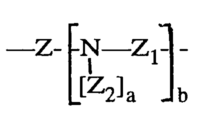 est un radical polyvalent choisi parmi ceux qui sont représentés par les formules suivantes : 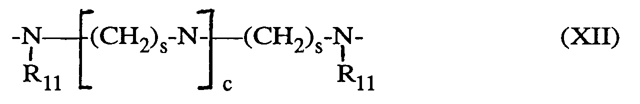 où R₁₁ représente un atome d'hydrogène ou un groupe alkyle en C₁₋₄, c représente un nombre entier valant de 1 à 5, et les indices s, qui peuvent être identiques ou différents les uns des autres, représentent des nombres entiers valant de 2 à 5 ; 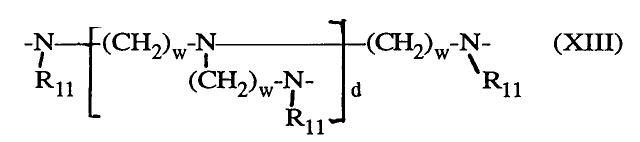 où R₁₁ représente un atome d'hydrogène ou un groupe alkyle en C₁₋₄, w représente un nombre entier valant de 2 à 4, et d vaut 1 ou 2.

2. Composés conformes à la revendication 1, dans lesquels au moins l'un des groupes R₁ et R₂, dans la formule générale (I), est un groupe de formule -(CₘH₂ₘ)-O-R₅ où m représente un nombre entier valant de 2 à 4 et R₅ représente un atome d'hydrogène ou un groupe alkyle en C₁₋₄.

3. Composés conformes à l'une des revendications 1 et 2, dans lesquels R, dans la formule générale (I), représente un atome d'hydrogène ou un groupe de formule -(CₙH₂ₙ)-Y où n représente un nombre entier valant de 1 à 4 et Y représente un atome d'hydrogène.

4. Procédé de préparation de composés de formule générale (I), conformes à l'une des revendications 1 à 3, qui comporte l'hydrolyse d'intermédiaires de formule générale (XIV) : 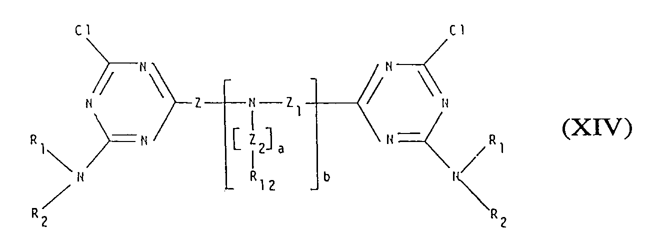 dans laquelle R₁, R₂, Z, Z₁, Z₂, a et b ont les significations définies dans la revendication 1 et R₁₂ représente un atome d'hydrogène ou un groupe de formule 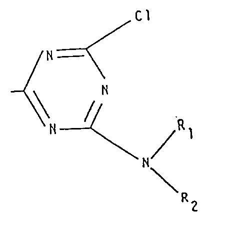 sa signification pouvant varier d'un motif répété à l'autre, ou la condensation d'intermédiaires de formule générale (XIV) avec un réactif de formule générale (XV)
R-OH (XV)
dans laquelle R possède les significations indiquées dans la revendication 1, sauf celle d'un atome d'hydrogène.

5. Procédé conforme à la revendication 4, dans lequel on effectue la réaction d'hydrolyse soit en présence d'un acide et à une température d'environ 60°C à environ 100°C, soit en présence d'une base et à une température d'environ 100°C à environ 180°C.

6. Procédé conforme à la revendication 4, dans lequel on effectue la réaction de condensation dans un solvant et/ou en présence d'un excès de réactif de formule générale (XV), en présence d'une base et à une température d'environ 60°C à environ 150°C.

7. Procédé de préparation de composés de formule générale (I), conformes à l'une des revendications 1 à 3, où R représente autre chose qu'un atome d'hydrogène, lequel procédé comporte la réaction de 1 mole d'une polyamine de formule générale (XVI) : 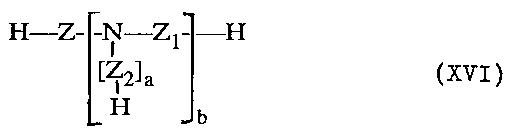 dans laquelle Z, Z₁, et Z₂, a et b ont les définitions indiquées dans la revendication 1,
avec au plus (2+b) moles de l'intermédiaire de formule générale (XXI) : 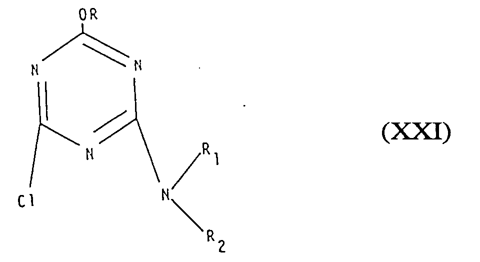 dans laquelle R, R₁ et R₂ ont les définitions indiquées dans la revendication 1, mais R représente autre chose qu'un atome d'hydrogène.

8. Compositions auto-extinguibles de polymères, comportant :
a) de 91 à 40 parties en poids d'un polymère thermoplastique et/ou d'un polymère présentant des propriétés d'élastomère,
b) de 6 à 33 parties en poids d'un ou de plusieurs phosphates et/ou phosphonates d'ammonium et/ou d'amine, et
c) de 3 à 27 parties en poids d'un ou de plusieurs composés de formule générale (I), conformes à l'une des revendications 1 à 4.

9. Compositions conformes à la revendication 8, dans lesquelles les phosphates d'ammonium (b) correspondent à la formule générale (NH₄)ₙ₊₂PₙO₃ₙ₊₁ où n représente un nombre entier valant au moins 2, ou bien à la formule générale (NH₄PO₃)ₙ où n vaut de 50 à 500.

10. Compositions conformes à l'une des revendications 8 et 9, dans lesquelles les phosphates d'amine (b) sont choisis parmi le phosphate de diméthylammonium, le phosphate de diéthylammonium, le phosphate d'éthylènediamine, l'orthophosphate de mélamine et le pyrophosphate de mélamine.

11. Compositions conformes à l'une des revendications 8 à 10, dans lesquelles les phosphonates d'ammonium (b) sont des phosphonates monosubstitués ou polysubstitués et sont choisis parmi les sels dérivés des acides monophosphoniques et polyphosphoniques.

12. Compositions conformes à l'une des revendications 8 à 11, dans lesquelles le polymère (a) est choisi parmi les terpolymères acrylonitrile/butadiène/styrène (ABS), les copolymères styrène/acrylonitrile (SAN), les polyuréthanes, le poly(éthylène téréphtalate), le poly(butylène téréphtalate), les polyamides, et les polymères et copolymères d'oléfines de formule générale R'-CH=CH₂ où R' représente un atome d'hydrogène ou un groupe alkyle en C₁₋₈ ou aryle en C₆₋₁₀, et en particulier :
(1) du polypropylène isotactique, ou en majeure partie isotactique,
(2) du polyéthylène HDPE, LLDPE ou LDPE,
(3) des copolymères cristallins de propylène et de petites proportions d'éthylène et/ou d'autres alpha-oléfines comme le butène-1, l'hexène-1, l'octène-1 et le 4-méthyl-pentène-1,
(4) des compositions hétérogènes comprenant (A) une fraction d'homopolymère de propylène ou de copolymères de propylène indiqués en (3) et (B) une fraction de copolymères, constituée de copolymères élastomères d'éthylène et d'une alpha-oléfine, contenant éventuellement une faible quantité d'un diène, cette alpha-oléfine étant de préférence choisie parmi le propylène et le butène-1, et
(5) des copolymères élastomères d'éthylène et d'alpha-oléfine(s) contenant éventuellement une faible quantité de diène.

13. Articles moulés obtenus à partir des compositions conformes à l'une des revendications 8 à 12.

## Revendications (Revendications pour l'(les) Etat(s) contractant(s) suivant(s): DK)

1. Dérivés d'améline, de formule générale (I) : 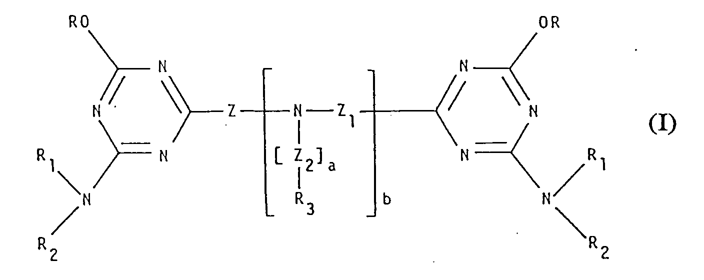 dans laquelle :
R représente un atome d'hydrogène, un groupe alcényle en C₂₋₆, cycloalkyle en C₆₋₁₂, alkyl-cycloalkyle en C₆₋₁₂, aryle en C₆₋₁₂ ou aralkyle en C₇₋₁₂, ou un groupe de formule -(CₙH₂ₙ)-Y où n représente un nombre entier valant de 1 à 8 et Y représente un atome d'hydrogène ou un groupe cyano, alcoxy en C₁₋₄, alcényloxy en C₂₋₄, cycloalkyle en C₆₋₁₂, alkyl-cycloalkyle en C₆₋₁₂ ou aryloxy en C₆₋₁₂, ou encore un groupe de formule -N(R₄)₂ où les symboles R₄ représentent des groupes alkyle en C₁₋₄ ou alcényle en C₃₋₄ qui peuvent être identiques ou différents l'un de l'autre, ou ce fragment -N(R₄)₂ est remplacé par un groupe hétérocyclique azoté, relié à la chaîne alkyle par un atome d'azote et choisi parmi les groupes pyrrolidinyle, pipéridinyle, morpholino, thiomorpholino, pipérazinyle, 4-méthylpipérazinyle et 4-éthylpipérazinyle ; les groupes représentés par R₁ et R₂, qui peuvent être identiques ou différents l'un de l'autre et identiques ou différents d'un cycle triazine à l'autre, sont choisis parmi les atomes d'hydrogène, les groupes alkyle en C₁₋₁₈, alcényle en C₂₋₈, cycloalkyle en C₆₋₁₆ ou alkyl-cycloalkyle en C₆₋₁₆, et les groupes de formule -(CₘH₂ₘ)-O-R₅ ou -(CₚH₂ₚ)-N(R₆)₂, où m représente un nombre entier valant de 2 à 8, p représente un nombre entier valant de 2 à 6, R₅ représente un atome d'hydrogène, un groupe alkyle en C₁₋₈ ou alcényle en C₂₋₆, ou un groupe de formule
-(C_{q}H_{2q})-O-R₇ où q représente un nombre entier valant de 1 à 4 et R₇ représente un atome d'hydrogène ou un groupe alkyle en C₁₋₄, cycloalkyle en C₆₋₁₂ ou alkyl-cycloalkyle en C₆₋₁₂, et les groupes représentés par R₆, qui peuvent être identiques ou différents l'un de l'autre, sont choisis parmi les atomes d'hydrogène et les groupes alkyle en C₁₋₈, alcényle en C₂₋₆, cycloalkyle en C₆₋₁₂, alkyl-cycloalkyle en C₆₋₁₂ ou hydroxyalkyle en C₁₋₄, ou encore le fragment -N(R₆)₂ est remplacé par un groupe hétérocyclique azoté, relié à la chaîne alkyle par un 'atome d'azote et choisi parmi les groupes aziridinyle, pyrrolidinyle, pipéridinyle, morpholino, thiomorpholino, pipérazinyle, 4-méthylpipérazinyle et 4-éthylpipérazinyle ;
ou bien, dans la formule générale (I), l'un des fragments -NR₁R₂ ou les deux sont remplacés chacun par un groupe hétérocyclique azoté, relié au cycle triazine par un atome d'azote et choisi parmi les groupes aziridinyle, pyrrolidinyle, pipéridinyle, morpholino, thiomorpholino, pipérazinyle, 4-méthylpipérazinyle, 4-éthylpipérazinyle, 2-méthylpipérazinyle, 2,5-diméthylpipérazinyle, 2,3,5,6-tétraméthylpipérazinyle, 2,2,5,5-tétraméthylpipérazinyle, 2-éthylpipérazinyle et 2,5-diéthylpipérazinyle ; a vaut 0 ou 1 ;
b vaut 0 ou représente un nombre entier valant de 1 à 5 ;
R₃ représente un atome d'hydrogène ou un groupe de formule 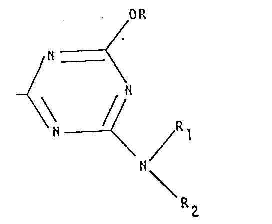 et sa signification peut varier d'un motif à l'autre ;
et si b vaut 0 :
Z représente un reste divalent, choisi parmi ceux qui correspondent aux formules suivantes : 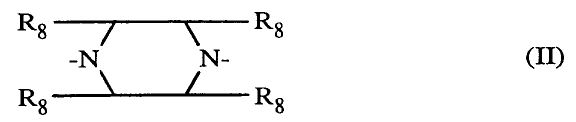 où les groupes représentés par R₈, qui peuvent être identiques ou différents les uns des autres, sont des atomes d'hydrogène ou des groupes alkyle en C₁₋₄ ; 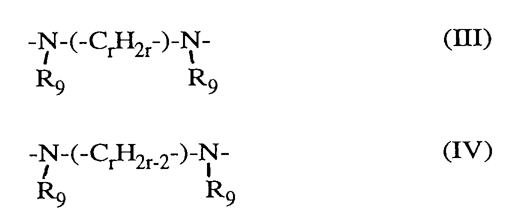 où r représente un nombre entier valant de 2 à 14 et R₉ est choisi parmi un atome d'hydrogène et un groupe alkyle en C₁₋₄, alcényle en C₂₋₆ ou hydroxyalkyle en C₁₋₄ ;
-NH-(-CH₂-)ₛ-O-(CH₂-)ₛ-NH- (V)
-NH-[(-CH₂-)ₛ-O]ₜ-(CH₂-)ₛ-NH- (VI)
où s représente un nombre entier valant de 2 à 5 et t représente un nombre entier valant de 1 à 3 ; 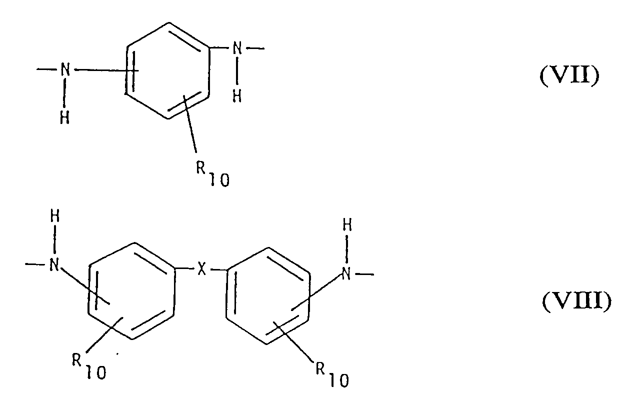 où X représente une liaison directe carbone-carbone, -O-, -S-, -S-S-, -SO-, -SO₂-, -NH-, -NH-SO₂-, -NH-CO-, -N=N-, ou -CH₂-, et R₁₀ représente un atome d'hydrogène ou un groupe hydroxy, alkyle en C₁₋₄ ou alcoxy en C₁₋₄ ; 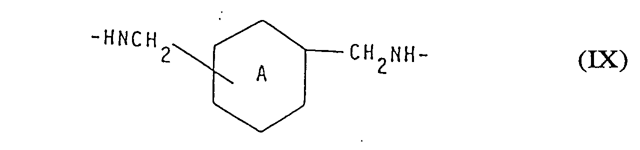 où A désigne un cycle saturé ou insaturé ; 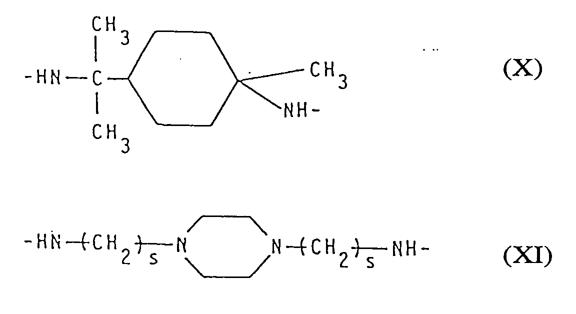 où s représente un nombre entier valant de 2 à 5 ;
si b représente un entier de 1 à 5 :
le fragment de formule 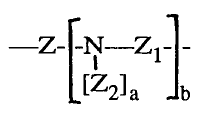 est un radical polyvalent choisi parmi ceux qui sont représentés par les formules suivantes : 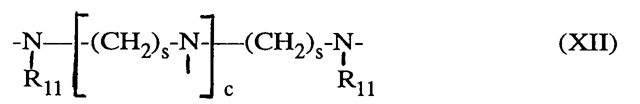 où R₁₁ représente un atome d'hydrogène ou un groupe alkyle en C₁₋₄, c représente un nombre entier valant de 1 à 5, et les indices s, qui peuvent être identiques ou différents les uns des autres, représentent des nombres entiers valant de 2 à 5 ; 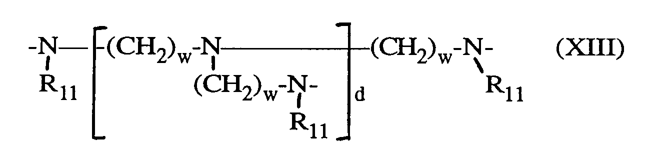 où R₁₁ représente un atome d'hydrogène ou un groupe alkyle en C₁₋₄, w représente un nombre entier valant de 2 à 4, et d vaut 1 ou 2.

2. Composés conformes à la revendication 1, dans lesquels au moins l'un des groupes R₁ et R₂, dans la formule générale (I), est un groupe de formule -(CₘH₂ₘ)-O-R₅ où m représente un nombre entier valant de 2 à 4 et R₅ représente un atome d'hydrogène ou un groupe alkyle en C₁₋₄.

3. Composés conformes à l'une des revendications 1 et 2, dans lesquels R, dans la formule générale (I), représente un atome d'hydrogène ou un groupe de formule -(CₙH₂ₙ)-Y où n représente un nombre entier valant de 1 à 4 et Y représente un atome d'hydrogène.

4. Procédé de préparation de composés de formule générale (I), conformes à l'une des revendications 1 à 3, qui comporte l'hydrolyse d'intermédiaires de formule générale (XIV) : 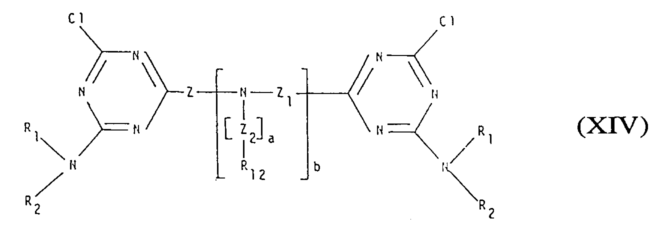 dans laquelle R₁, R₂, Z, Z₁, Z₂, a et b ont les significations définies dans la revendication 1 et R₁₂ représente un atome d'hydrogène ou un groupe de formule 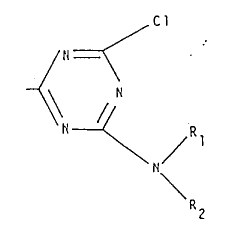 sa signification pouvant varier d'un motif répété à l'autre, ou la condensation d'intermédiaires de formule générale (XIV) avec un réactif de formule générale (XV)
R-OH (XV)
dans laquelle R possède les significations indiquées dans la revendication 1, sauf celle d'un atome d'hydrogène.

5. Procédé conforme à la revendication 4, dans lequel on effectue la réaction d'hydrolyse soit en présence d'un acide et à une température d'environ 60°C à environ 100°C, soit en présence d'une base et à une température d'environ 100°C à environ 180°C.

6. Procédé conforme à la revendication 4, dans lequel on effectue la réaction de condensation dans un solvant et/ou en présence d'un excès de réactif de formule générale (XV), en présence d'une base et à une température d'environ 60°C à environ 150°C.

7. Procédé de préparation de composés de formule générale (I), conformes à l'une des revendications 1 à 3, où R représente autre chose qu'un atome d'hydrogène, lequel procédé comporte la réaction de 1 mole d'une polyamine de formule générale (XVI) : 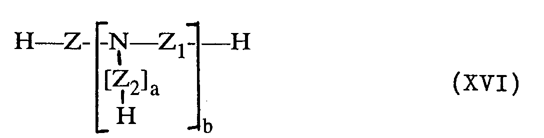 dans laquelle Z, Z₁, Z₂, a et b ont les définitions indiquées dans la revendication 1,
avec au plus (2+b) moles de l'intermédiaire de formule générale (XXI) : 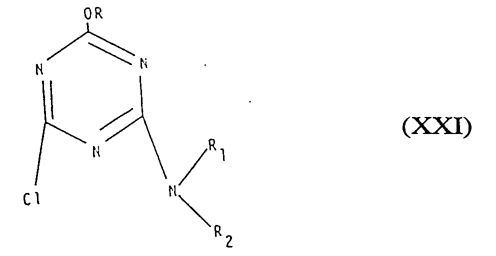 dans laquelle R, R₁ et R₂ ont les définitions indiquées dans la revendication 1, mais R représente autre chose qu'un atome d'hydrogène.

8. Compositions auto-extinguibles de polymères, comportant :
a) de 91 à 40 parties en poids d'un polymère thermoplastique et/ou d'un polymère présentant des propriétés d'élastomère,
b) de 6 à 33 parties en poids d'un ou de plusieurs phosphates et/ou phosphonates d'ammonium et/ou d'amine, et
c) de 3 à 27 parties en poids d'un ou de plusieurs composés de formule générale (I), conformes à l'une des revendications 1 à 4.

9. Compositions conformes à la revendication 8, dans lesquelles les phosphates d'ammonium (b) correspondent à la formule générale (NH₄)ₙ₊₂PₙO₃ₙ₊₁ où n représente un nombre entier valant au moins 2, ou bien à la formule générale (NH₄PO₃)ₙ où n vaut de 50 à 500.

10. Compositions conformes à l'une des revendications 8 et 9, dans lesquelles les phosphates d'amine (b) sont choisis parmi le phosphate de diméthylammonium, le phosphate de diéthylammonium, le phosphate d'éthylènediamine, l'orthophosphate de mélamine et le pyrophosphate de mélamine.

11. Compositions conformes à l'une des revendications 8 à 10, dans lesquelles les phosphonates d'ammonium (b) sont des phosphonates monosubstitués ou polysubstitués et sont choisis parmi les sels dérivés des acides monophosphoniques et polyphosphoniques.

12. Compositions corformes à l'une des revendications 8 à 11, dans lesquelles le polymère (a) est choisi parmi les terpolymères acrylonitrile/butadiène/styrène (ABS), les copolymères styrène/acrylonitrile (SAN), les polyuréthanes, le poly(éthylène téréphtalate), le poly(butylène téréphtalate), les polyamides, et les polymères et copolymères d'oléfines de formule générale R'-CH=CH₂ où R' représente un atome d'hydrogène ou un groupe alkyle en C₁₋₈ ou aryle en C₆₋₁₀, et en particulier :
(1) du polypropylène isotactique, ou en majeure partie isotactique,
(2) du polyéthylène HDPE, LLDPE ou LDPE,
(3) des copolymères cristallins de propylène et de petites proportions d'éthylène et/ou d'autres alpha-oléfines comme le butène-1, l'hexène-1, l'octène-1 et le 4-méthyl-pentène-1,
(4) des compositions hétérogènes comprenant (A) une fraction d'homopolymère de propylène ou de copolymères de propylène indiqués en (3) et (B) une fraction de copolymères, constituée de copolymères élastomères d'éthylène et d'une alpha-oléfine, contenant éventuellement une faible quantité d'un diène, cette alpha-oléfine étant de préférence choisie parmi le propylène et le butène-1, et
(5) des copolymères élastomères d'éthylène et d'alpha-oléfine(s) contenant éventuellement une faible quantité de diène.

13. Articles moulés obtenus à partir des compositions conformes à l'une des revendications 8 à 12.

## Revendications (Revendications pour l'(les) Etat(s) contractant(s) suivant(s): ES)

1. Procédé de préparation de dérivés d'améline, de formule générale (I) : 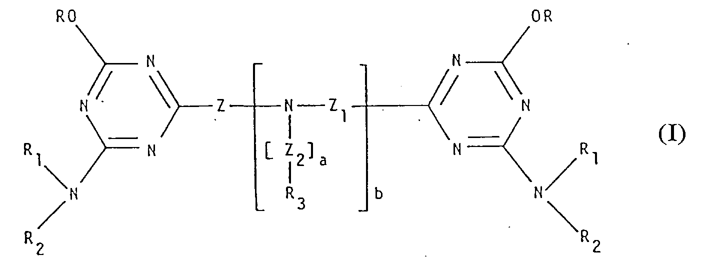 dans laquelle :
R représente un atome d'hydrogène, un groupe alcényle en C₂₋₆, cycloalkyle en C₆₋₁₂, alkyl-cycloalkyle en C₆₋₁₂, aryle en C₆₋₁₂ ou aralkyle en C₇₋₁₂, ou un groupe de formule -(CₙH₂ₙ)-Y où n représente un nombre entier valant de 1 à 8 et Y représente un atome d'hydrogène ou un groupe cyano, alcoxy en C₁₋₄, alcényloxy en C₂₋₄, cycloalkyle en C₆₋₁₂, alkyl-cycloalkyle en C₆₋₁₂ ou aryloxy en C₆₋₁₂, ou encore un groupe de formule -N(R₄)₂ où les symboles R₄ représentent des groupes alkyle en C₁₋₄ ou alcényle en C₃₋₄ qui peuvent être identiques ou différents l'un de l'autre, ou ce fragment -N(R₄)₂ est remplacé par un groupe hétérocyclique azoté, relié à la chaîne alkyle par un atome d'azote et choisi parmi les groupes pyrrolidinyle, pipéridinyle, morpholino, thiomorpholino, pipérazinyle, 4-méthylpipérazinyle et 4-éthylpipérazinyle ; les groupes représentés par R₁ et R₂, qui peuvent être identiques ou différents l'un de l'autre et identiques ou différents d'un cycle triazine à l'autre, sont choisis parmi les atomes d'hydrogène, les groupes alkyle en C₁₋₁₈, alcényle en C₂₋₈, cycloalkyle en C₆₋₁₆ ou alkyl-cycloalkyle en C₆₋₁₆, et les groupes de formule -(CₘH₂ₘ)-O-R₅ ou -(CₚH₂ₚ)-N(R₆)₂, où m représente un nombre entier valant de 2 à 8, p représente un nombre entier valant de 2 à 6, R₅ représente un atome d'hydrogène, un groupe alkyle en C₁₋₈ ou alcényle en C₂₋₆, ou un groupe de formule
-(C_{q}H_{2q})-O-R₇ où q représente un nombre entier valant de 1 à 4 et R₇ représente un atome d'hydrogène ou un groupe alkyle en C₁₋₄, cycloalkyle en C₆₋₁₂ ou alkyl-cycloalkyle en C₆₋₁₂, et les groupes représentés par R₆, qui peuvent être identiques ou différents l'un de l'autre, sont choisis parmi les atomes d'hydrogène et les groupes alkyle en C₁₋₈, alcényle en C₂₋₆, cycloalkyle en C₆₋₁₂, alkyl-cycloalkyle en C₆₋₁₂ ou hydroxyalkyle en C₁₋₄, ou encore le fragment -N(R₆)₂ est remplacé par un groupe hétérocyclique azoté, relié à la chaîne alkyle par un 'atome d'azote et choisi parmi les groupes aziridinyle, pyrrolidinyle, pipéridinyle, morpholino, thiomorpholino, pipérazinyle, 4-méthylpipérazinyle et 4-éthylpipérazinyle ;
ou bien, dans la formule générale (I), l'un des fragments -NR₁R₂ ou les deux sont remplacés chacun par un groupe hétérocyclique azoté, relié au cycle triazine par un atome d'azote et choisi parmi les groupes aziridinyle, pyrrolidinyle, pipéridinyle, morpholino, thiomorpholino, pipérazinyle, 4-méthylpipérazinyle, 4-éthylpipérazinyle, 2-méthylpipérazinyle, 2,5-diméthylpipérazinyle, 2,3,5,6-tétraméthylpipérazinyle, 2,2,5,5-tétraméthylpipérazinyle, 2-éthylpipérazinyle et 2,5-diéthylpipérazinyle ; a vaut 0 ou 1 ;
b vaut 0 ou représente un nombre entier valant de 1 à 5 ;
R₃ représente un atome d'hydrogène ou un groupe de formule 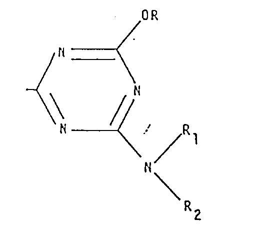 et sa signification peut varier d'un motif à l'autre ;
et si b vaut 0 :
Z représente un reste divalent, choisi parmi ceux qui correspondent aux formules suivantes : 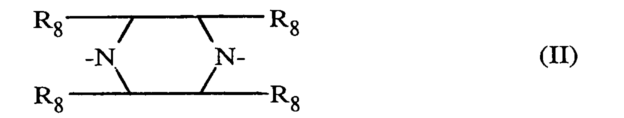 où les groupes représentés par R₈, qui peuvent être identiques ou différents les uns des autres, sont des atomes d'hydrogène ou des groupes alkyle en C₁₋₄ ; 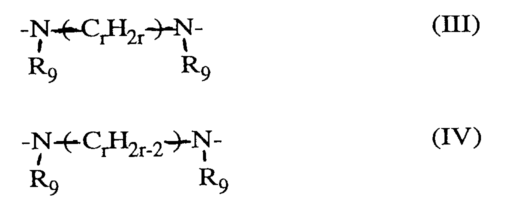 où r représente un nombre entier valant de 2 à 14 et R₉ est choisi parmi un atome d'hydrogène et un groupe alkyle en C₁₋₄, alcényle en C₂₋₆ ou hydroxyalkyle en C₁₋₄ ;
-NH-(-CH₂-)ₛ-O-(CH₂-)ₛ-NH- (V)
-NH-[(-CH₂-)ₛ-O]ₜ-(CH₂-)ₛ-NH- (VI)
où s représente un nombre entier valant de 2 à 5 et t représente un nombre entier valant de 1 à 3 ; 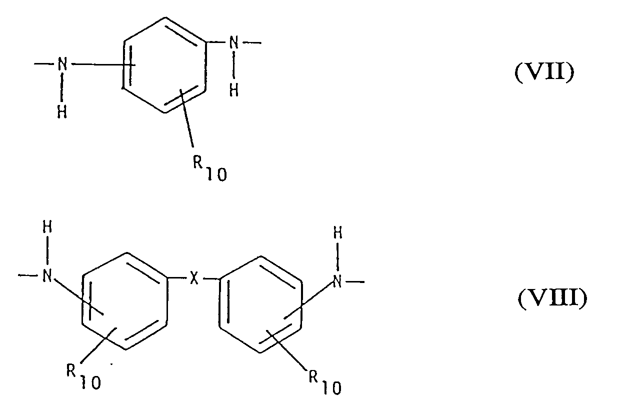 où X représente une liaison directe carbone-carbone, -O-, -S-, -S-S-, -SO-, -SO₂-, -NH-, -NH-SO₂-, -NH-CO-, -N=N-, ou -CH₂-, et R₁₀ représente un atome d'hydrogène ou un groupe hydroxy, alkyle en C₁₋₄ ou alcoxy en C₁₋₄ ; 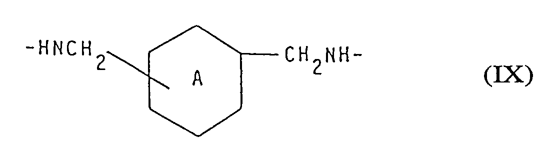 où A désigne un cycle saturé ou insaturé ; 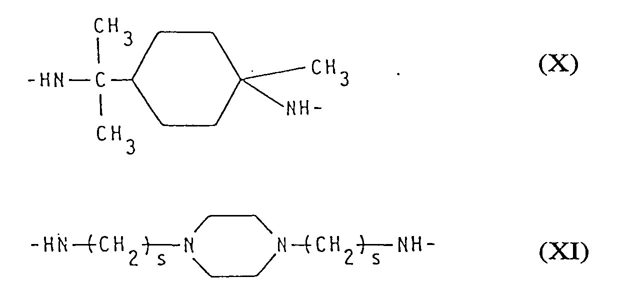 où s représente un nombre entier valant de 2 à 5 ;
sous réserve que, si R représente un groupe alkyle en C₁₋₈, cycloalkyle en C₆₋₁₂, aralkyle en C₇₋₁₂, phényle ou naphtyle, portant éventuellement des substituants inertes, et si R₁ et R₂ représentent des groupes alkyle en C₁₋₃ ou cycloalkyle en C₆₋₁₂ ou si -NR₁R₂ représente un groupe pipéridinyle ou morpholino, Z ne peut pas être un reste de formule (II) où les quatre symboles R₈ représenteraient tous des atomes d'hydrogène ;
si b représente un entier de 1 à 5 :
le fragment de formule 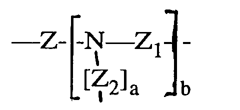 est un radical polyvalent choisi parmi ceux qui sont représentés par les formules suivantes : 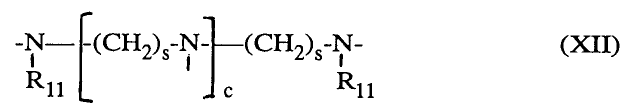 où R₁₁ représente un atome d'hydrogène ou un groupe alkyle en C₁₋₄, c représente un nombre entier valant de 1 à 5, et les indices s, qui peuvent être identiques ou différents les uns des autres, représentent des nombres entiers valant de 2 à 5 ; 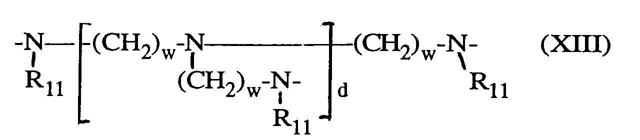 où R₁₁ représente un atome d'hydrogène ou un groupe alkyle en C₁₋₄, w représente un nombre entier valant de 2 à 4, et d vaut 1 ou 2 ;
ledit procédé comportant :
a) si R représente un atome d'hydrogène, l'hydrolyse d'intermédiaires de formule générale (XIV) : 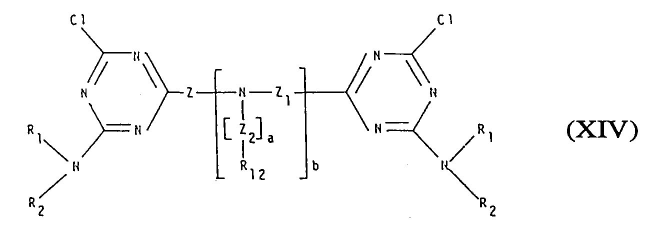 dans laquelle R₁, R₂, Z, Z₁, Z₂, a et b ont les significations définies plus haut et R₁₂ représente un atome d'hydrogène ou un groupe de formule 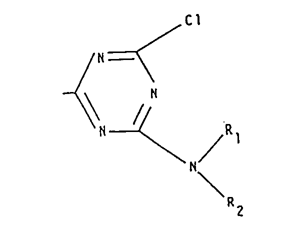 sa signification pouvant varier d'un motif répété à l'autre ; ou bien,
b) si R représente autre chose qu'un atome d'hydrogène, la condensation d'intermédiaires de formule générale (XIV) avec un réactif de formule générale (XV)
R-OH (XV)
dans laquelle R possède les significations indiquées plus haut, sauf celle d'un atome d'hydrogène ; ou encore,
c) si R représente autre chose qu'un atome d'hydrogène, la réaction de 1 mole d'une polyamine de formule générale (XVI) : 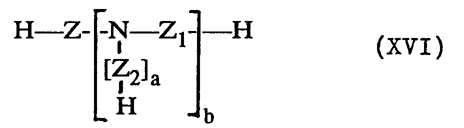 dans laquelle Z, Z₁, Z₂, a et b ont les définitions indiquées dans la revendication 1,
avec au plus (2+b) moles de l'intermédiaire de formule générale (XXI) : 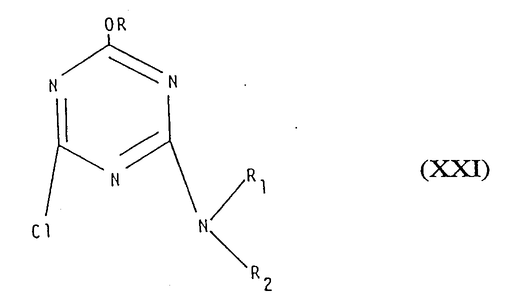 dans laquelle R, R₁ et R₂ ont les définitions indiquées dans la revendication 1, mais R représente autre chose qu'un atome d'hydrogène.

2. Procédé conforme à la revendication 1, dans lequel on prépare des composés dans lesquels au moins l'un des groupes R₁ et R₂, dans la formule générale (I), est un groupe de formule -(CₘH₂ₘ)-O-R₅ où m représente un nombre entier valant de 2 à 4 et R₅ représente un atome d'hydrogène ou un groupe alkyle en C₁₋₄.

3. Procédé conforme à l'une des revendications 1 et 2, dans lequel R, dans la formule générale (I), représente un atome d'hydrogène ou un groupe de formule -(CₙH₂ₙ)-Y où n représente un nombre entier valant de 1 à 4 et Y représente un atome d'hydrogène.

4. Procédé conforme à l'une des revendications 1 à 3, variante (a), dans lequel on effectue la réaction d'hydrolyse soit en présence d'un acide et à une température d'environ 60°C à environ 100°C, soit en présence d'une base et à une température d'environ 100°C à environ 180°C.

5. Procédé conforme à l'une des revendications 1 à 3, variante (b), dans lequel on effectue la réaction de condensation dans un solvant et/ou en présence d'un excès de réactif de formule générale (XV), en présence d'une base et à une température d'environ 60°C à environ 150°C.

6. Compositions auto-extinguibles de polymères, comportant :
a) de 91 à 40 parties en poids d'un polymère thermoplastique et/ou d'un polymère présentant des propriétés d'élastomère,
b) de 6 à 33 parties en poids d'un ou de plusieurs phosphates et/ou phosphonates d'ammonium et/ou d'amine, et
c) de 3 à 27 parties en poids d'un ou de plusieurs composés de formule générale (I), définis dans l'une des revendications 1 à 3.

7. Compositions conformes à la revendication 6, dans lesquelles les phosphates d'ammonium (b) correspondent à la formule générale (NH₄)ₙ₊₂PₙO₃ₙ₊₁ où n représente un nombre entier valant au moins 2, ou bien à la formule générale (NH₄PO₃)ₙ où n vaut de 50 à 500.

8. Compositions conformes à l'une des revendications 6 et 7, dans lesquelles les phosphates d'amine (b) sont choisis parmi le phosphate de diméthylammonium, le phosphate de diéthylammonium, le phosphate d'éthylènediamine, l'orthophosphate de mélamine et le pyrophosphate de mélamine.

9. Compositions conformes à l'une des revendications 6 à 8, dans lesquelles les phosphonates d'ammonium (b) sont des phosphonates monosubstitués ou polysubstitués et sont choisis parmi les sels dérivés des acides monophosphoniques et polyphosphoniques.

10. Compositions conformes à l'une des revendications 6 à 9, dans lesquelles le polymère (a) est choisi parmi les terpolymères acrylonitrile/butadiène/styrène (ABS), les copolymères styrène/acrylonitrile (SAN), les polyuréthanes, le poly(éthylène téréphtalate), le poly(butylène téréphtalate), les polyamides, et les polymères et copolymères d'oléfines de formule générale R'-CH=CH₂ où R' représente un atome d'hydrogène ou un groupe alkyle en C₁₋₈ ou aryle en C₆₋₁₀, et en particulier :
(1) du polypropylène isotactique, ou en majeure partie isotactique,
(2) du polyéthylène HDPE, LLDPE ou LDPE,
(3) des copolymères cristallins de propylène et de petites proportions d'éthylène et/ou d'autres alpha-oléfines comme le butène-1, l'hexène-1, l'octène-1 et le 4-méthyl-pentène-1,
(4) des compositions hétérogènes comprenant (A) une fraction d'homopolymère de propylène ou de copolymères de propylène indiqués en (3) et (B) une fraction de copolymères, constituée de copolymères élastomères d'éthylène et d'une alpha-oléfine, contenant éventuellement une faible quantité d'un diène, cette alpha-oléfine étant de préférence choisie parmi le propylène et le butène-1, et
(5) des copolymères élastomères d'éthylène et d'alpha-oléfine(s) contenant éventuellement une faible quantité de diène.

11. Articles moulés obtenus à partir des compositions conformes à l'une des revendications 6 à 10.
